# EUROPÄISCHE PATENTSCHRIFT

(11) **EP 0 417 725 B1**
(45) Veröffentlichungstag und Bekanntmachung des Hinweises auf die Patenterteilung: **23.04.1997**
(21) Anmeldenummer: 90117470.6
(22) Anmeldetag: 11.09.1990
(51) Int. Cl.: C07J 9/00, C07J 41/00, C07J 31/00, C07J 51/00, A61K 31/575

(54) **Gallensäurederivate, Verfahren zu ihrer Herstellung, Verwendung als Arzneimittel**
Bile-acid derivatives, a process for their production and their use as medicines
Dérivés des acides biliorés, leur procédé de préparation et leur usage comme médicaments

(30) Priorität: 14.09.1989 DE 3930696
(43) Veröffentlichungstag der Anmeldung: 20.03.1991
(73) Patentinhaber: HOECHST AKTIENGESELLSCHAFT, 65926 Frankfurt am Main (DE)
(72) Erfinder: Kramer, Werner, Dr., D-6500 Mainz (DE); Wess, Günther, Dr., D-6455 Erlensee (DE)

(56) Entgegenhaltungen:
- EP-A- 0 105 404
- EP-A- 0 232 788
- US-A- 2 441 129
- US-A- 3 937 815
- US-A- 4 418 059
- US-A- 4 499 020
- J. PRAKT. CHEMIE, Bd. 321, Heft 3, 1979, Seiten 522-528, I. NICULESCU et al.
- EXPER. CLIN. CHEMOTHER., Bd. III, , 1984, Georg Thieme Verlag, Seiten 35-91, G. EISENBRAND et al.

## Beschreibung

Die orale Gabe von Arzneimitteln ist die häufigste und bequemste Art der Arzneimittelapplikation. Damit aber ein Wirkstoff in die Blutbahn gelangt, muß ein Resorptionsprozeß im Magen-Darm-Trakt erfolgen. Danach verteilt sich der Arzneistoff im Körper entsprechend seiner spezifischen Eigenschaften. Zahlreiche Arzneistoffe werden aber nur sehr schlecht oder praktisch nicht resorbiert, so daß eine orale Applikation nicht möglich ist.

Bekannt ist, daß durch Kopplung von nicht-resorbierbaren Pharmaka an Vitamin B12 eine enterale Aufnahme dieser Pharmaka erreicht werden kann (Proceed. Symp. Control. Rel. Bioact. Mater. 15 (1988) Controlled Release Society Inc., see PCT/AU 86/0299).

Wegen der sehr geringen Kapazität dieses Aufnahmeweges über Vitamin B12 können aber auch nur sehr geringe Mengen des Pharmakons in den Körper eingebracht werden.

US 2,441,129, US 3,937,815 und EP 0 105 404 beschreiben natürliche Gallensäuren, die über die Seitenkette (C-24-Carboxylgruppe) derivatisiert sind.

US 4,418,059 beschreibt Gallensäurederivate, die in 3-Position eine Carbonamidgruppe tragen.

US 4,499,020 beschreibt Gallensäurederivate, die über eine Estergruppe in 3-Position weiter funktionalisiert sind.

Es wurden nun Gallensäurederivate gefunden, die dazu verwendet werden, schwer oder nicht resorbierbare Wirkstoffe resorbierbar zu machen und damit die orale Applikation dieser Stoffe auch in hohen Dosen zu ermöglichen. Darüberhinaus kann mit den Gallensäurederivaten eine leberspezifische Wirkung von Wirkstoffen erreicht werden.

Die Erfindung betrifft Gallensäurederivate der Formel I

W-X-G I

bestehend aus
einem modifizierten Gallensäurerest G: 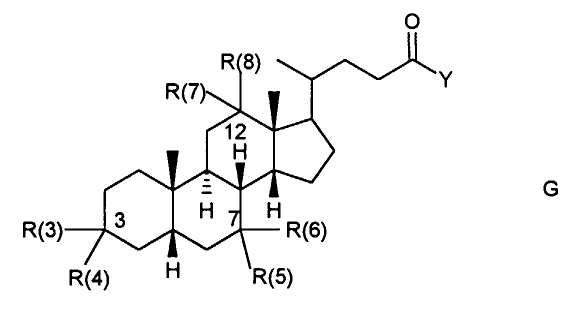 in welcher R(3) - R(8) gleich oder verschieden sind und folgende Bedeutung haben:
eine Bindung zu W-X-, wobei insgesamt bis zu zwei W-X-Einheiten gebunden sein können,
R(3) und R(4), R(5) und R(6), R(7) und R(8) jeweils gemeinsam der Sauerstoff einer Carbonylgruppe, 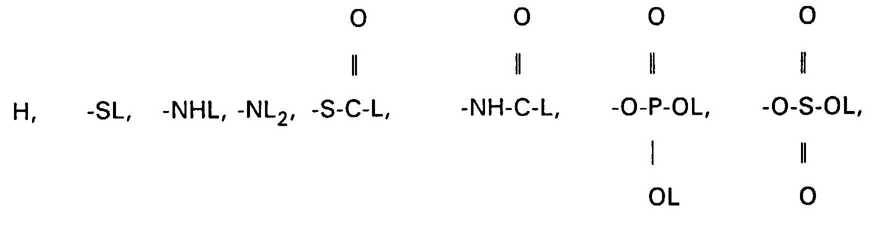 -L, wobei L bedeutet:
H, einen gesättigten oder ungesättigten Alkylrest mit 1 - 10 C-Atomen, der verzweigt oder unverzweigt ist, Cycloalkyl mit 3 - 8 C-Atomen, einen Phenylrest (der unsubstituiert oder 1 - 3-fach substituiert ist mit F, Cl, Br, (C₁-C₄)-Alkyl oder (C₁-C₄)-Alkoxy, einen Benzylrest, der unsubstituiert oder 1 - 3-fach substituiert ist mit F, Cl, Br, (C₁-C₄)-Alkyl oder (C₁-C₄)-Alkoxy; und in der Y folgende Bedeutung hat
-OH oder
eine über die Aminogruppe gebundene Aminosäure oder Aminosulfonsäure und deren Alkali- und Erdalkalisalze, -OKa, wobei Ka ein Kation bedeutet wie z. B. ein Alkali oder Erdalkaliion oder auch ein quartäres Ammoniumion;
einem Verbindungsglied X, das eine direkte Bindung oder ein Zwischenglied X ist: 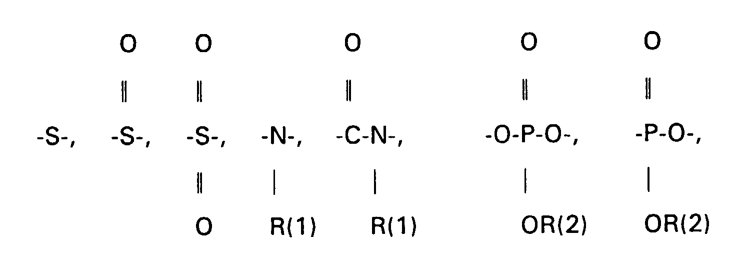 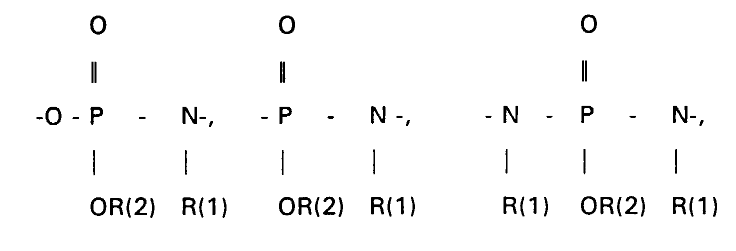 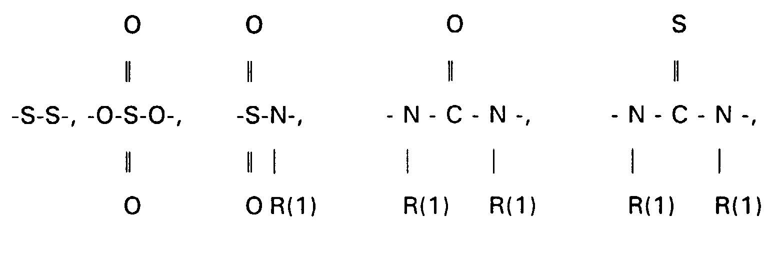 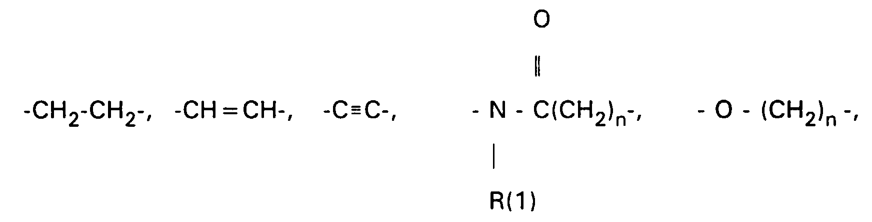 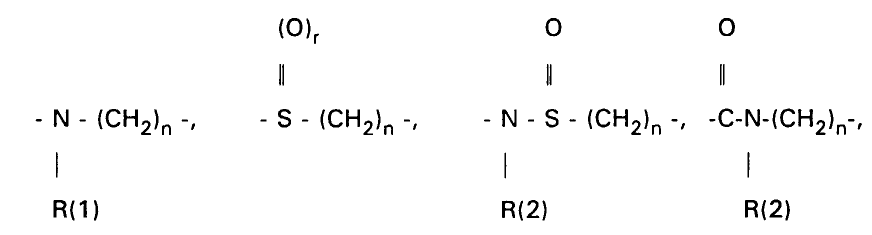 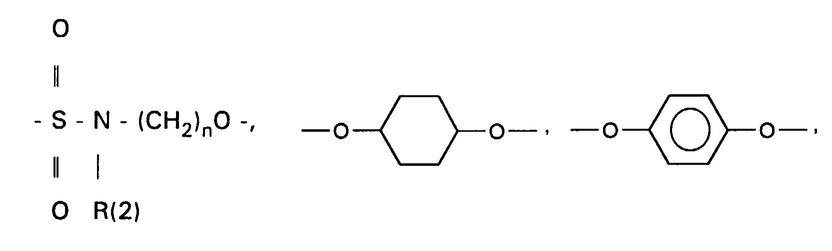 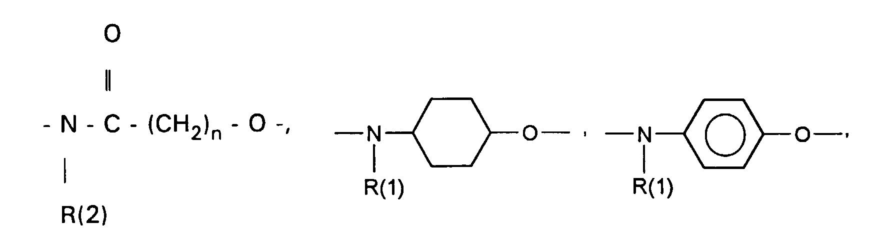 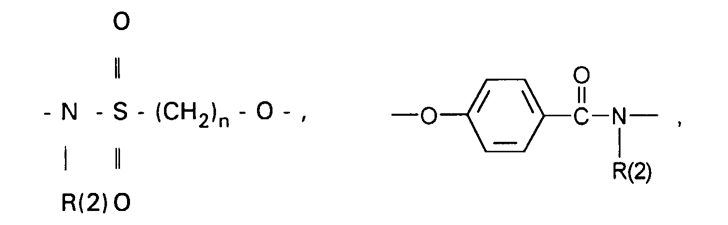 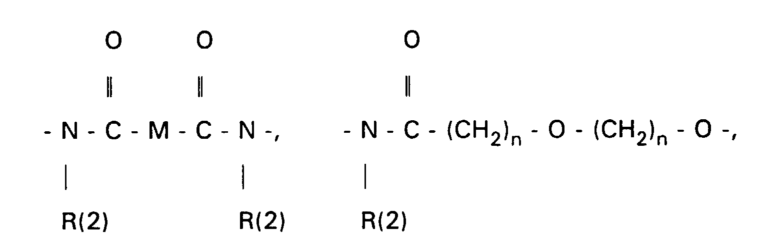 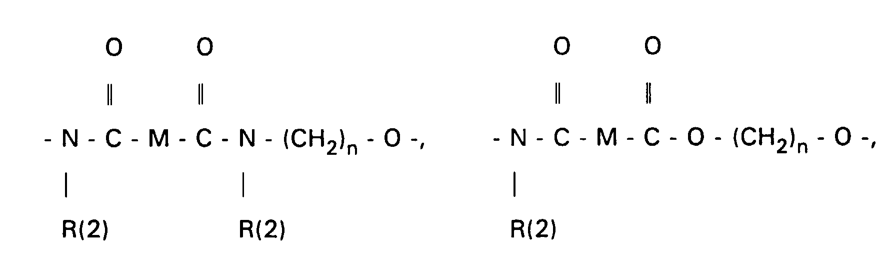 worin
- R(1) =: H, (C₁-C₈)-Alkyl; die Gruppe 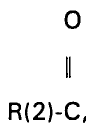 Phenyl, Benzyl, unsubstituiert oder 1 - 3-fach substituiert mit F, Cl, Br, (C₁-C₄)-Alkyl oder Alkoxy mit 1 - 4 C-Atomen
- R(2) =: H, (C₁-C₈)-Alkyl, Phenyl, Benzyl, unsubstituiert oder 1 - 3-fach substituiert mit F, Cl, Br, (C₁-C₄)-Alkyl oder Alkoxy mit 1 - 4 C-Atomen
- m =: 0-6
- n =: 1-16
- p =: 1, 2, 3
- r =: 0 - 2 und
- M =: -(CH₂)ₘ-, -(C=C)ₚ-, -C≡C-, 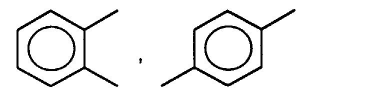
und einem Wirkstoffteil W, der ein Peptid, ein Antibiotikum z. B. ein β-Lactam-Antibiotikum, eine antivirale Substanz, ein Krebsmittel wie z. B. Chlorambucil, ein Leberschutzmittel, ein Antihyperlipidämikum z. B. ein HMG-CoA Reductase-Hemmer, ein Diuretikum, ein Blutdrucksenker, ein Renininhibitor oder ein Prolylhydroxylase-Hemmer ist,
wobei eine Verbindung der Formel 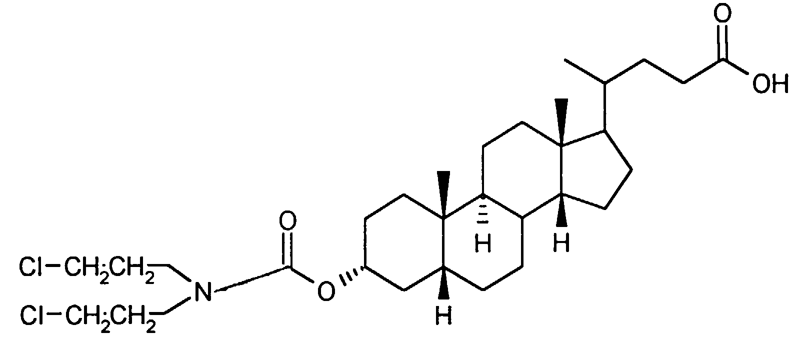 ausgenommen ist.

Bevorzugt sind Verbindungen der Formel I, worin die Reste folgende Bedeutung haben:
G 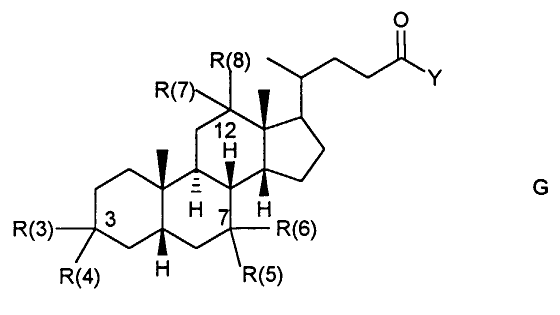 mit R(3) - R (8) gleich oder verschieden und folgende Bedeutung haben: eine Bindung zu W-X-, wobei bis zu zwei W-X-Einheiten gebunden sein können, 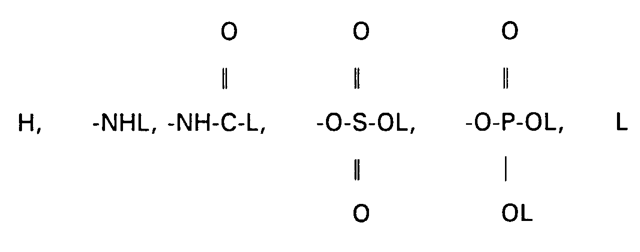 wobei L bedeutet:
H, einen gesättigten Alkylrest mit 1 - 6 C-Atomen, der verzweigt oder unverzweigt sein kann, einen Phenylrest, der unsubstituiert oder 1 - 3 fach substituiert ist mit F, Cl, Br, (C₁-C₄)-Alkyl oder (C₁-C₄)-Alkoxy
- Y:: -OH,
-Oka, wobei Ka ein Alkali oder Erdalkalikation oder ein Ammoniumion bedeutet 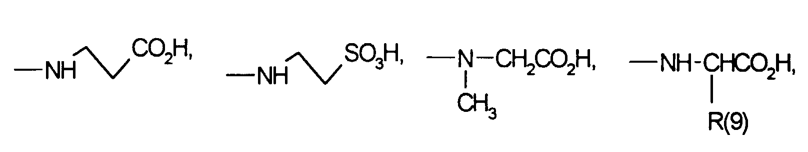
wobei R(9) bedeutet
Methyl, Isopropyl, Isobutyl, 2-Butyl, Benzyl, 4-Hydroxybenzyl, Hydroxymethyl, 1-Hydroxyethyl, H₃CSCH₂CH₂-, HO₂CCH₂-, HO₂CCH₂CH₂-
das Verbindungsglied X, wobei G und W an beliebigen Enden von X gebunden sein können, 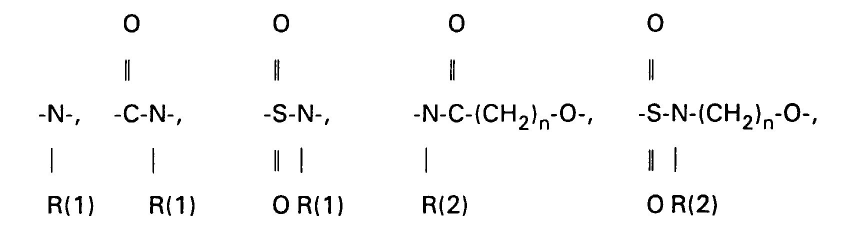 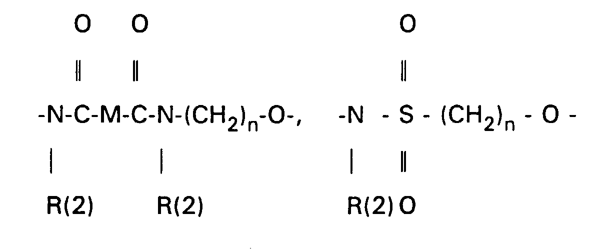
- mit R(1) =: H, (C₁-C₈)-Alkyl, 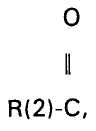 Phenyl, Benzyl, wobei die aromatischen Ringe unsubstituiert oder 1 - 3 fach substituiert sind mit F, Cl, Br, (C₁-C₄)-Alkyl und (C₁-C₄)-Alkoxy;
- mit R(2) =: H, (C₁-C₈)-Alkyl, Phenyl, Benzyl; jeweils unsubstituiert oder 1 - 3 fach substituiert mit F, Cl, Br, (C₁-C₄)-Alkyl oder Alkoxy mit 1 - 4 C-Atomen
- n =: 1 - 16 und
- M =: -CH₂-CH₂-,
-CH=CH-
W
ein Peptid, ein Antibiotikum z. B. ein β-Lactam-Antibiotikum, eine antivirale Substanz, ein Krebsmittel wie z. B. Chlorambucil, ein Leberschutzmittel, ein Antihyperlipidämikum z. B. einen HMG-CoA Reductase-Hemmer, ein Diuretikum, einen Blutdrucksenker, einen Renininhibitor, einen Prolylhydroxylase-Hemmer, wobei eine Verbindung der Formel 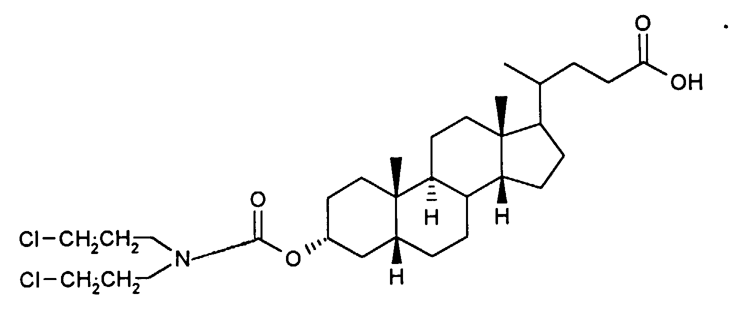 ausgenommen ist.

Die Erfindung betrifft ferner ein Verfahren zur Herstellung einer Verbindung I, dadurch gekennzeichnet, daß man
a) im Falle von X = eine direkte Bindung geeignete reaktionsfähige Formen von G und W nach bekannten Verfahren miteinander zur Reaktion bringt oder
b) im Falle von X = ein Zwischenglied
   α) reaktionsfähige Formen von G-X mit W bzw.,
   β) reaktionsfähige Formen von W-X mit G nach bekannten Verfahren miteinander zur Reaktion bringt.
**a) X = eine direkte Bindung**
   Die Gallensäuren werden entweder in freier Form oder in geschützter Form eingesetzt. Nach der Verknüpfung mit W erfolgt gegebenenfalls die Abspaltung der Schutzgruppen und die Umwandlung der C-24 Carboxylfunktion in ein erfindungsgemäßes Derivat. Als Schutzgruppen eignen sich für die Alkoholgruppen zweckmäßigerweise Acetyl, Tetrahydropyranyl, t-Butyldimethylsilyl oder Benzyl. Als Schutzgruppen für die C-24 Carboxylgruppe kommen verschiedene Alkyl- oder Benzylester in Frage, aber auch z.B. Orthoester.
   Beispielsweise reagiert Gallensäure an Position 3 bevorzugt, aber auch an Position 7 mit aktivierten Formen von Carbonsäuren, wie Säurechloriden oder gemischten Anhydriden unter Zusatz von Basen wie Trialkylamin, Pyridin, aber auch NaOH bei Zimmertemperatur in geeigneten Lösungsmitteln wie Tetrahydrofuran, Methylenchlorid oder Essigester, aber auch Dimethylformamid (DMF) oder Dimethoxyethan (DME).
   Die verschiedenen Isomeren können z.B. chromatographisch getrennt werden.
   Durch die Verwendung von geeigneten Schutzgruppen läßt sich die Reaktion selektiv durchführen. Analog lassen sich entsprechende Aminogallensäuren in entsprechende Amide überführen. Auch hier kann die Reaktion entweder mit geschützten oder freien Gallensäuren durchgeführt werden.
   Analog lassen sich weitere erfindungsgemäße Verbindungen nach bekannten Standardverfahren verknüpfen.
**b) X = ein Zwischenglied**
   Die unter a) angegebenen Verfahren werden auch angewendet, um die Verknüpfung W-X mit G bzw. W mit X-G durchzuführen. Zweckmäßigerweise setzt man auch hier den Gallensäureteil entweder geschützt oder ungeschützt ein.
   Ein bevorzugtes Herstellungsverfahren besteht darin, reaktionsfähige Formen von W mit reaktionsfähigen Formen X-G umzusetzen. Gegebenenfalls schließen sich nach der Verknüpfung die Abspaltung von Schutzgruppen und die Umwandlung von C-24 Carboxyl in die erfindungsgemäßen Verbindungen an.
   Die Herstellung von reaktionsfähigen Gallensäurebausteinen X-G ist im Formelschema 1-4 Punkt c) am Beispiel der Cholsäure angegeben.
c) Herstellung von reaktionsfähigen Gallensäurebausteinen X-G am Beispiel der Cholsäure, Schema 1-4

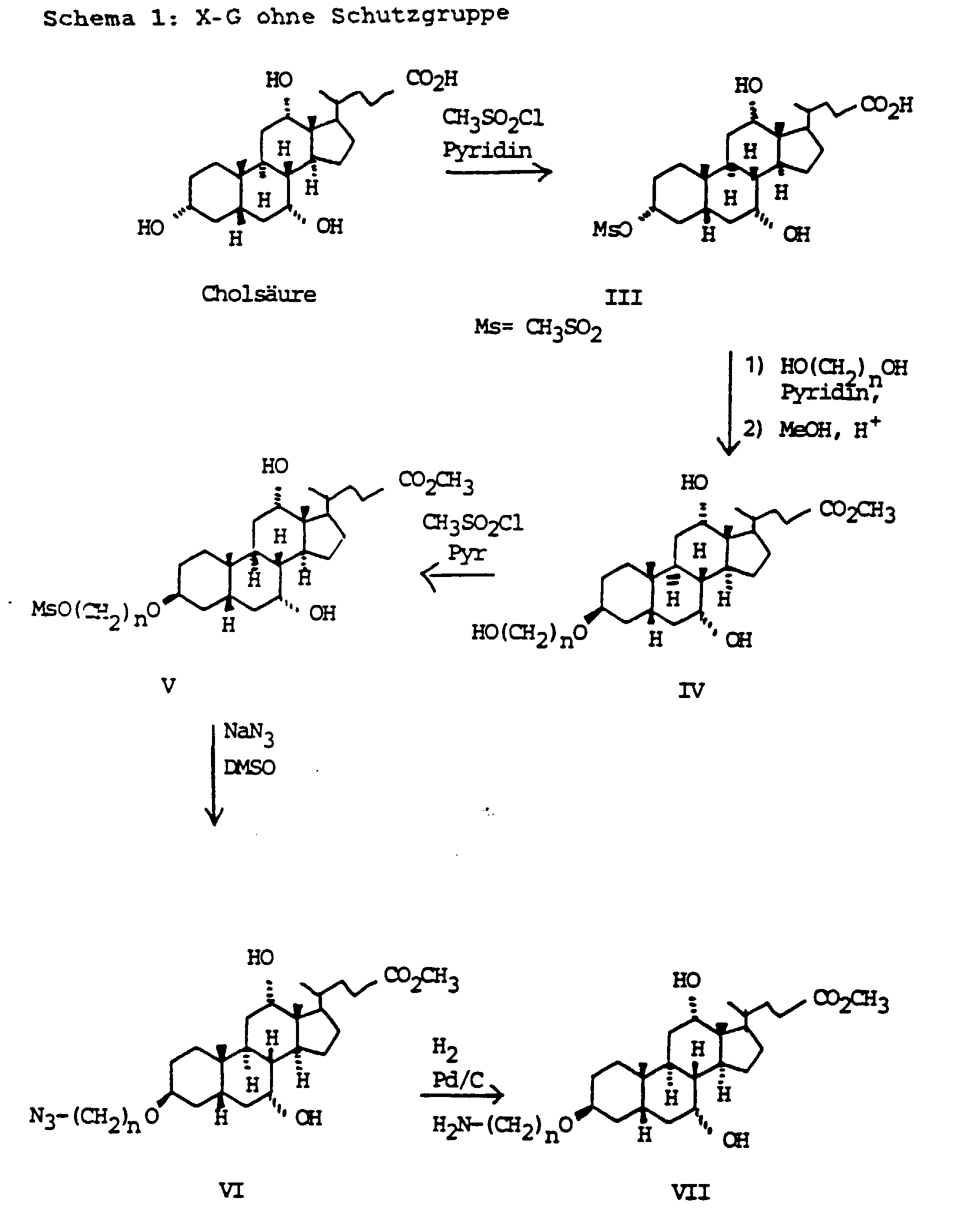

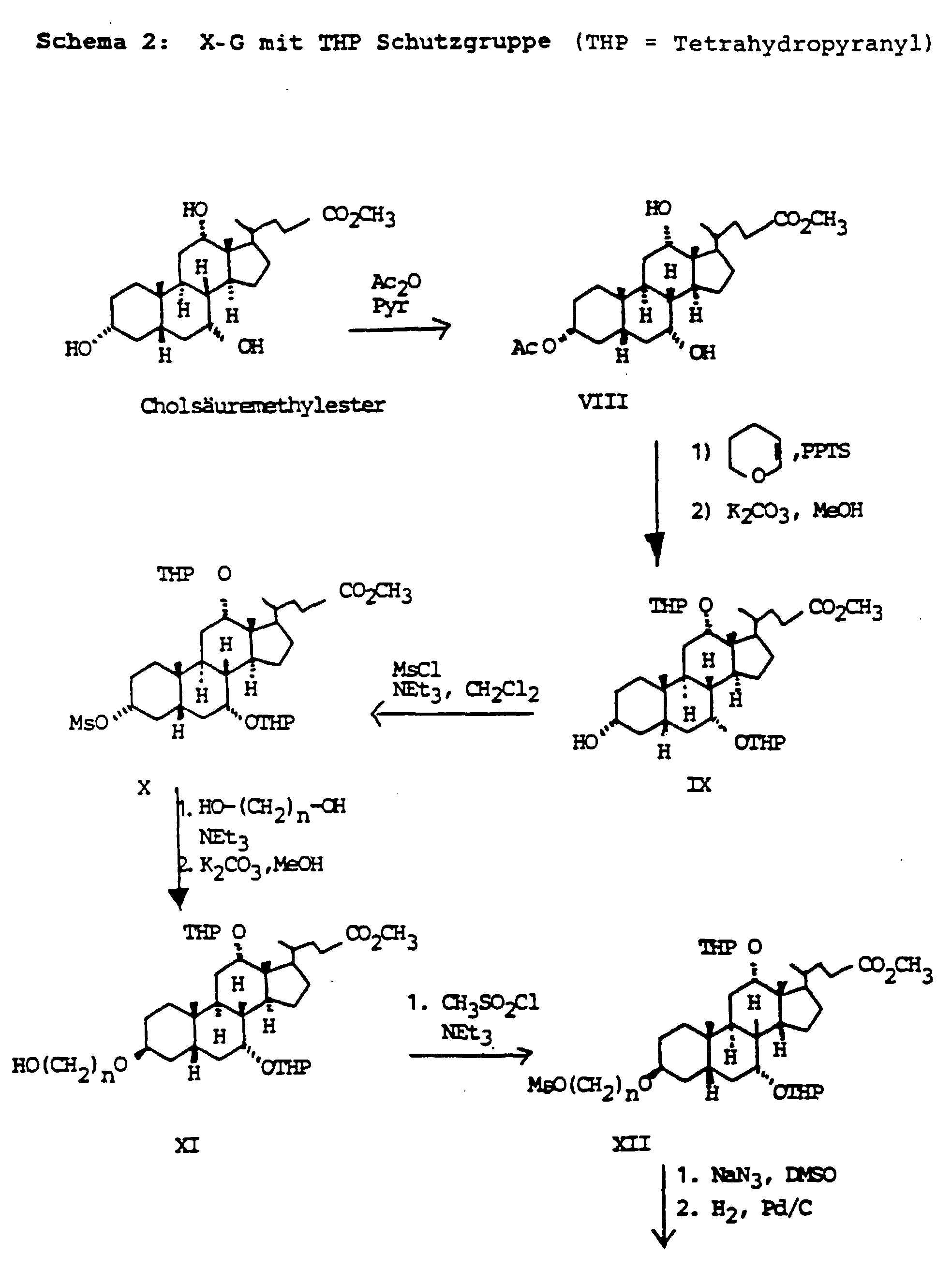

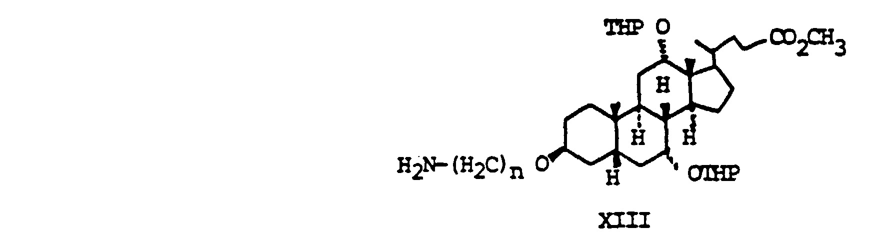

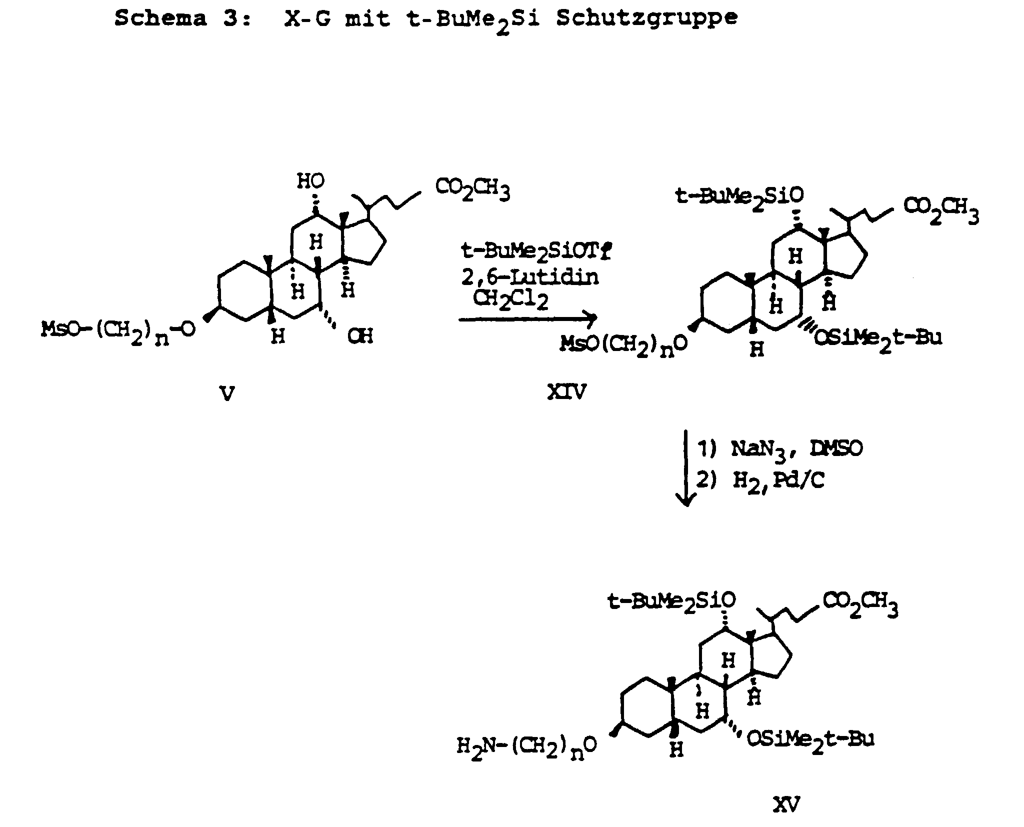

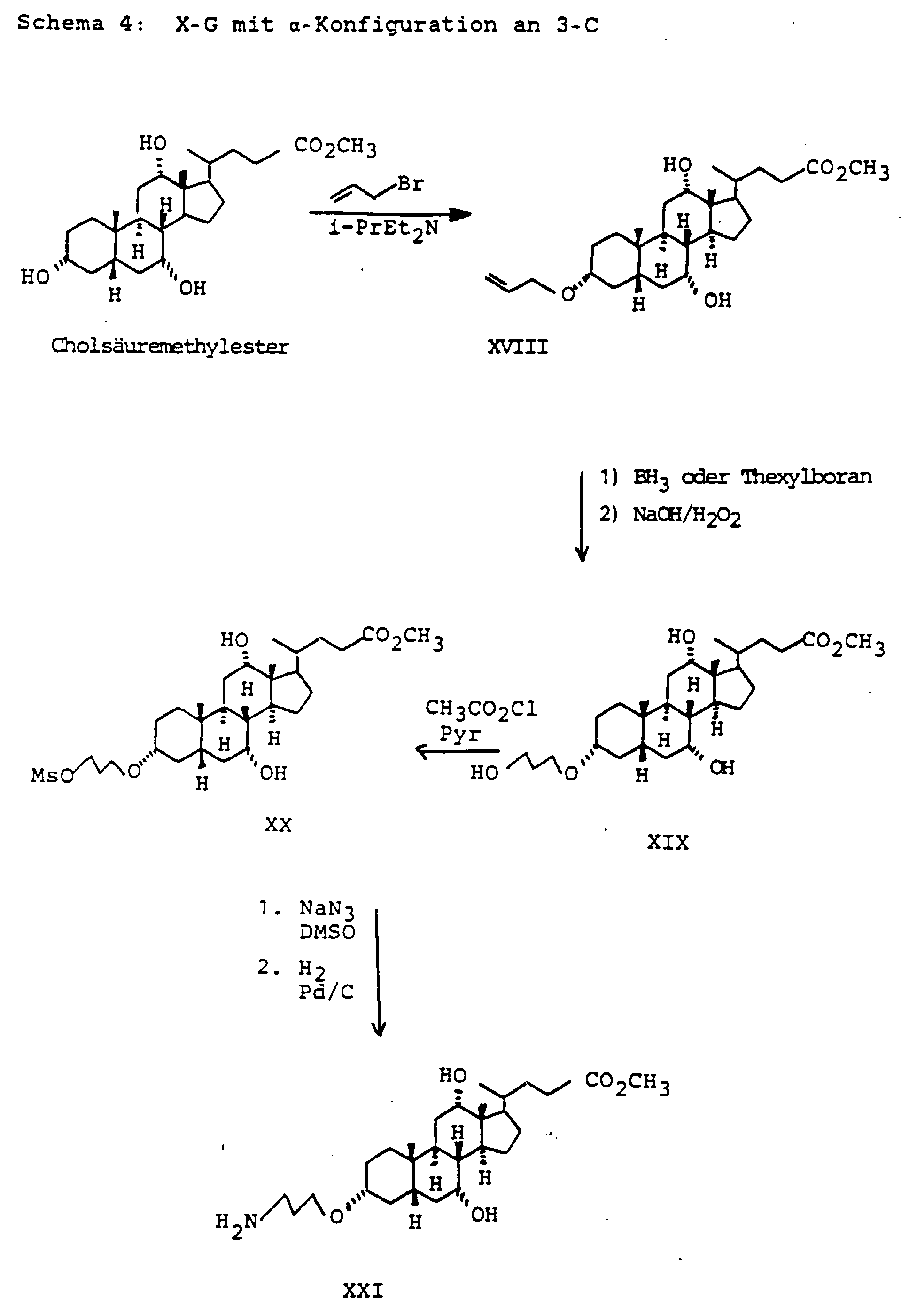

Der Austausch der 3-OH Gruppen durch Diole HO(CH₂)ₙOH gelingt durch Umsetzung der entsprechenden Mesylate mit den entsprechenden Diolen, die man bevorzugt im Überschuß einsetzt, unter Zusatz von Basen wie Pyridin, Lutidin, aber auch Triethylamin.

Die primäre OH-Gruppe der Verbindungen IV und XI lassen sich nach Standardverfahren weiter umsetzen. So kann z.B. XI mit Oxidationsmitteln in die entsprechende Carbonsäure XVI [mit R(11) gleich THP] überführt werden, bevorzugt mit Chrom-VI-Reagenzien oder verschiedenen Kaliumpermanganat-Systemen. Entsprechend eignen sich auch andere Schutzgruppen.

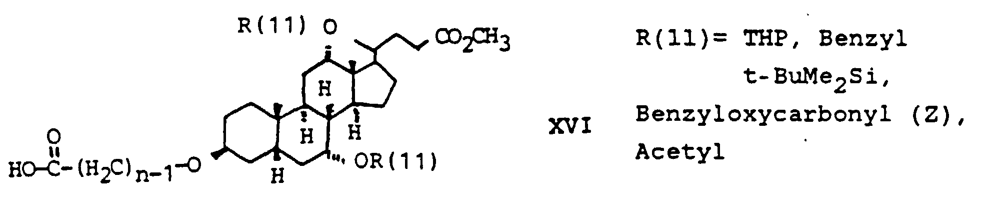

Die Amine VII, XIII und XV können mit Bernsteinsäureanhydrid in geeigneten Lösungsmitteln, vorzugsweise Methylenchlorid, Toluol oder auch Pyridin in Carbonsäuren XVII überführt werden.

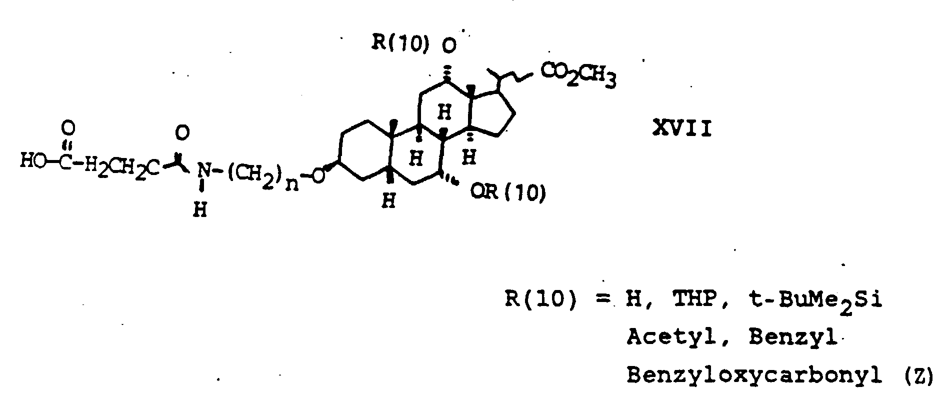

Schema 4 beschreibt die Herstellung von Gallensäurebausteinen mit 3α-Konfiguration. Zur Hydroborierung von XVIII eignen sich verschiedene Borane wie BH₃, Thexylboran oder 9-BBN. XVIII kann entweder wie in Schema 4 an den Alkoholgruppen ungeschützt oder auch geschützt mit THP, Acetyl, Benzyl u.a. eingesetzt werden.

### Verwendung als Arzneimittel

Gallensäuren spielen eine wichtige physiologische Rolle bei der Verdauung von Lipiden. Sie werden aus der Leber über die Gallenblase in den Darm abgegeben und entfalten dort ihre physiologische Wirkung bei der Verdauung von Lipiden. Der größte Teil der sezernierten Gallensäuren wird über den enterohepatischen Kreislauf wieder zurückgewonnen. Sie gelangen über die Mesenterialvenen des Dünndarms und das Pfortadersystem wieder zur Leber zurück. Bei der Rückresorption im Darm spielen sowohl aktive als auch passive Transportprozesse eine Rolle. Im enterohepatischen Kreislauf treten die Gallensäuren sowohl als freie Säuren, aber auch in Form von Glycin- und Taurinkonjugaten in Erscheinung.

Es wurde gefunden, daß die erfindungsgemäßen Verbindungen I resorbiert werden und in die Blutbahn gelangen. Dadurch ist es möglich, unter Ausnutzung der natürlichen Rückresorptionsmechanismen der Gallensäuren, eine verbesserte Resorption von nicht oder schwer resorbierbaren Pharmaka zu erreichen.

Darüberhinaus besitzt dieses System eine weitere wichtige Eigenschaft: Es läßt sich für Arzneimittel und zwar für nicht resorbierbare und für resorbierbare eine organselektive Wirkung erzielen nämlich bezüglich solcher Organe, die Transportmechanismen für Gallensäuren besitzen, wie es z.B. in den Geweben des enterohepatischen Kreislaufs der Fall ist (z.B. hepatotrope Wirkung). Dadurch lassen sich die systemischen Nebenwirkungen einer Reihe von Arzneimitteln gezielt vermindern oder sogar verhindern.

Eine verbesserte Resorption bzw. eine organselektive Wirkung ist für eine Reihe von Pharmaka wünschenswert wie z.B. Peptide, Antibiotika, antivirale Substanzen, Krebsmittel, Leberschutzmittel, Antihyperlipidämika, Diuretika, Blutdrucksenker, Renininhibitoren, Prolylhydroxylase-Hemmer, Antidiabetika.

Die pharmakologisch wirksamen Moleküle können ihre Aktivität auf verschiedene Weise entfalten:
- in der erfindungsgemäß verknüpften Form W-X-G
- nach Abspaltung des Gallensäurerestes mit einem Verbindungsglied X
- in einer freien Form W ohne X und G
- in den drei genannten Fällen gleichzeitig

Die Verbindungen der Formel I werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 5000 mg, vorzugsweise jedoch in Dosisbereich 10-500 mg. Die erfindungsgemäßen Verbindungen können zur Anwendung kommen gelöst oder suspendiert in pharmakologisch unbedenklichen organischen Lösungsmitteln, wie ein- oder mehrwertigen Alkoholen, wie z.B. Ethanol oder Glycerin, in Triacetin, Ölen wie z.B. Sonnenblumenöl, Lebertran, Ethern, wie z.B. Diethylenglykoldimethylether oder auch Polyethern wie z.B. Polyethylenglykol, oder auch in Gegenwart anderer pharmakologisch unbedenklicher Polymerträger, wie z.B. polyvinylpyrrolidon, oder anderen pharmazeutisch annehmbaren Zusatzstoffen wie Stärke, Cyclodextrin oder Polysacchariden. Ferner können die erfindungsgemäßen Verbindungen in Kombination mit anderen Arzneistoffen gegeben werden.

Die Erfindung betrifft ferner Zwischenprodukte der Formel XXII

SG-X-G XXII

in der
SG gleich H oder eine Schutzgruppe ist,
G 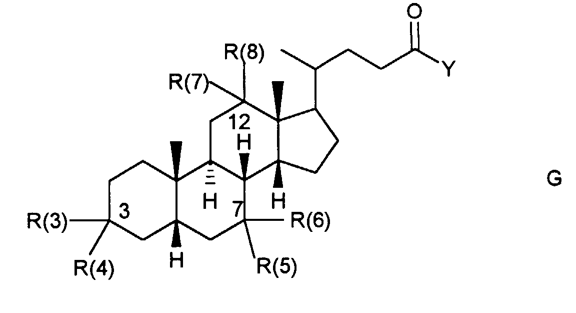 in welcher R(3) - R (8) gleich oder verschieden sind und folgende Bedeutung haben: eine Bindung zu SG-X-, wobei insgesamt bis zu zwei SG-X-Einheiten gebunden sein können,
R(3) und R(4), R(5) und R(6), R(7) und R(8) jeweils gemeinsam der Sauerstoff einer Carbonylgruppe, 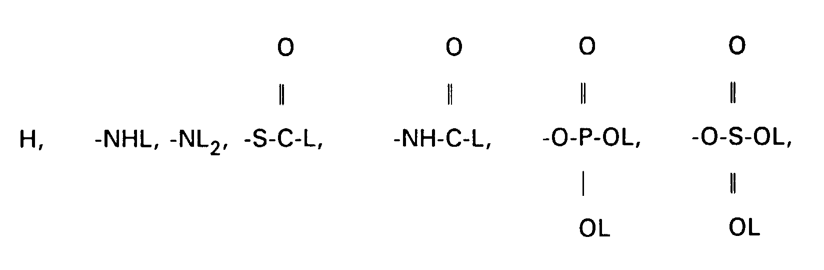 -L, wobei L bedeutet:
H, einen gesättigten oder ungesättigten Alkylrest mit 1 - 10-C-Atomen, der verzweigt und unverzweigt ist, Cycloalkyl mit 3 - 8 C-Atomen, einen Phenylrest (der unsubstituiert oder 1 - 3fach substituiert ist mit F, Cl, Br, (C₁-C₄)-Alkyl oder (C₁-C₄)-Alkoxyl), einen Benzylrest (der unsubstituiert oder 1 - 3 fach substituiert ist mit F, Cl, Br, (C₁-C₄)-Alkyl oder (C₁-C₄)-Alkoxyl),
und in der Y folgende Bedeutung hat:
-OH oder eine über die Aminogruppe gebundene Aminosäure oder
Aminosulfonsäure und deren Alkali- und Erdalkalisalze, -OKa, wobei Ka ein Kation bedeutet wie z. B. ein Alkali oder Erdalkaliion oder auch ein quartäres Ammoniumion,
das Verbindungsglied X 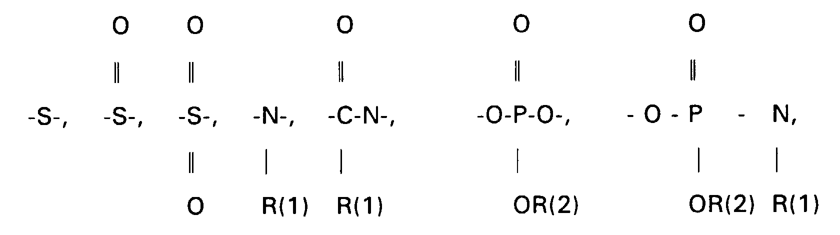 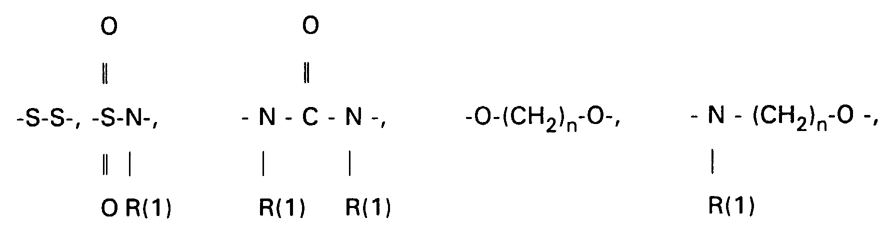 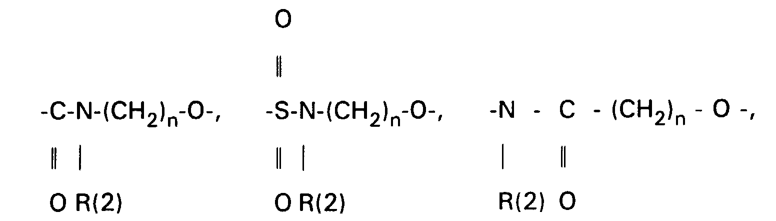 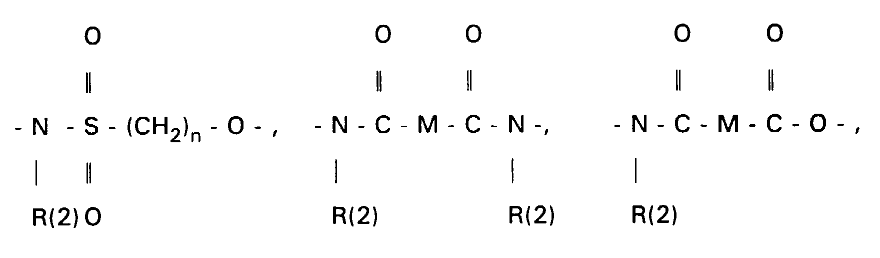 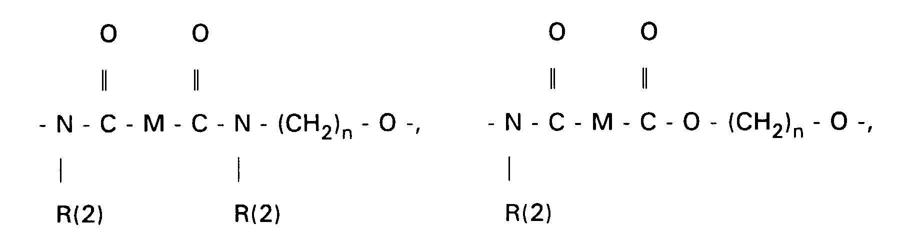 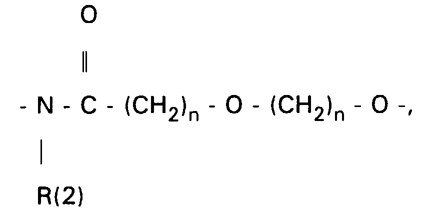 mit
- R(1) =: H, (C₁-C₈)-Alkyl; die Gruppe 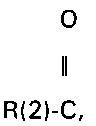 Phenyl, Benzyl, unsubstituiert oder im Kern 1 - 3 fach substituiert mit F, Cl, Br, (C₁-C₄)-Alkyl, Alkoxy mit 1 - 4 C-Atomen;
- R(2) =: H, (C₁-C₈)-Alkyl, Phenyl, Benzyl; jeweils unsubstituiert oder 1 - 3 fach substituiert mit F, Cl, Br, (C₁-C₄)-Alkyl oder Alkoxy mit 1 - 4 C-Atomen;
- n =: 1 - 16;
- M =: -(CH₂)ₘ- mit m = 2.

Diese Zwischenprodukte werden für die Erfindung meistens als Verbindungen XXII mit freier NH₂- oder OH-Gruppe SG eingesetzt, wenn sie zu Verbindungen I W-X-G umgesetzt werden sollen.

Die Schutzgruppe SG ist in der Hauptsache während der Synthese der Verbindungen I und XXII vorhanden, wird aber meistens vor der Umsetzung von XXII zu I in ihre Form SG gleich Wasserstoff umgewandelt.

Die Zwischenprodukte SG-X-G sind sowohl für die Herstellung der Verbindungen I von großer Bedeutung, als auch für die Synthese anderer Endprodukte, z.B. von Polymeren mit Vinylacetat, welche die Gruppe -X-G enthalten.

Bevorzugt sind Zwischenprodukte XXII mit
G wie oben definiert
SG gleich H und
X gleich -O(CH₂)₂₋₁₀-O-,
   -HN-(CH₂)₂₋₁₀-O-
   -O(CH₂)₂₋₄-O(CH₂)₂₋₄-O-
   -HN-(CH₂)₂₋₄-O-(CH₂)₂₋₄-O-

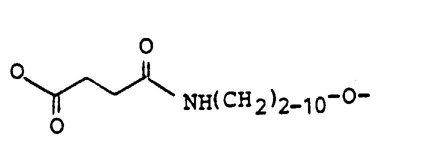

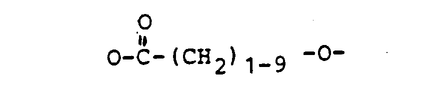

Besonders bevorzugt sind Verbindungen XXII mit
G wie oben definiert, SG gleich H und
X gleich -O(CH₂)₂₋₄-O- und
   -HN-(CH₂)₂₋₁₀-O-.

* Die Gallensäurederivate der Beispiele 11-46, 48-63 sowie 69-78 fallen nicht unter den Anspruchswortlaut.

### Beispiele *

### 1. W-X-G, X = eine direkte Bindung

### Beispiel 1

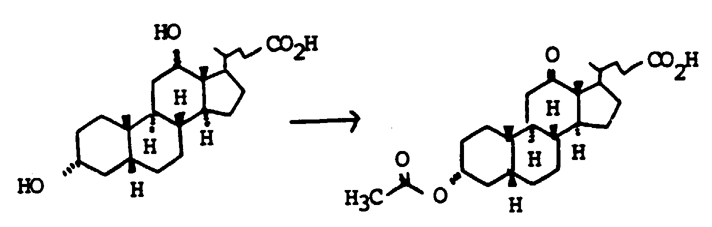

20 g (51 mmol) Desoxycholsäure wurden in 50 ml Dioxan und 20 ml Pyridin vorgelegt und bei Zimmertemperatur mit 30 ml Acetanhydrid versetzt. Nach 3 Tagen wurde eingedampft und der Rückstand in 300 ml Essigsäure gelöst. Zur Oxidation wurden 14 g Kaliumchromat in 60 ml Wasser zugegeben. Man ließ 3 Tage bei Zimmertemperatur stehen. Zur Aufarbeitung wurden 4 l Wasser zugesetzt und mit Ether extrahiert (3 x). Die vereinigen organischen Phasen wurden mit Natriumsulfat getrocknet und eingedampft. Den Rückstand löste man in 200 ml 1n wäßriger KOH. Nach 24 h wurde das Produkt mit 1n HCl ausgefällt. Chromatographie auf Kieselgel (Cyclohexan/ Essigester/Essigsäure = 40/100/1) ergab 14.0 g (35.9 mmol, 70 %) "Beispiel 1".

### Beispiel 2

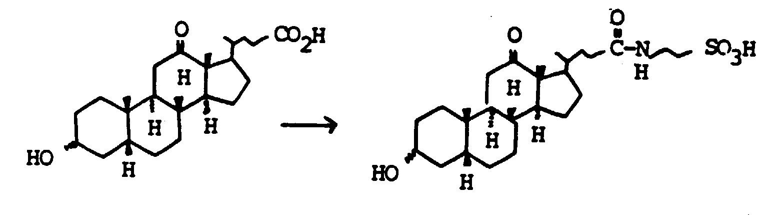

14.0 g (35.9 mmol) "Beispiel 1" wurden in 100 ml Dioxan gelöst und mit 8.3 ml Tri-n-butylamin versetzt. Anschließend wurden 3.75 ml Chlorameisensäureethylester zugetropft. Nach 30 min bei Zimmertemperatur gab man 4.75 g Taurin, gelöst in 38 ml 1n NaOH, zu. Nach 24 h Rühren bei Zimmertemperatur wurde eingedampft und der Rückstand zwischen 400 ml 1n HCl und Ether verteilt. Die wäßrige Phase wurde dreimal mit Ether extrahiert. Die vereinigten organischen Phasen wurden eingedampft und der Rückstand mit heißem Ethanol aufgenommen, filtriert und eingeengt, wobei das Produkt kristallisierte.
Ausbeute: 9.5 g "Beispiel 2"

### Beispiel 3

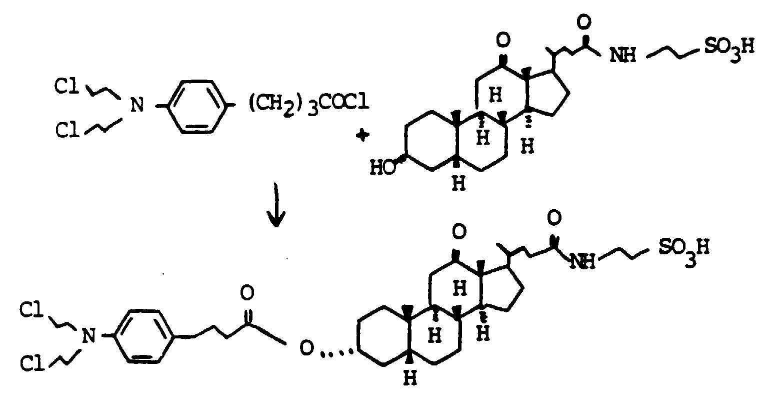

500 mg Chlorambucil wurden in 25 ml Dioxan mit 1,5 ml Oxalylchlorid bei Zimmertemperatur in Gegenwart von 2 g Molekularsieb (4Å) innerhalb 24 h in das Säurechlorid überführt. Es wurde eingedampft und der Rückstand mit 15 ml trockenem Dioxan aufgenommen. Diese Lösung wurde unter N₂ innerhalb 30 min zu einer siedenden Lösung von 900 mg Beispiel 2 in 60 ml Dioxan getropft. Nach 5 h Rückfluß wurde eingedampft und der Rückstand auf Kieselgel chromatographiert (CHCl₃/MeOH/HOAc = 12/1/2)
Ausbeute: 250 mg "Beispiel 3"

### Beispiel 4

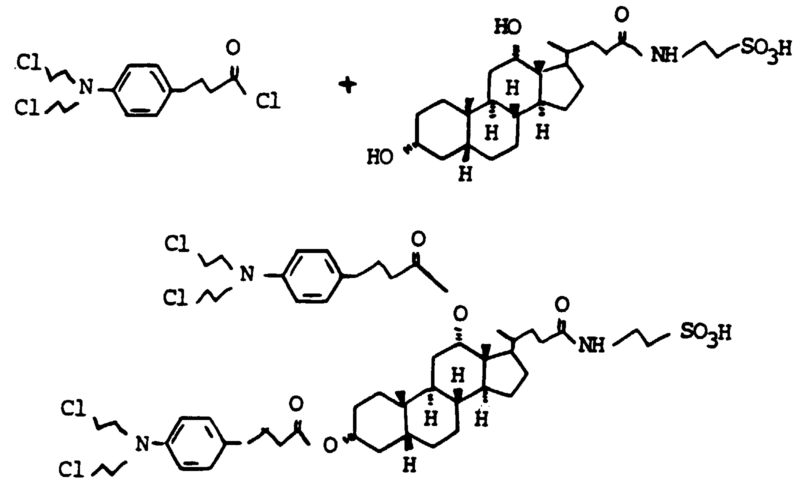

Wie in Beispiel 3 beschrieben wurde eine Lösung des Säurechlorids von Chlorambucil aus 500 mg Chlorambucil hergestellt und in gleicher Weise mit 400 mg Desoxytaurocholsäure umgesetzt. Chromatographie auf Kieselgel (Essigester/Cyclohexan/HOAc = 100/90/1; dann CHCl₃/MeOH = 5/1) ergab 350 mg "Beispiel 4".

### Beispiel 5

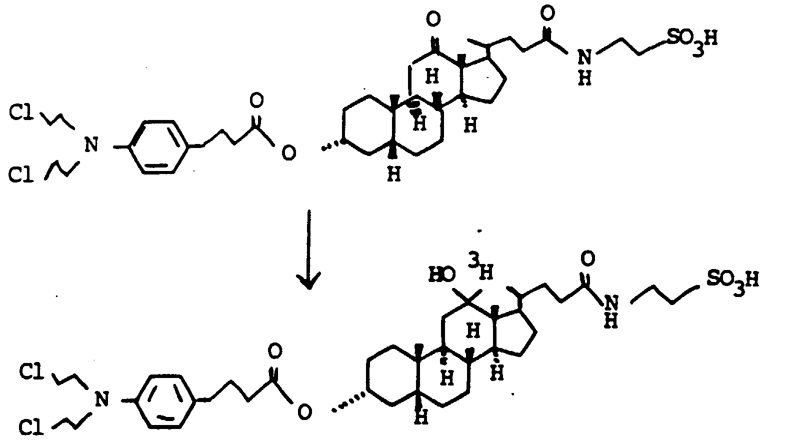

5 mg "Beispiel 3" wurden in 150 µl Dioxan/15 µl 100 mmol Natriumphosphatpuffer pH 7.4 gelöst und zu 100 mCi Na[³H]BH₄ (11.8 Ci/mmol) gegeben. Nach 2 h bei Zimmertemperatur wurden 20 µl 5n HCl zugegeben. Präparative DC (HPTLC-DC-Platte, 0.5 mm, Butanol/HOAc/H₂O = 9/1/2) ergab 2 mCi "Beispiel 5".

### Beispiel 6

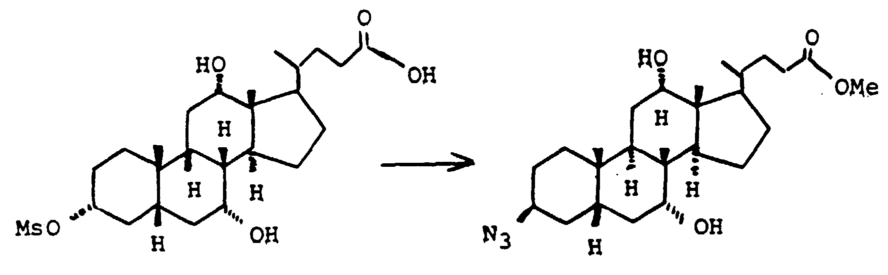

a) Eine Lösung von 43.6 g (89.6 mmol) "Beispiel 10" in 1 l trockenem DMF wurde mit 6.2 g (95.4 mmol) Natriumazid versetzt und 45 min. bei 130°C gerührt. Nach dem Abkühlen wurde auf Wasser gegossen und mit Diethylether (3x) extrahiert. Die vereinigten organischen Phasen wurden mit MgSO₄ getrocknet und eingedampft.
b) Die Veresterung wurde analog zu "Beispiel 11b" durchgeführt. Chromatographie auf Kieselgel (Cyclohexan/Essigester 1:1) ergab 18.9 g (42.2 mmol, 47 %) Azid "Beispiel 6".
   C₂₅H₄₁N₃O₄ (447) MS (FAB, 3-NBA/LiCl): 454 (M+Li⁺)

### Beispiel 7

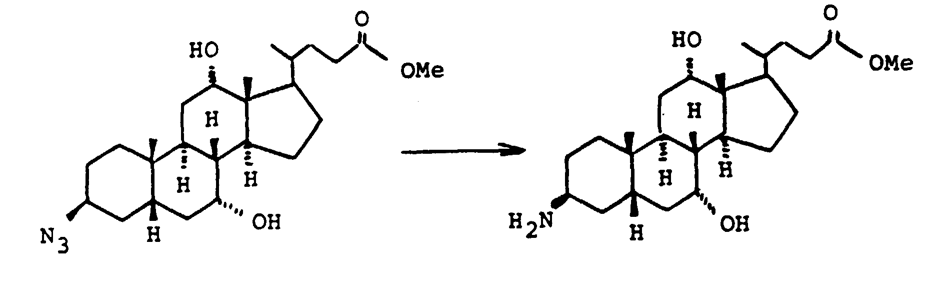

3.0 g (6.7 mmol) "Beispiel 6" wurden in 100 ml MeOH gelöst und mit 2 g Pd/C bei Zimmertemperatur und Normaldruck hydriert. Der Katalysator wurde abfiltriert und das Filtrat eingedampft. Chromatographie auf Kieselgel (MeOH/NEt₃ 95:5) ergab 1.6 g (3.8 mmol, 57 %) Amin "Beispiel 7".
C₂₅H₆₃N O₄ (421) MS (FAB) : 422 (M+H⁺)

### Beispiel 8

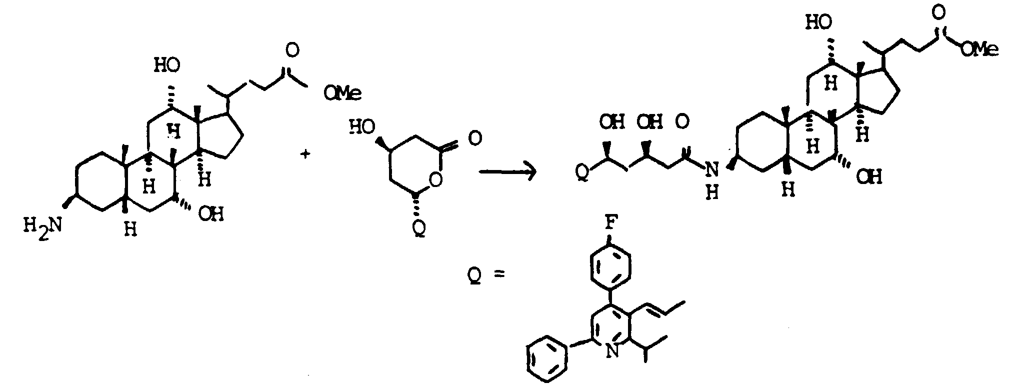

Ein Gemisch aus 500 mg (1.19 mmol) Beispiel 7, 10 ml Triethylamin, 200 mg (1.61 mmol) 4-Dimethylaminopyridin und 520 mg (1.20 mmol) Lacton "Beispiel 125" (Herstellung siehe DE 38 23 045-A, US 4 925 852) in 25 ml trockenem THF wurde 48 h unter Rückfluß erhitzt. Das Lösungsmittel wurde abgedampft und der Rückstand auf Kieselgel chromatographiert (Chloroform/Methanol 9:1). Ausbeute 520 mg (0.61 mmol, 51 %) Beispiel 8".
C₅₂H₆₉FN₂O₇ (852), MS (FAB, 3-NBA/LiCl): 859 (M+Li⁺)

### Beispiel 9

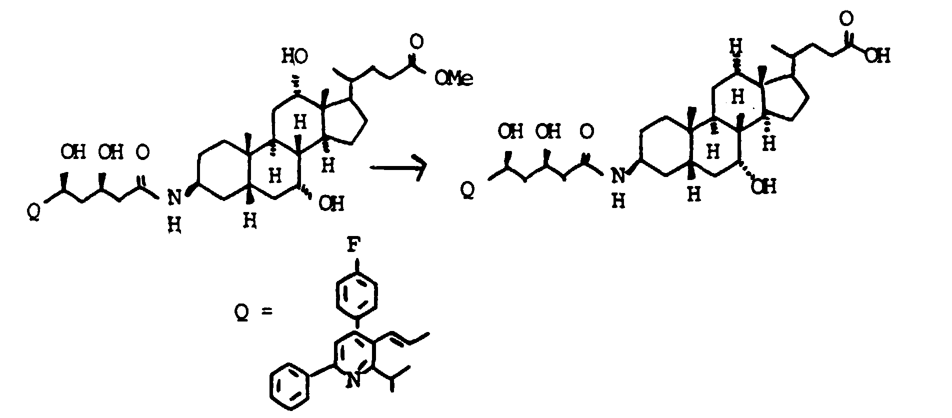

Analog zu den Beispielen 79-88 wurde "Beispiel 9" erhalten.
C₅₁H₆₇FN₂O₇ (840), MS (FAB, 3-NBA/LiCl): 847 (M+Li⁺).

### 2. X-G, X = ein Zwischenglied

### Beispiel 10

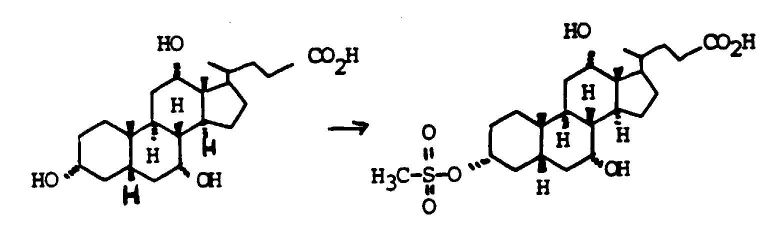

Zu 100 g (0.245 mol) Cholsäure in 500 ml Pyridin wurden bei 0°C 23.1 ml (0.294 mol) Methansulfonsäurechlorid getropft. Man ließ 30 min bei 0°C und 2 h bei Zimmertemperatur rühren. Die Mischung wurde auf 3000 ml Wasser/400 ml conc. H₂SO₄ gegossen und mit Essigester extrahiert (3 x). Die vereinigten organischen Phasen wurden mit MgSO₄ getrocknet und eingedampft. Chromatographie auf Kieselgel (Essigester/Cyclohexan/HOAc= 5:5:1) ergab quantitativ "Beispiel 10". Für präparative Zwecke war eine weitere Reinigung nicht erforderlich.

### Beispiel 11

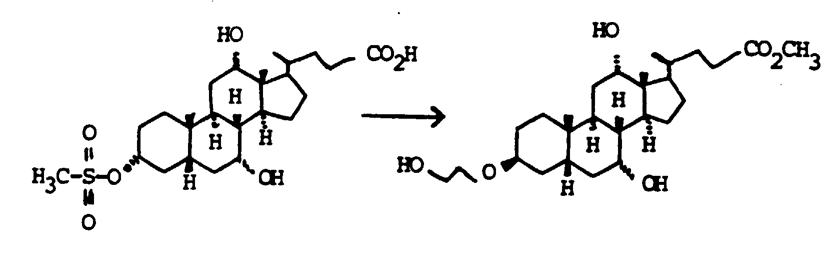

a) 119 g (0.245 mol) "Beispiel 10" wurden in 500 ml Ethylenglykol/100 ml Pyridin 2 h auf 100°C erhitzt. Die Mischung wurde auf 1500 ml Wasser/100 ml conc. H₂SO₄ gegossen und mit Essigester extrahiert (3x). Die vereinigten organischen Phasen wurden getrocknet (MgSO₄) und eingedampft.
b) Zur Veresterung wurde der Rückstand in 1100 ml methanolischer HCl (hergestellt durch Zutropfen von 100 ml Acetylchlorid zu 1000 ml Methanol) gelöst und über Nacht bei Zimmertemperatur gerührt. Die Lösung wurde auf 2000 ml Wasser gegossen und mit Ether extrahiert (3x). Die vereinigten organischen Phasen wurden mit gesättigter wäßriger NaHCO₃-Lösung gewaschen und getrocknet (MgSO₄). Abdampfen des Lösungsmittels und Flash-Chromatographie auf Kieselgel (Essigester dann Essigester/MeOH = 10/1) ergab 37.1 g (0.08 mol, 33 %) "Beispiel 11".
   C₂₇H₄₆O₆ (466), MS (FAB, 3-NBA/LiJ): 473 (M+Li⁺)

Das Produkt enthält bis zu 10 % des 3α-Isomeren, das gegebenenfalls nach entsprechender Derivatisierung abgetrennt werden kann.

Analog zu Beispiel 11 wurden die Verbindungen der Tabelle 1 hergestellt.
(Neben kleineren Anteilen der α-Isomeren wurden überwiegend die β-Isomeren erhalten).

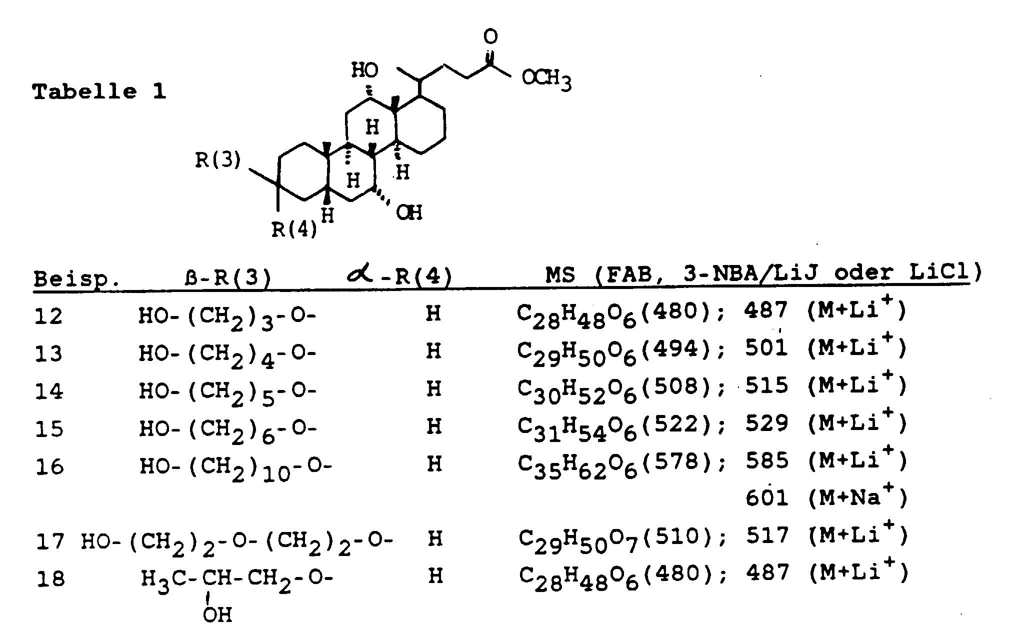

### Beispiel 19

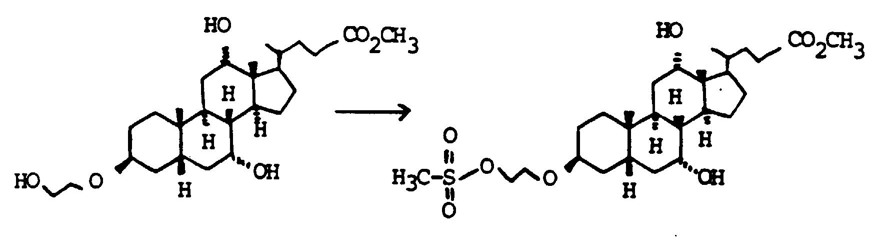

Zu 37.1 g (0.08 mol) "Beispiel 11" in 150 ml Pyridin wurden bei 0°C 6.6 mol (0.084 mol) Methansulfonsäurechlorid getropft. Man ließ 15 min bei 0°C und 1 h bei Zimmertemperatur rühren. Die Reaktionsmischung wurde auf 500 ml Wasser gegossen und mit Essigester extrahiert (3x). Trocknen der vereinigten organischen Phasen (MgSO₄), Abziehen des Lösungsmittels und Chromatographie auf Kieselgel (Essigester/Cyclohexan = 3/1) ergab 37.7 g (0.07 mol, 87 %) Mesylat "Beispiel 19".
C₂₈H₄₈O₈S (544), MS (FAB, 3-NBA/LiJ): 551(M+Li⁺).

### Beispiel 20

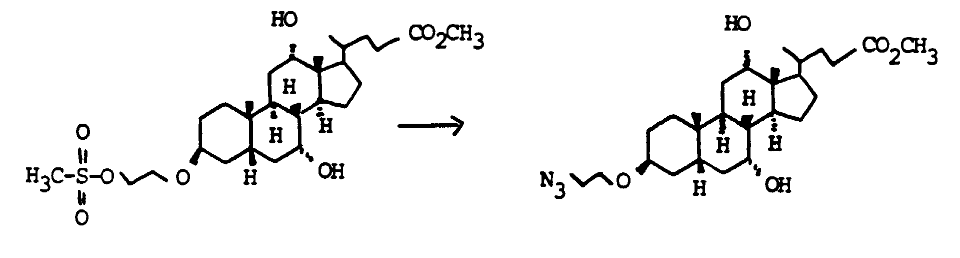

37.7 g (0.07 mol) Mesylat "Beispiel 19" wurden mit 4.95 g (0.076 mol) Natriumazid in 150 ml trockenem DMSO 2 h bei 70°C gerührt. Das Reaktionsgemisch wurde auf Wasser gegossen und mit Essigester extrahiert (3x). Die vereinigten organischen Phasen wurden getrocknet (MgSO₄) und eingedampft. Der Rückstand wurde mit Toluol aufgenommen und das Toluol wieder im Rotationsverdampfer abgezogen (2x). Ausbeute 34.5 g "Beispiel 20" (quantitiativ). Das Azid wurde ohne weitere Reinigung unmittelbar zur nächsten Stufe umgesetzt.

### Beispiel 21

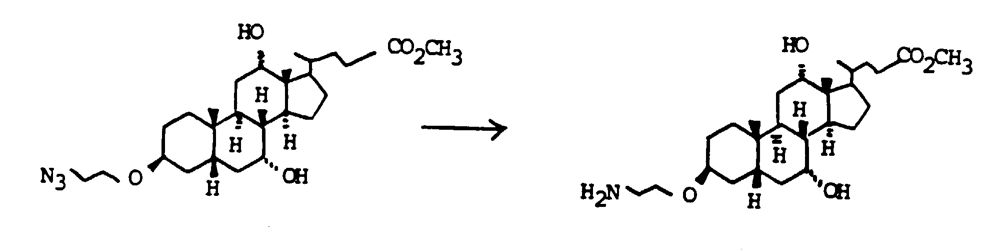

31.1 g (0.063 mol) "Beispiel 20" wurden in 500 ml Essigester mit 20 g Pd/C (10 %) bei Zimmertemperatur und Normaldruck hydriert. Der Katalysator wurde abfiltriert und das Filtrat eingedampft. Chromatographie auf Kieselgel (Essigester/ Methanol/NEt₃ = 5/1/1) ergab 21.0 g (0,045 mol, 71 %) Amin "Beispiel 21".
C₂₇H₄₇NO₅ (465), MS (FAB, 3-NBA/LiJ): 472 (M+Li⁺).

Analog zu den Beispielen 19-21 wurden die Verbindungen der Tabelle 2 hergestellt.

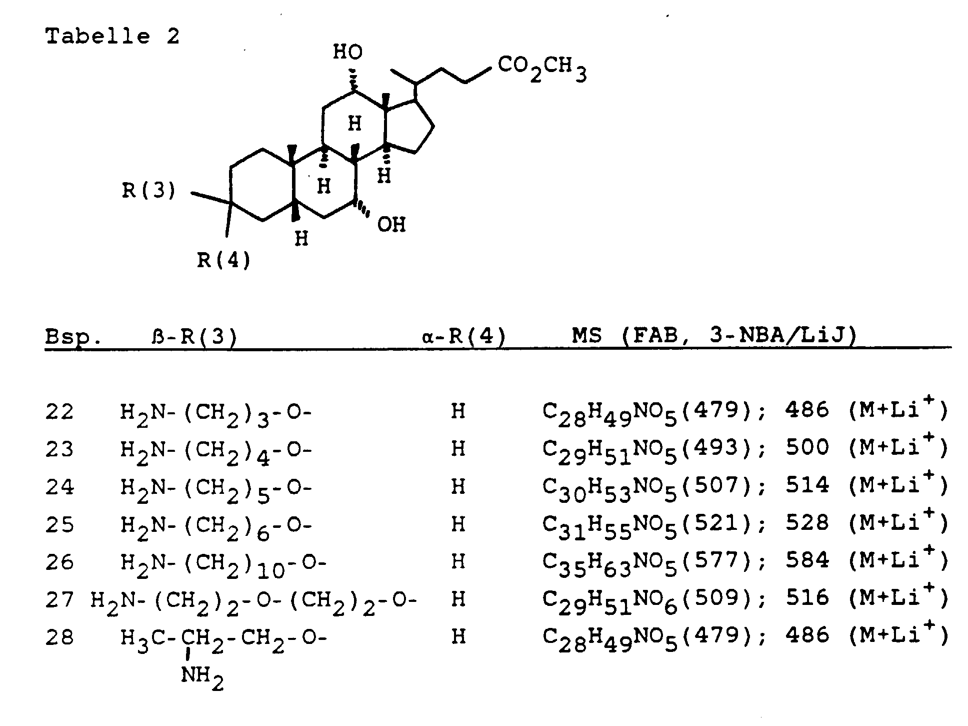

In Analogie zur Cholsäure wurden andere Gallensäuren entsprechend den Beispielen 10-28 umgesetzt und Verbindungen entsprechend Tabelle 3 erhalten.
a) ausgehend von Desoxycholsäure: 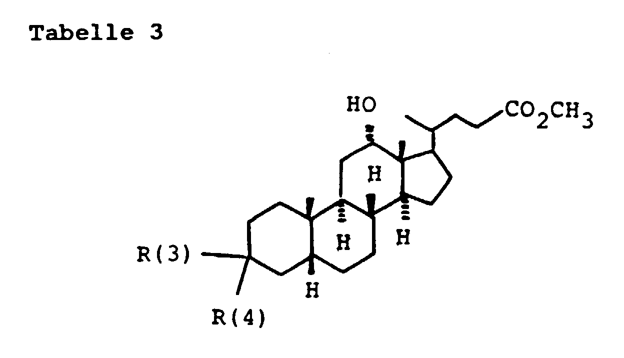 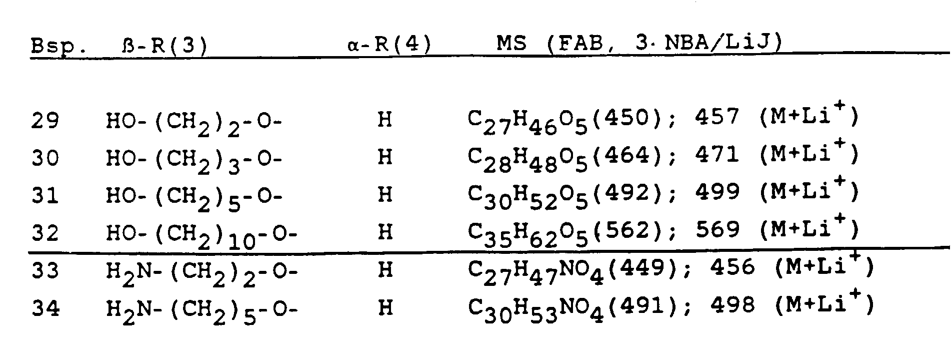
b) ausgehend von Chenodesoxycholsäure 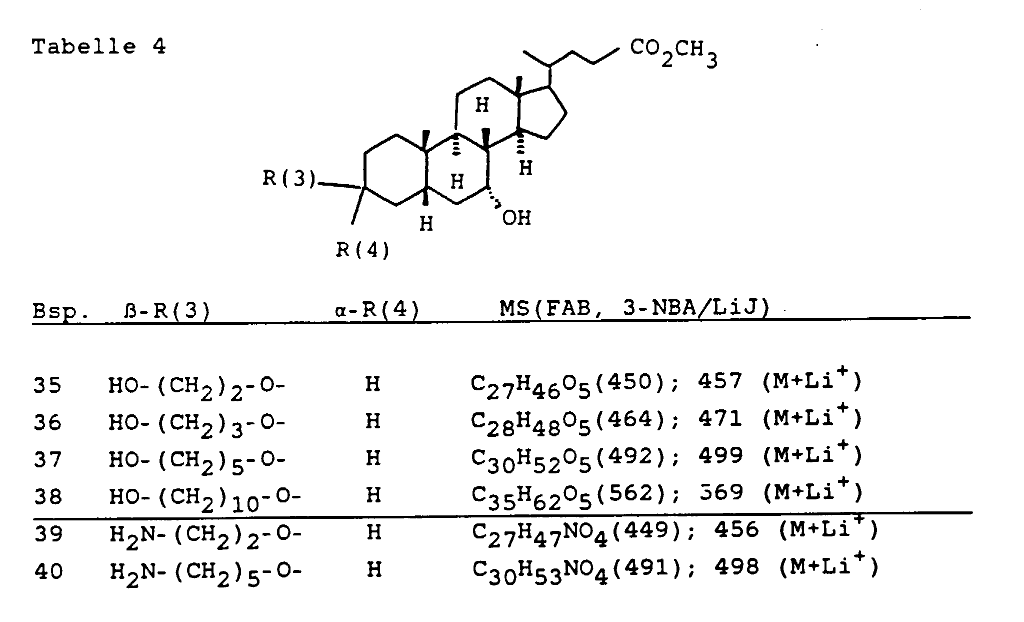
c) ausgehend von Lithocholsäure

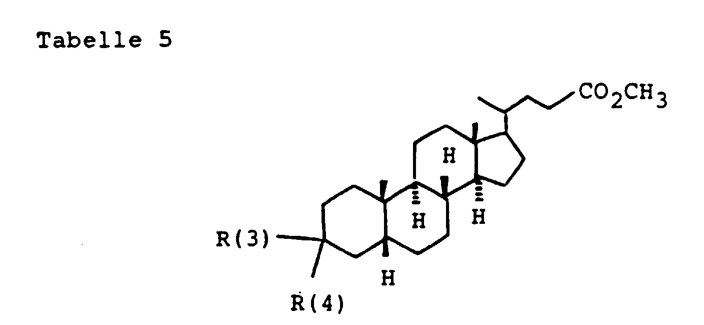

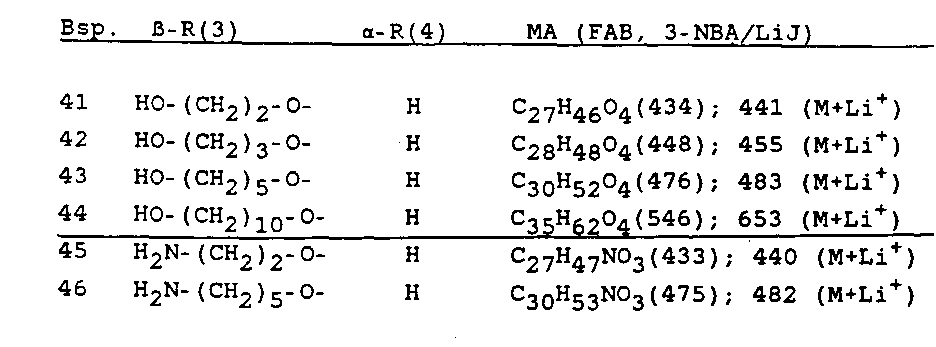

### Beispiel 47

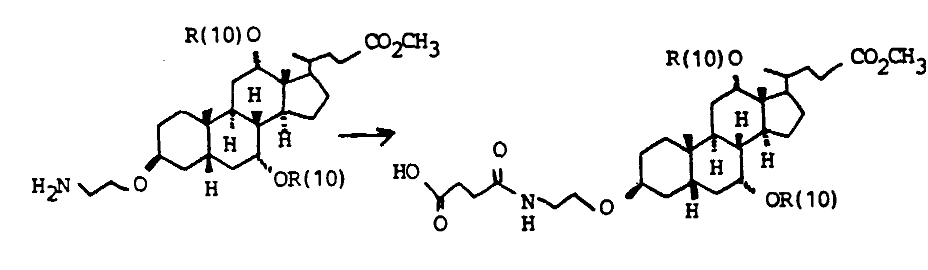

### Beispiel 47 a) (R(10) = H)

2.0 g (4.3 mmol) "Beispiel 21" wurden in 25 ml THF/5 ml Triethylamin mit 430 mg (4.3 mmol) Bernsteinsäureanhydrid 30 min bei Zimmertemperatur gerührt. Das Reaktionsgemisch wurde auf 2n HCl gegossen und mit Essigester extrahiert (3x). Trocknen der vereinigten organischen Phasen (MgSO₄) und Abziehen des Lösungsmittels ergab 2.4 g (4.2 mmol, 98 %) "Beispiel 47a" (R(10) = H)
C₃₁H₅₁NO₈ (565) : MS (FAB, 3-NBA/LiJ): 578 (M+2Li⁺-H)

### Beispiel 47 b) (R(10) = t-BuMe₂Si)

Völlig analog zu 47 a) wurde "Beispiel 47 b)" aus Beispiel 58 gewonnen.

### Beispiel 47 c) (R(10) = Tetrahydropyranyl = THP)

Völlig analog zu 47 a) wurde "Beispiel 47 c)" aus Beispiel 55 gewonnen.

### Beispiel 48

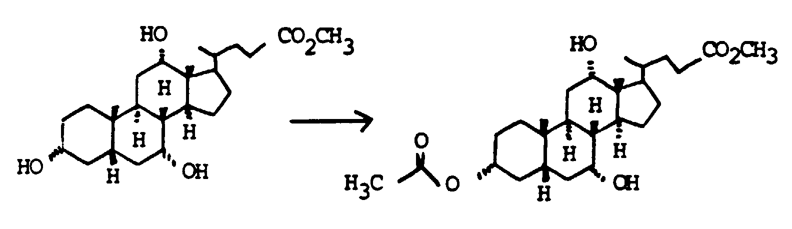

Zu 100 g (0.237 mol) Cholsäuremethylester in 750 ml Pyridin wurden bei 0°C 20.3 ml (0.284 mol) Acetylchlorid getropft. Nach 2 h Rühren bei Zimmertemperatur gab man bei 0°C nochmals 3.4 ml (0.047 mol) Acetylchlorid zu und ließ noch 1 h bei Zimmertemperatur rühren. Das Reaktionsgemisch wurde auf Eiswasser gegossen und mit Essigester extrahiert (3x). Die vereinigten organischen Phasen wurden getrocknet (MgSO₄) und eingedampft. Chromatographie des Rückstandes auf Kieselgel (Cyclohexan/Essigester = 1,5/1) ergab 95.8 g (0.206 mol, 87 %) Monoacetat "Beispiel 48".

### Beispiel 49

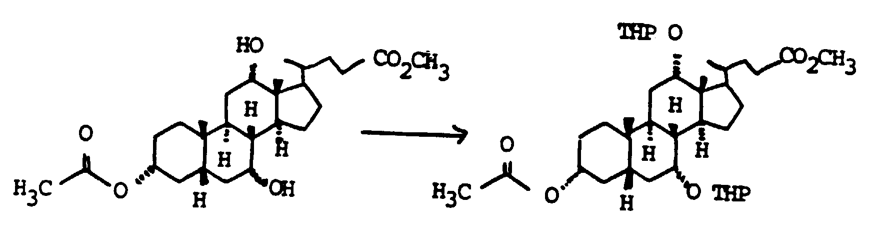

50.0 g (0.108 mol) Monoacetat "Beispiel 48" wurden in 250 ml Dichlormethan/250 ml Dihydropyran gelöst und bei Zimmertemperatur mit 10.0 g Pyridinium-(toluol-4-sulfonat) versetzt und zwei Tage bei Zimmertemperatur gerührt. Man verdünnte die Reaktionslösung mit 1500 ml Diethylether, wusch die organische Phase zweimal mit halbgesättigter wäßriger Kochsalzlösung und trocknete mit MgSO₄. Abziehen des Lösungsmittels ergab 75 g (quant.) Bis-THP-Ether "Beispiel 49", der ohne weitere Reinigung zur nächsten Stufe verwendet wurde.

### Beispiel 50

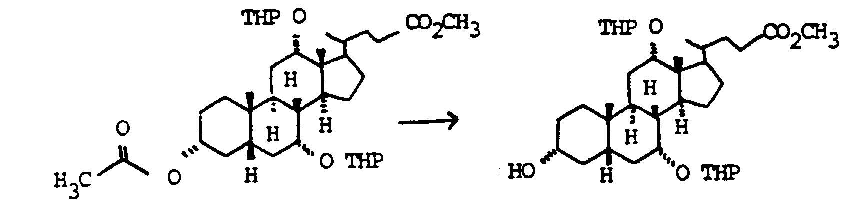

46.6 g (ca. 0.055 mol) "Beispiel 49" in 300 ml trockenem Methanol wurden bei Zimmertemperatur mit 37.8 g (0.275 mol) Kaliumcarbonat versetzt und 3 h gerührt. Das Lösungsmittel wurde weitgehend abgezogen und der Rückstand auf 2 n Salzsäure/Toluol gegossen. Die wäßrige Phase wurde zweimal mit Toluol extrahiert und die vereinigten organischen Phasen einmal mit Wasser und zweimal mit gesättigter wäßriger NaHCO₃-Lösung gewaschen. Trocknen mit MgSO₄, Abziehen des Lösungsmittels und Chromatographie auf Kieselgel (Cyclohexan/ Essigester = 3/2) ergab 28.8 g (0.049 mmol, 89 %) "Beispiel 50".
C₃₅H₅₈O₇ (590), MS (FAB, 3-NBA/LiJ): 597 (M+Li⁺)

### Beispiel 51

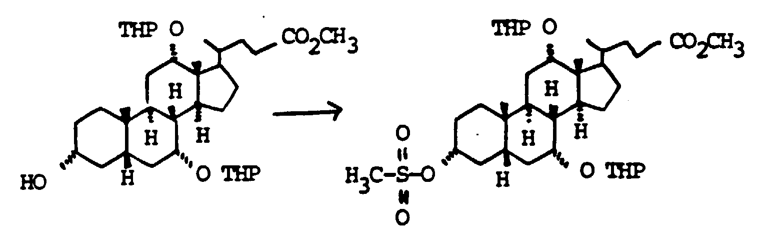

In Analogie zu Beispiel 10 wurden aus 28.8 g (0.048 mol) Beispiel 14 30.3 g (0.045 mol, 94 %) Mesylat "Beispiel 51" gewonnen.

### Beispiel 52

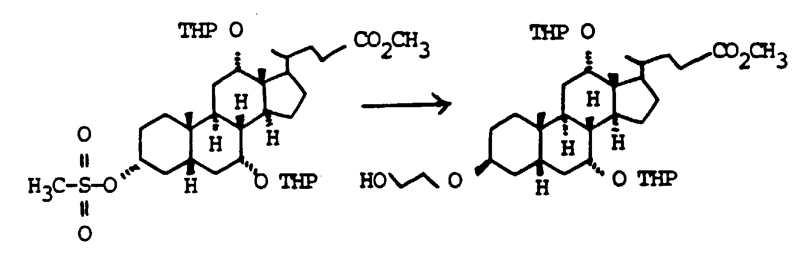

a) 46.0 g (0.068 mol) Mesylat "Beispiel 51" wurden mit 300 ml Ethylenglykol/75 ml Triethylamin 2.5 h unter Rückfluß gekocht. Das Reaktionsgemisch wurde auf 1n Salzsäure gegossen und mit Diethylether extrahiert (2x). Die vereinigten organischen Phasen wurden einmal mit Wasser, zweimal mit gesättigter wäßriger Natriumhydrogencarbonatlösung gewaschen, getrocknet (MgSO₄) und vom Lösungsmittel befreit. Der Rückstand wurde mit Toluol aufgenommen und eingedampft (2x).
b) Der Rückstand wurde in 500 ml trockenem Methanol gelöst mit 40.0 g Kaliumcarbonat versetzt und 1.5 h bei Zimmertemperatur gerührt. Die Reaktionsmischung wurde im Vakuum weitgehend vom Methanol befreit und der Rückstand auf 2n Salzsäure/Toluol gegossen. Die wäßrige Phase wurde zweimal mit Toluol extrahiert, die vereinigten organischen Phasen einmal mit gesättigter wäßriger Natriumhydrogencarbonatlösung gewaschen, getrocknet (MgSO₄) und eingedampft. Flash-Chromatographie auf Kieselgel (Cyclohexan/Essigester = 2/1) ergab 25.6 g (0.040 mol, 60 %) "Beispiel 52".
   C₃₇H₆₂O₈ (634), MS (FAB, 3-NBA/LiJ): 641 (M+Li⁺).

### Beispiel 53

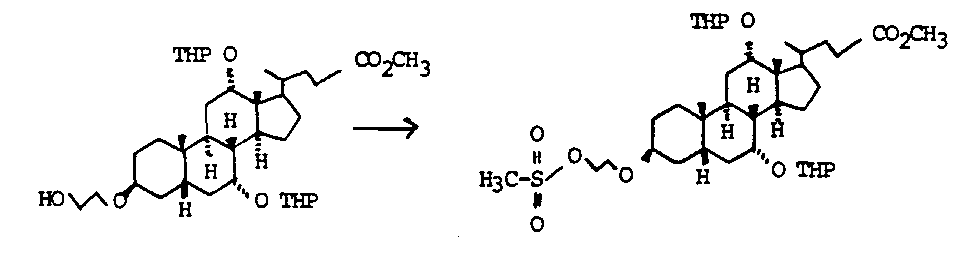

In Analogie zu Beispiel 19 wurde "Beispiel 53" erhalten.

### Beispiel 54

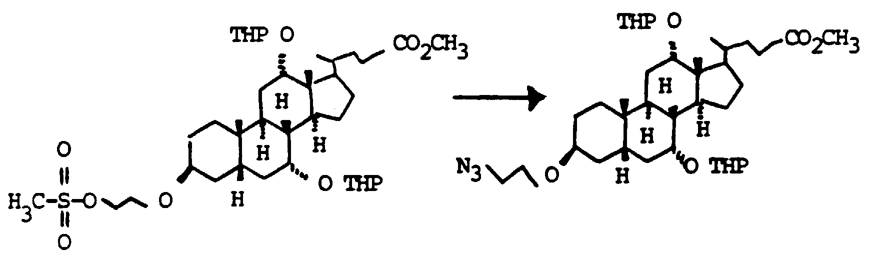

In Analogie zu Beispiel 20 wurde "Beispiel 54" erhalten.

### Beispiel 55

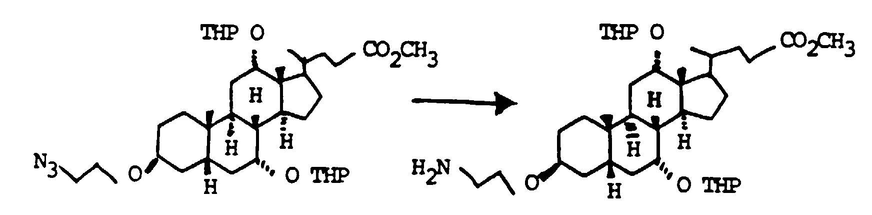

In Analogie zu Beispiel 21 wurde "Beispiel 55" erhalten.
C₃₇H₆₃NO₇ (633), MS (FAB, 3-NBA/LiJ): 640 (M+Li⁺).

### Beispiel 56

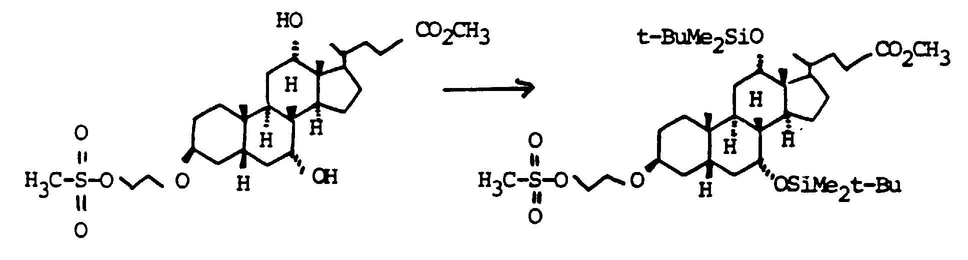

Zu einer Lösung von 24.5 g (0.045 mol) "Beispiel 19" und 26.4 ml (0.227 mol) 2,6-Dimethylpyridin in 150 ml Dichlormethan wurden bei 0°C 31.2 ml (0.136 mol) tert-Butyldimethylsilyltriflat getropft. Man ließ 15 min bei 0°C und 2 h bei Zimmertemperatur rühren. Das Reaktionsgemisch wurde auf gesättigte wäßrige NaHCO₃ Lösung gegossen und mit Dichlormethan extrahiert (3x). Die vereinigten organischen Phasen wurden mit Magnesiumsulfat getrocknet und eingedampft. Flash-Chromatographie auf Kieselgel (Essigester/Cyclohexan = 1/3) ergab 23.5 g (0.03 mol, 67 %) "Beispiel 56".
C₄₀H₇₆O₈Si₂S (772), MS (FAB, 3-NBA/LiJ): 779 (M+Li⁺)

### Beispiel 57

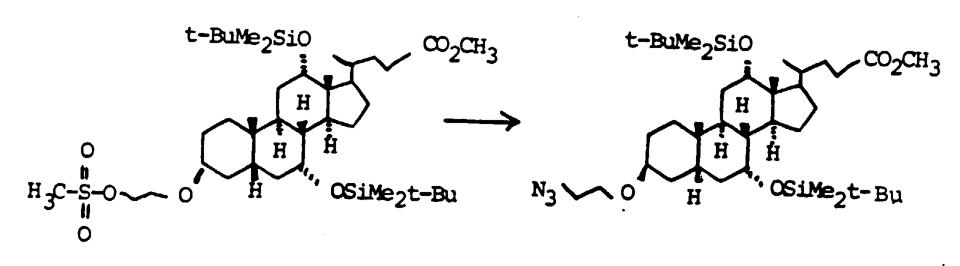

23.4 g (0.03 mol) Beispiel 56 wurden analog Beispiel 20 quantitativ in das Azid "Beispiel 57" überführt.

### Beispiel 58

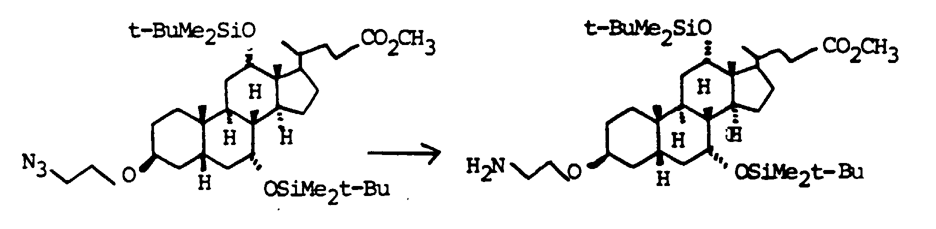

Das Azid "Beispiel 57" wurde in Analogie zu Beispiel 21 hydriert. Ausbeute nach Chromatographie auf Kieselgel (Essigester/MeOH/NEt₃ = 18/1/1) 10.0 g (0.014 mmol, 48 % bezogen auf 0.03 mol "Beispiel 56".
C₃₉H₇₅NO₅Si₂ (693), MS (FAB, 3-BNA/LiJ): 700 (M+Li⁺)

### Beispiel 59

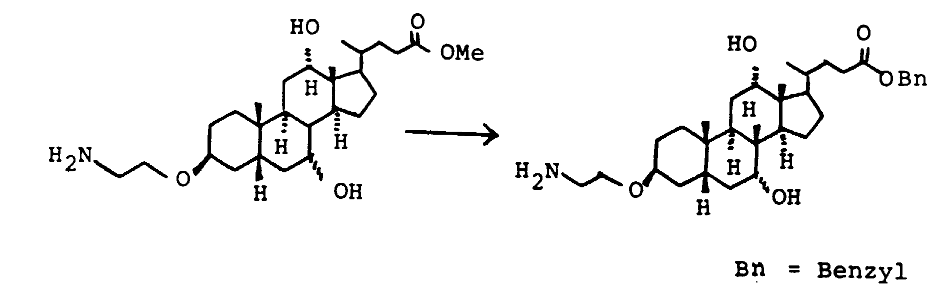

Eine Lösung von 500 mg (1.07 mmol) Beispiel 21 und 76 mg (0.33 mmol) Tetraethylorthotitanat in 10 ml trockenem Benzylalkohol wurde 8 h bei 100°C gerührt. Nach dem Abkühlen wurden 100 ml Essigester zugegeben. Das Gemisch wurde mit 1N HCl (1x) und 8 % NaHCO₃-Lösung (1x) ausgeschüttelt. Die organische Phase wurde mit MgSO₄ getrocknet und eingedampft. Chromatographie auf Kieselgel (Essigester/Methanol/NEt₃ 5:1:1) ergab 360 mg (6.64 mmol, 62 %) Benzylester "Beispiel 59".
C₃₃H₅₁NO₅ (541), MS (FAB): 542 (M+H⁺).

### Beispiel 60

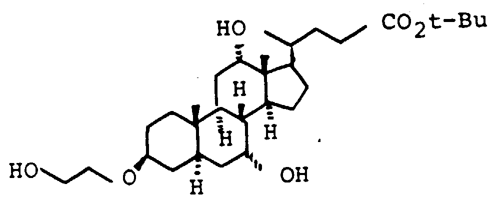

### Beispiel 60a

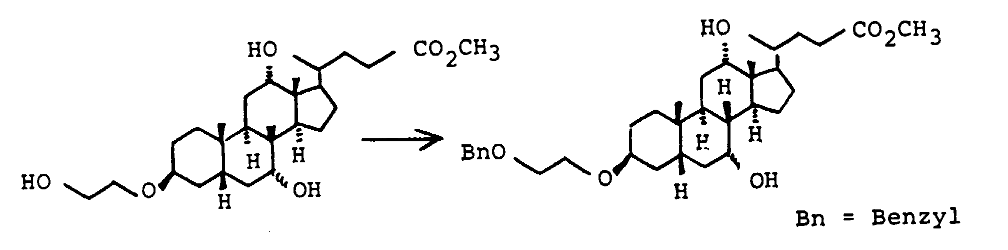

42 g (0,09 mol) Beispiel 11 wurden in 270 ml N-Ethyldiisopropylamin mit 61,5 g (0,36 mol) Benzylbromid 3 h bei 100°C (Badtemperatur) gerührt. Danach wurde das Reaktionsprodukt auf ein Gemisch aus 1,8 Liter Wasser und 180 ml konzentrierte Schwefelsäure gegossen und mit Essigester (2x) extrahiert. Die vereinten organischen Phasen wurden je einmal mit 1N Salzsäure, Wasser und gesättigter, wäßriger NaHCO₃-Lösung gewaschen, über MgSO₄ getrocknet und eingedampft.
Chromatographie des Rückstandes auf Kieselgel mit Essigester/Cyclohexan = 1/1 ergab 14,54 g (0,026 mol, 29,0%) "Beispiel 60a".
C₃₄H₅₂O₆ (556), MS (FAB, 3-NBA/LiCl): 563/M+Li⁺)

### Beispiel 60b

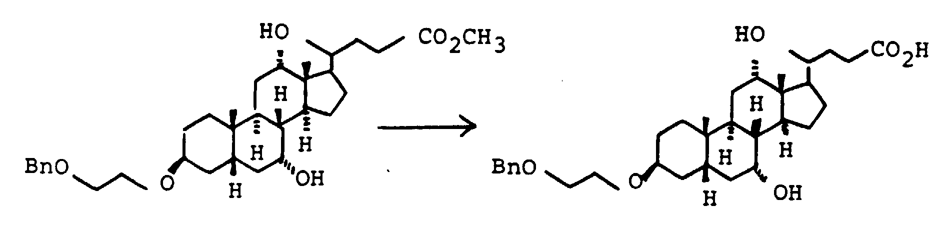

16,14 g (0,029 mol) Beispiel 60a wurden in 450 ml Methanol gelöst, mit 37 ml (0,037 mol) 1 N wäßriger Natronlauge versetzt und 8 h unter Rückfluß gekocht. Anschließend wurde das Methanol am Rotationsverdampfer abgezogen, der Rückstand in 320 ml Wasser gelöst und mit 37 ml (0,037 mol) 1 N wäßriger Salzsäure versetzt. Die entstandene Säure wurde mit Essigester extrahiert (2x). Die vereinten organischen Phasen wurden 2x mit Wasser gewaschen, über MgSO₄ getrocknet und eingedampft. Der kristalline Rückstand wurde mit 150 ml Diisopropylether angerieben, abgesaugt und im Vakuum getrocknet. Es wurden 13,85 g (0,025 mol, 88,0 %) "Beispiel 60b" vom Schmelzpunkt 144-146°C erhalten.
C₃₃H₅₀O₆ (542), MS (FAB, 3-NBA/TFA): 565 (M+Na⁺)

### Beispiel 60c

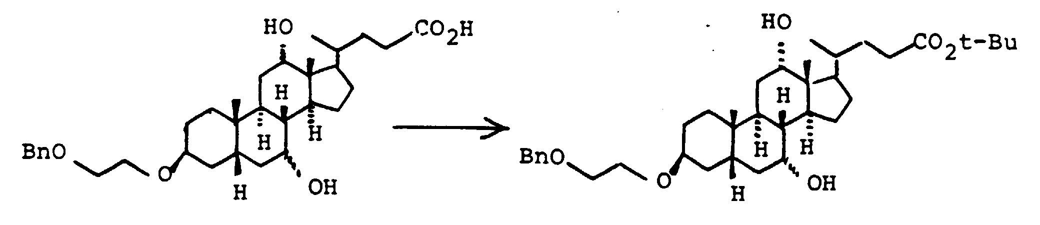

a) Anhydridbildung
   13,8 g (0,0254 mol) Beispiel 60b wurden in 250 ml abs. Tetrahydrofuran und 7,69 g (0,0762 mol) Triethylamin gelöst. Bei Zimmertemperatur wurden 6,28 (0,03 mol)
   2,6-Dichlorbenzoylchlorid zugetropft und anschließend 3 h unter Rückfluß gerührt.
b) Esterbildung
   Die unter a) erzeugte Anhydridlösung wurde auf +10°C abgekühlt und nacheinander mit 28,12 g (0,38 mol) tert. Butanol und 3,1 g (0,0254 mol) 4 -Dimethylaminopyridin versetzt, dann wurde innerhalb von 1 h zum Sieden erhitzt und 4 h unter Rückfluß gerührt. Danach wurde die Reaktionsmischung im Vakuum weitgehend vom Tetrahydrofuran befreit. Der Rückstand wurde in Essigester aufgenommen und 3x mit Wasser durchgewaschen, und mit MgSO₄ getrocknet und eingedampft.
   Chromatographie des Rückstandes auf Kieselgel mit
   Essigester/Cyclohexan = 1/1 ergab 6,85 g "Beispiel 60c". (0,0114 mol, 45,0 %) Schmelzpunkt: 79-80°C;

### Beispiel 60d

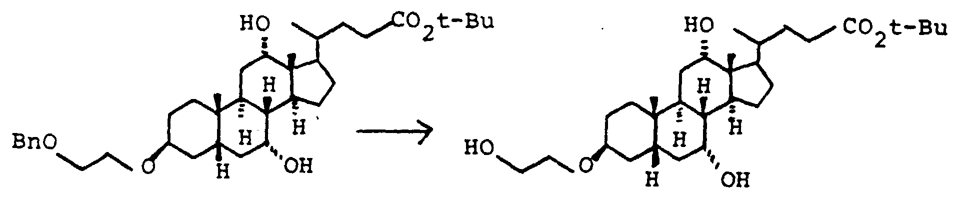

7,14 g (0,0119 mol) Beispiel 60c wurden in 250 ml Essigester über 1,5 g Pd/C-Katalysator (10 %) bei Zimmertemperatur und Normaldruck hydriert. Nach Beendigung der Wasserstoffaufnahme wurde der Katalysator abfiltriert und das Filtrat eingedampft. Es wurden 6,0 g "Beispiel 60d" erhalten (0,0117 mol, 98,9%)
C₃₀H₅₂O₆ (508), MS (FAB, 3-NBA/LiCl, 515 M+Li⁺)

### Beispiel 60e

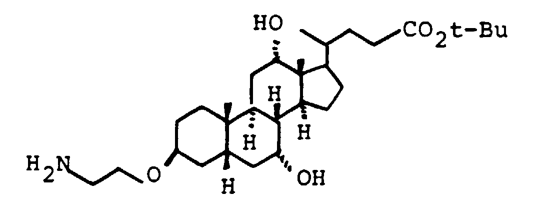

In Analogie zu den Beispielen 19-28 wurde aus Beispiel 60d "Beispiel 60e" hergestellt.
C₃₀H₅₃N O₅ (507); MS (FAB, 3-NBA/LiCl): 514 (M+Li⁺)

### Beispiel 61

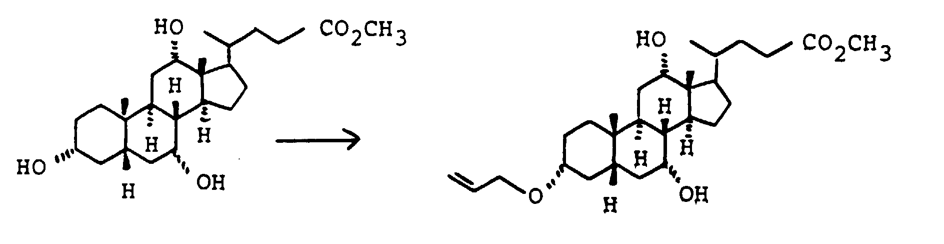

42,2 g (0.1 mol) Cholsäuremethylester, 300 ml (1.8 mol) N-Ethyldiisopropylamin und 10 ml (0.12 mol) Allylbromid wurden 8h unter Rückfluß erhitzt. Nach jeder Stunde Reaktionszeit wurden nochmals jeweils 5 ml Allylbromid zugegeben (DC-Kontrolle, Cyclohexan/Essigester = 1:1). Das Reaktionsgemisch wurde auf 400 ml konz. H₂SO₄/2000 ml Wasser gegossen und mit Essigester extrahiert (3x). Die vereinigten organischen Phasen wurden jeweils einmal mit 1 N HCl, Wasser und gesättigter NaHCO₃-Lösung gewaschen. Trocknen (MgSO₄), Abziehen des Lösungsmittels und Chromatographie des Rückstandes auf Kieselgel (n-Heptan/Essigester = 4:1→3:1→2:1) ergab 21,91 g (0,047 mol, 47 %) "Beispiel 61".
C₂₈H₄₆O₅ (462), MS (FAB, 3-NBA/LiCl): 469 (M+Li⁺)

### Beispiel 62

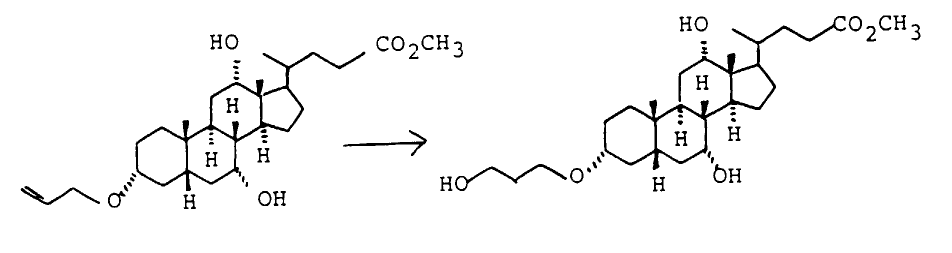

**(1) Herstellung von Thexylboran:** Unter Argonatmosphäre wurden bei 0°C zu 85 ml 1 molarer BH₃.THF-Lösung (THF) 85 ml 1 molare 2,3-Dimethylbutenlösung (THF) getropft. Man ließ bei 0°C rühren.
**(2) Hydroborierung:** Zu der nach (1) bereiteten Lösung wurden bei 0°C 8,6 g (18.59 mmol) Olefin Beispiel 61 in 25 ml THF getropft. Nach 3h bei 0°C ließ man auf Zimmertemperatur kommen (DC-Kontrolle). Nach 16 h bei Zimmertemperatur wurde frisch hergestellte Thexylboranlösung (THF) zugetropft. Man ließ erneut bei Zimmertemperatur rühren. Nachdem kein Ausgangsmaterial mehr nachweisbar war, wurde das Reaktionsgemisch unter Argonatmosphäre vorsichtig unter intensivem Rühren in wäßrige Natronlauge transferiert (pro Äquivalent Boran 1 Äquivalent NaOH). Anschließend wurde unter Eiskühlung 30 prozentige Wasserstoffperoxidlösung zugetropft. (2 Äquivalente pro 1 Äquivalent Boran). Nach 20 min bei 0°C wurde 30 min auf 50°C erwärmt. Zur besseren Phasentrennung wurde gesättigte Kochsalzlösung zugegeben. Die wäßrige Phase wurde mit Ethylacetat extrahiert (2x) und die vereinigten organischen Phasen mit gesättigter Natriumbisulfitlösung (2x) und anschließend mit Natriumchloridlösung (1x) gewaschen. Trocknen mit MgSO₄, Abziehen des Lösungsmittels und Chromatographie auf Kieselgel (Ethylacetat →Ethylacetat/MeOH = 20:1) ergab 5,0 g (10.4 mmol, 56 %) "Beispiel 62"
   Rf (Ethylacetat): 0.18
   C₂₈H₄₈O₆ (480); MS (FAB, 3-NBA/LiCl): 487 (M+Li⁺)
   Daneben wurden 1.0 g des sekundären Alkohols erhalten.
   Rf (Ethylacetat): 0,27

### Beispiel 63

In Analogie zu den Beispielen 19-28 wurde aus Beispiel 62 "Beispiel 63" erhalten. 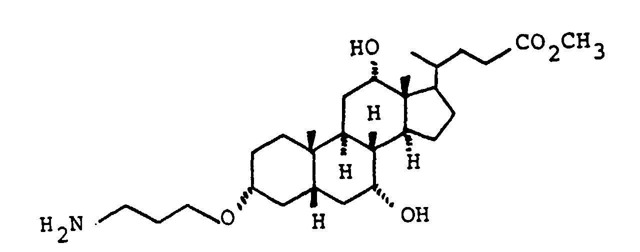 (X-G mit α-Konfiguration an 3-C)
C₂₈H₄₉NO₅ (479); MS (FAB, 3-NBA/LiCl): 486 (M+Li⁺)

### Beispiel 64

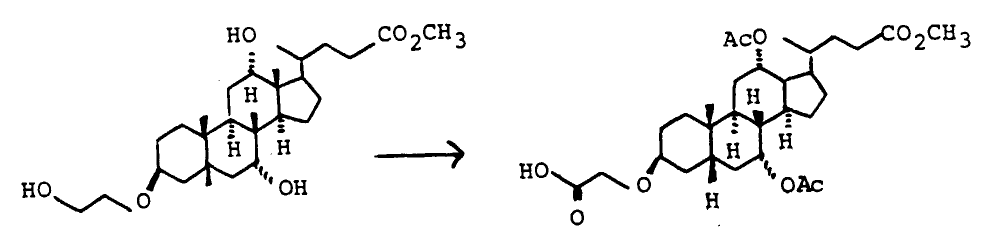

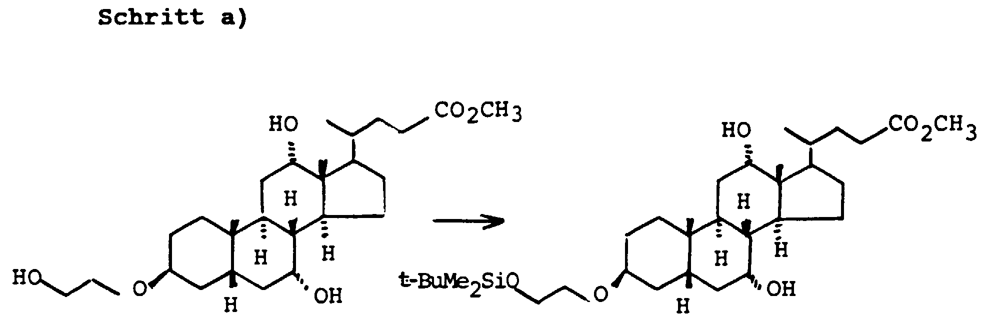

75 g (0.161 mol) Beispiel 11 wurden mit 21.6 g (0.177 mol) 4-Dimethylaminopyridin und 26.7 g (0.177 mol) tert.-Butyldimethylsilylchlorid in 500 ml trockenem Dichlormethan 4 h bei Zimmertemperatur gerührt. Das Reaktionsgemisch wurde auf Wasser gegossen und mit Essigester extrahiert (3 x). Die vereinigten organischen Phasen wurden getrocknet (MgSO₄) und eingedampft. Ausbeute 93.6 g (quantitativ) Silylether. Für präparative Zwecke war eine weitere Reinigung nicht erforderlich.
C₃₃H₆₀O₆Si (580); MS (FAB, 3-NBA/LiCl): 587 M+Li⁺).

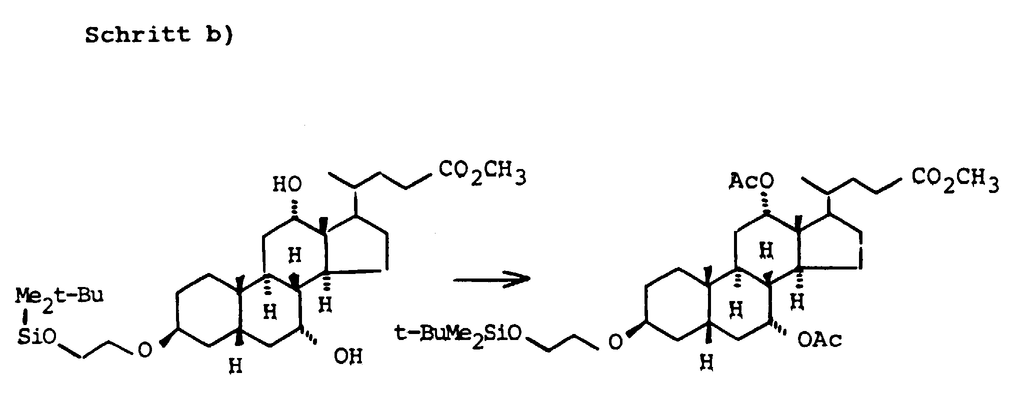

Zu 10 g (0.0172 mol) des nach Schritt a) gewonnenen Silylethers und 5.3 g (0.043 mol) 4-Dimethylaminopyridin in 100 ml trockenem Pyridin wurden bei 0°C 4.1 ml (0.043 mol) Essigsäureanhydrid getropft. Man ließ 4 h bei Zimmertemperatur rühren. Das Reaktionsgemisch wurde auf Wasser gegossen und mit Essigester extrahiert (3 x). Die vereinigten organischen Phasen wurden mit gesättigter wäßriger NaHCO₃-Lösung gewaschen und getrocknet (MgSO₄). Abdampfen des Lösungsmittels und Chromatographie auf Kieselgel (Essigester/Cyclohexan = 1/3) ergabe 10 g (0.015 mol, 87.7 %) Diacetat.
C₃₇H₆₄O₈Si (664); MS (FAB, 3-NBA/LiCl): 671 (M+Li⁺).

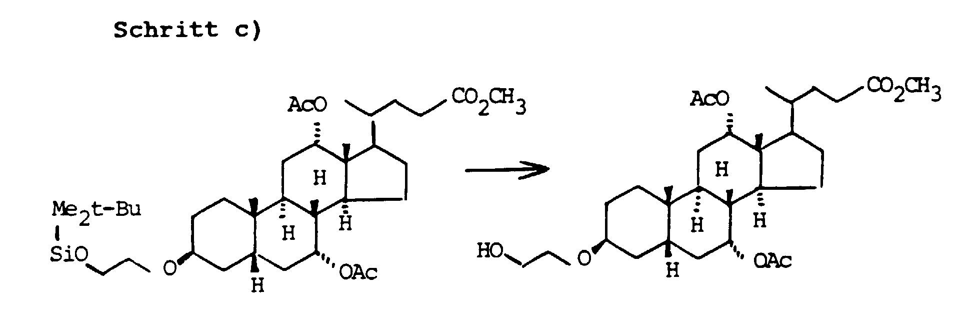

10 g (0.015 mol) des nach Schritt b) gewonnenen Diacetats wurden mit 5.2 g (0.0165 mol) Tetrabutylammoniumfluorid-Trihydrat in 100 ml Tetrahydrofuran 1 h bei Zimmertemperatur gerührt. Das Reaktionsgemisch wurde auf Wasser gegossen und mit Essigester extrahiert (3 x). Die vereinigten organischen Phasen wurden getrocknet (MgSO₄) und eingedampft. Ausbeute an Alkohol quantitativ. Für präparative Zwecke war eine weitere Reinigung nicht erforderlich.
C₃₁H₅₀O₈ (550); MS (FAB, 3-NBA/LiCl): 557 (M+Li⁺).

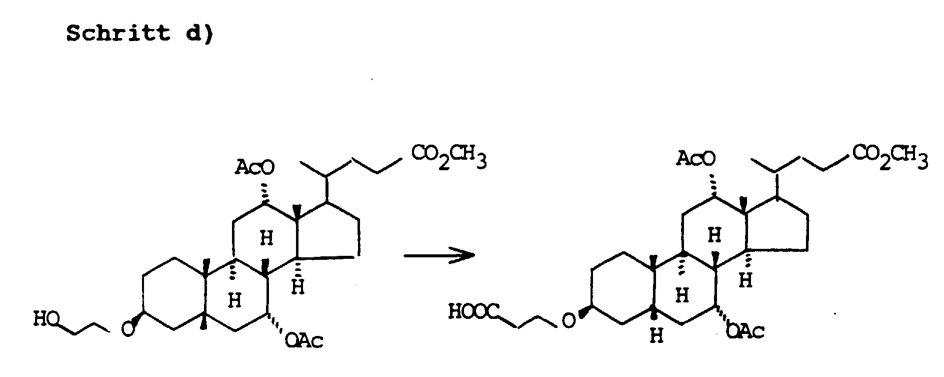

7.35 g (0.0133 mol) des nach Schritt c) gewonnenen Alkohols wurden mit 50 g (0.133 mol) Pyridiniumdichromat in 150 ml trockenem Dimethylformamid 24 h bei Zimmertemperatur gerührt. Das Reaktionsgemisch wurde auf Wasser gegossen und mit Diethylether extrahiert (3 x). Die vereinigten organischen Phasen wurden getrocknet (MgSO₄) und eingedampft. Chromatographie auf Kieselgel (Essigester/Cyclohexan = 9/1) ergab 5.1 g (0.009 mol, 58 %) "Beispiel 64".
C₃₁H₄₈O₉ (564); MS (FAB, 3-NBA/LiCl): 571 (M+Li⁺).

### 3. W-X-G, X = ein Zwischenglied

### Beispiel 65

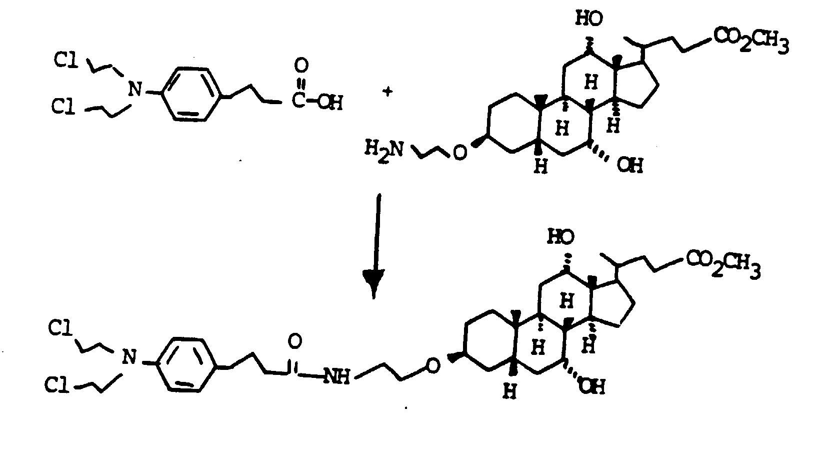

Zu 1.96 g (6.44 mmol) Chlorambucil (Sigma) in 100 ml THF/20 ml Triethylamin wurden bei 0°C 0.62 ml (6.44 mmol) Chlorameisensäureethylester getropft und 15 min gerührt. Bei 0°C wurden 3.0 g (6.44 mmol) Beispiel 21, gelöst in THF, zugetropft und anschließend 30 min bei Zimmertemperatur gerührt. Das Reaktionsgemisch wurde auf Wasser gegossen und dreimal mit Essigester extrahiert. Die vereinigten organischen Phasen wurden getrocknet (MgSO₄) und eingedampft. Chromatographie auf Kieselgel (Essigester/Methanol = 9/1) ergab 3.9 g (5,2 mmol, 81 %) "Beispiel 65".
Schmp.: 45 - 50°C
C₄₁H₆₄Cl₂N₂O₆ (750), MS (FAB, 3-NBA/LiJ): 751 (M+Li⁺)

### Beispiel 66

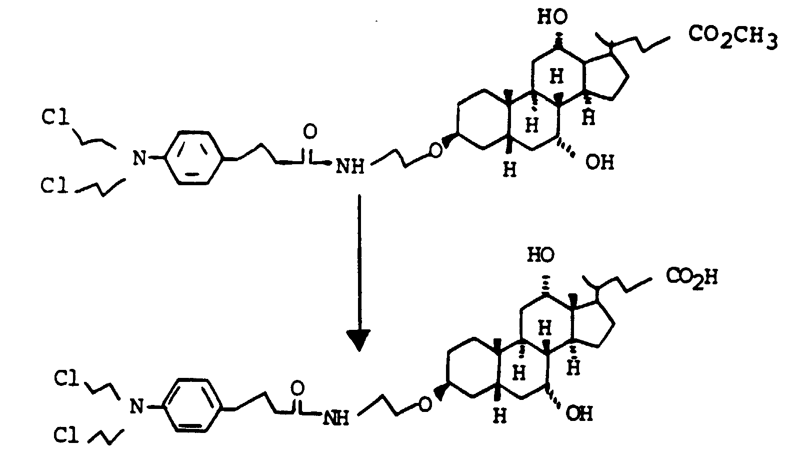

Zu einer Lösung von 1.96 g (2.6 mmol) Beispiel 65 in 20 ml Ethanol tropfte man 5 ml 1n wäßrige Natronlauge. Nach 3 h bei Zimmertemperatur wurden weitere 5 ml 1n wäßrige Natronlauge zugesetzt und erneut 3 h gerührt. Das Reaktionsgemisch wurde auf 200 ml Wasser gegossen, mit 1n wäßriger Salzsäure neutralisiert und dreimal mit Essigester extrahiert. Die vereinigten organischen Phasen wurden getrocknet (MgSO₄) und eingedampft. Ausbeute: 1.91 g (2.6 mmol, quant.) "Beispiel 66"
Schmp.: 60 - 70°C
C₄₀H₆₂Cl₂N₂O₆ (736), MS (FAB, 3-NBA/LiJ): 743 (M+Li⁺)

### Beispiel 67

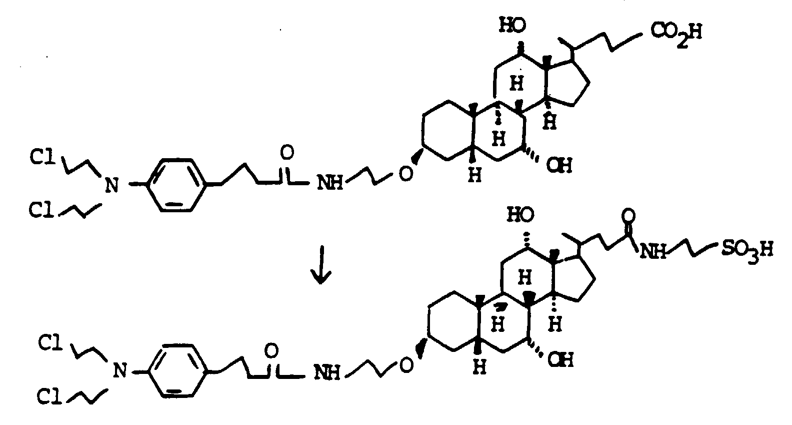

Zu einer Lösung von 1.5 g (2.03 mmol) Beispiel 66 und 0.97 ml (4.06 mmol) Tri-n-butylamin in 10 ml Dioxan wurden bei 0°C 0.2 ml (2.03 mmol) Chlorameisensäureethylester getropft und 15 min bei 0°C gerührt. Anschließend wurde eine Lösung von 0.508 g (4.06 mmol) Taurin in 4 ml 1n wäßriger Natronlauge bei 0°C zugetropft. Man ließ 1 h bei Zimmertemperatur rühren, goß auf 200 ml Wasser und neutralisierte mit 1n wäßriger Salzsäure. Es wurde mit Essigester unter Zusatz von wenig Methanol extrahiert (3x) und die vereinigten organischen Phasen getrocknet (MgSO₄). Abziehen des Lösungsmittels und Flash-Chromatographie auf Kieselgel (Essigester/Methanol = 4/1, dann 2/1) ergab 1.59 g (1.89 mmol, 93 %) "Beispiel 67".
Schmp.: 130 - 140°C
C₄₂H₆₇Cl₂N₃O₈S (843), MS (FAB, 3-NBA/LiJ): 856 (M+Li⁺-H)

### Beispiel 68

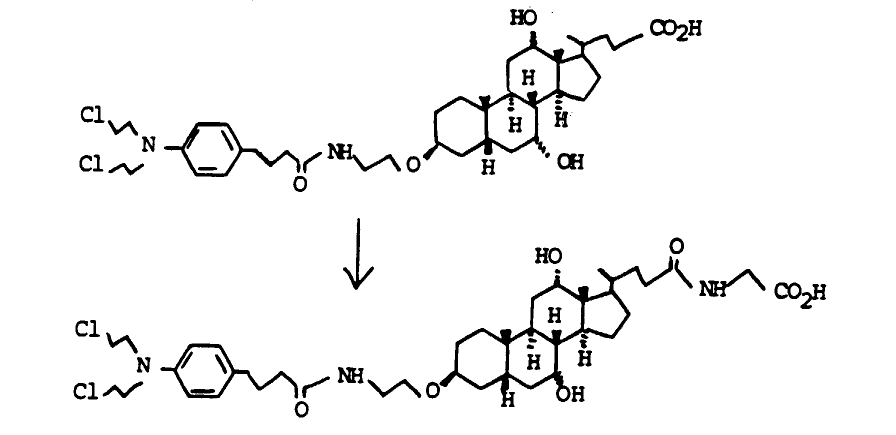

Zu einer Lösung von 1.5 g (2.03 mmol) Beispiel 66 und 0.97 ml (4.06 mmol) Tri-n-butylamin in 10 ml Dioxan wurden bei 0°C 0.2 ml (2.03 mmol) Chlorameisensäureethylester getropft und 15 min bei 0°C gerührt. Anschließend wurde eine Lösung von 0,305 g (4.06 mmol) Glycin in 4,0 ml 1n wäßriger Natronlauge zugegeben und 4 h bei Zimmertemperatur gerührt. Das Reaktionsgemisch wurde auf 200 ml Wasser gegossen, mit 1n wäßriger Salzsäure neutralisiert und mit Essigester extrahiert. Die vereinigten organischen Phasen wurden getrocknet (MgSO₄), eingedampft und auf Kieselgel Flashchromatographiert (Essigester/Methanol = 2/1).
Ausbeute 1.15 g (1.45 mmol, 71 %) "Beispiel 68".
Schmp.: 75 - 85°C
C₄₂H₆₅Cl₂N₃O₇ (793), MS (FAB, 3-NBA/LiJ): 806 (M+2 Li⁺-H)

### ZU: W-X-G, mit X = ein Zwischenglied

### Beispiele 69-78

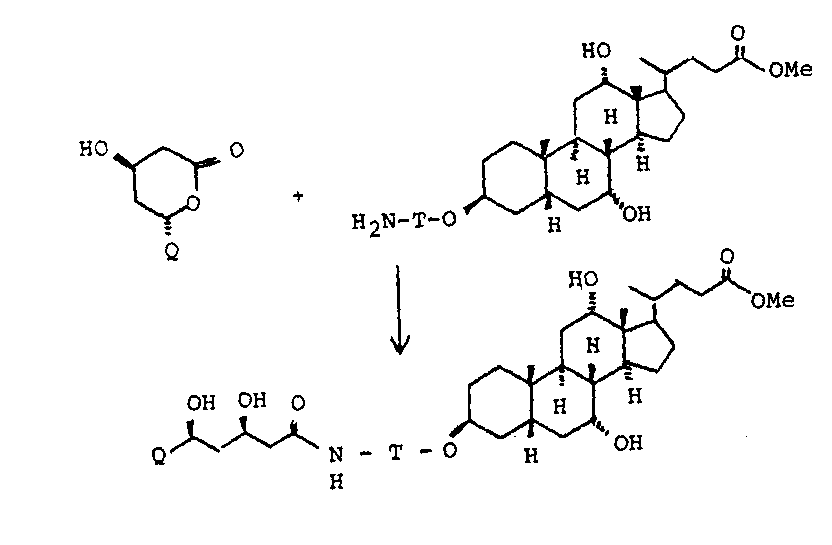

### Beispiel 69

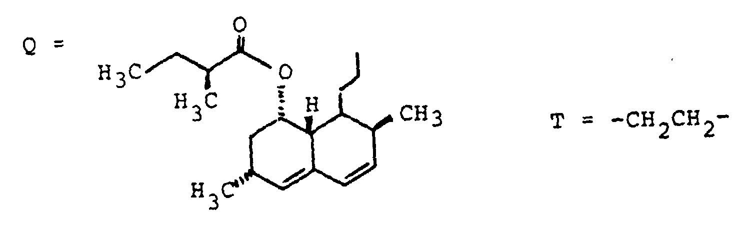

Ein Gemisch aus 750 mg (1.61 mg) Beispiel 21, 5 ml Triethylamin, 200 mg (1.61 mmol) Dimethylaminopyridin (DMAP) und 651 mg (1.61 mmol) Mevinolin in 25 ml trockenem THF wurde 48 h unter Rückfluß erhitzt. Das Lösungsmittel wurde abgezogen und der Rückstand auf Kieselgel chromatographiert (Essigester/ MeOH = 19:1).
Ausbeute: 900 mg (1.03 mmol, 64 %) "Beispiel 69"
Schmp.: 78-80°C
C₅₁H₈₃NO₁₀ (869), MS (FAB, 3-NBA/LiJ): 876 (M⁺Li⁺).

### Beispiel 70

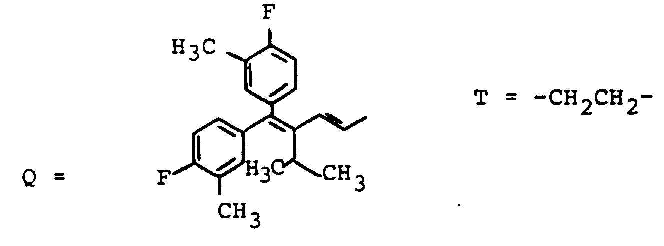

Analog zu Beispiel 69 wurde mit Beispiel 21 "Beispiel 70" erhalten.
Schmp.: 70-75°C
C₅₃H₇₅F₂NO₂ (891); MS (FAB, 3-NBA/LiJ): 898 (M+Li⁺)
(Zur Herstellung der Lactonkomponente siehe DE 37 22 807-A); siehe auch Tetrahedron Letters 29, 929-930 [1988], wo Verbindungen ohne die dem F benachbarten Methylgruppen beschrieben sind).

### Beispiel 71

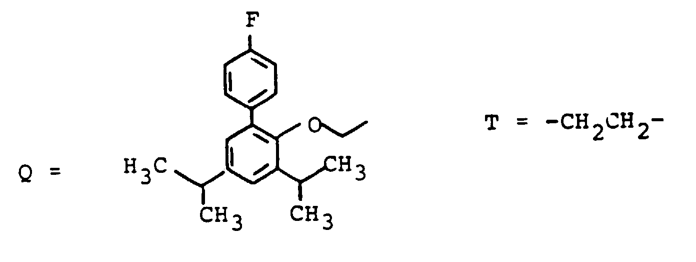

Analog zu 69 wurde mit Beispiel 21 "Beispiel 71" erhalten.
Schmp.: 65-70°C
C₅₁H₇₆FNO₉ (865), MS (FAB, 3-NBA/LiJ): 872 (M+Li⁺)
(Zur Herstellung der Lactonkomponente siehe DE 38 19 999-A), Beispiel 1 siehe auch die Beschreibung vor dem pharmakologischen Teil der vorliegenden Anmeldung).

### Beispiel 72

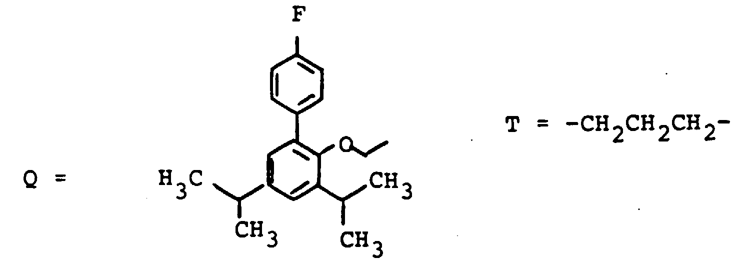

Analog zu 69 wurde mit Beispiel 22 "Beispiel 72" erhalten.
C₅₂H₇₈FNO₉ (879), MS (FAB, 3-NBA/LiCl): 886 (M+Li⁺)

### Beispiel 73

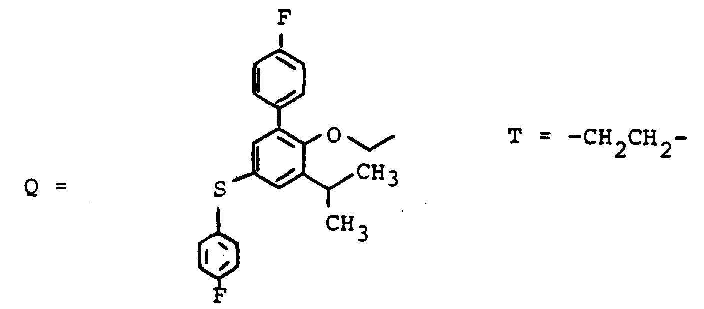

Analog zu 69 wurde mit Beispiel 21 "Beispiel 73" erhalten.
C₅₄H₇₃F₂NO₉S (949), MS (FAB, 3-NBA/LiCl): 956 (M+Li⁺).
(Zur Herstellung der Lactonkomponente siehe DE P 39 29 913).

### Beispiel 74

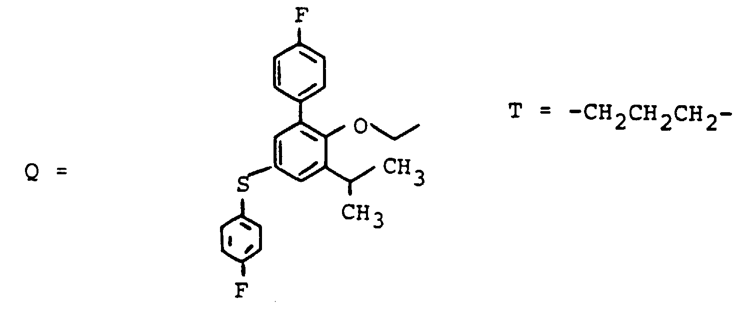

Analog zu 69 wurde mit Beispiel 22 "Beispiel 74" erhalten.
C₅₅H₇₅F₂NO₉S (963), MS (FAB, 3-NBA/LiCl): 970 (M+Li⁺)

### Beispiel 75

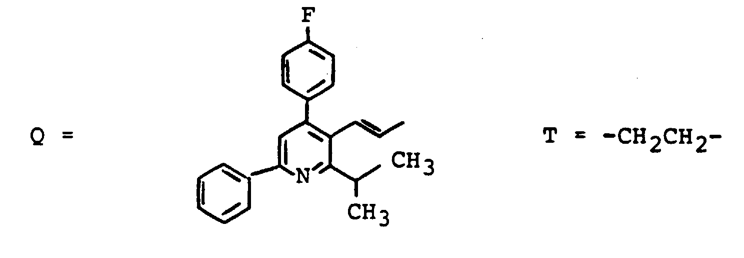

Analog zu 69 wurde mit Beispiel 21 "Beispiel 75" erhalten
Schmp.: 74-76°C
C₅₄H₇₃FN₂O₈ (896), MS (FAB, 3-NBA/LiJ): 903 (M+Li⁺)

### Beispiel 76

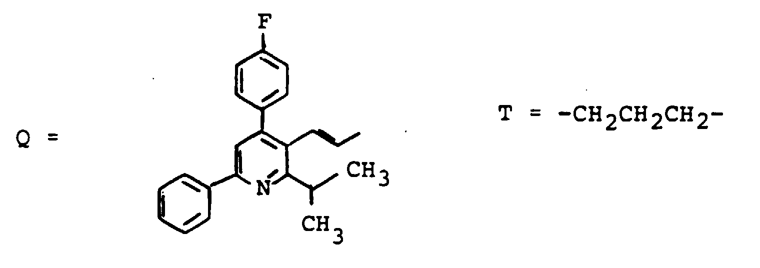

Analog zu 69 wurde mit Beispiel 22 "Beispiel 76" erhalten.
C₅₅H₇₅FN₂O₈ (910), MS (FAB, 3-NBA/LiCl): 917 (M+Li⁺).

### Beispiel 77

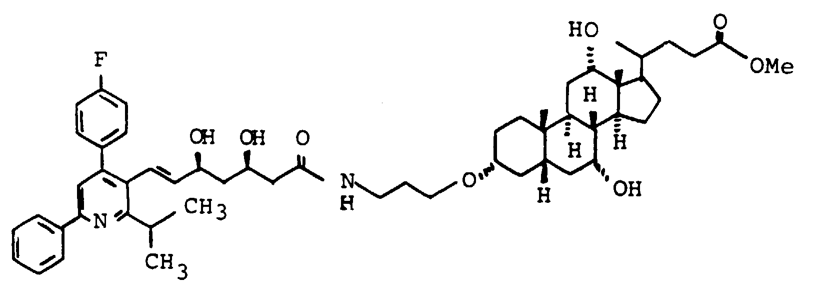

Analog zu 69 wurde mit Beispiel 63 "Beispiel 77" erhalten.
C₅₅H₇₅FN₂O₈ (910), MS (FAB, 3-NBA/LiCl): 917 (M+Li⁺).

### Beispiel 78

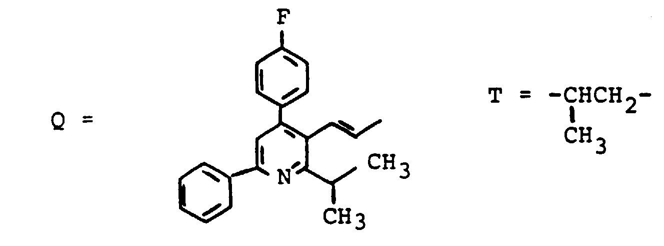

Analog zu 69 wurde mit Beispiel 28 "Beispiel 78" erhalten.
C₅₅H₇₅FN₂O₈ (910), MS (FAB, 3-NBA/LiCl): 917 (M+Li⁺)

### Beispiele 79-88

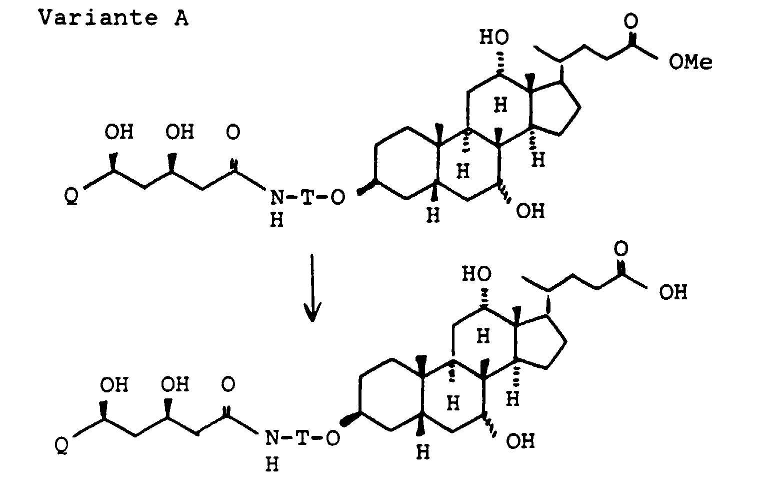

### Beispiel 79

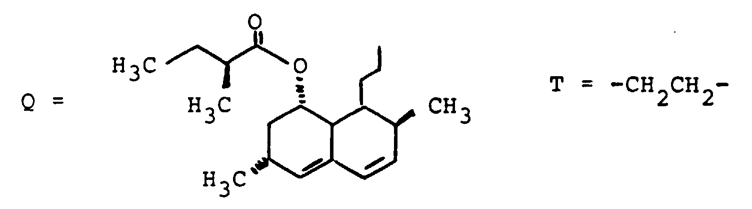

250 mg (0.29 mmol) Beispiel 69 wurden in 5 ml Ethanol gelöst und mit 2.0 ml 1N wäßriger Natronlauge versetzt. Nach 6 h Rühren bei Zimmertemperatur wurde mit 1N Salzsäure neutralisiert und mit Essigester extrahiert (3x). Die vereinigten organischen Phasen wurden mit MgSO₄ getrocknet und eingedampft.
Ausbeute 230 mg (0.27 mmol, 94 %) "Beispiel 79".
C₅₀H₈₁NO₁₀ (855), MS (FAB, 3-NBA/LiJ): 862 (M+Li⁺)

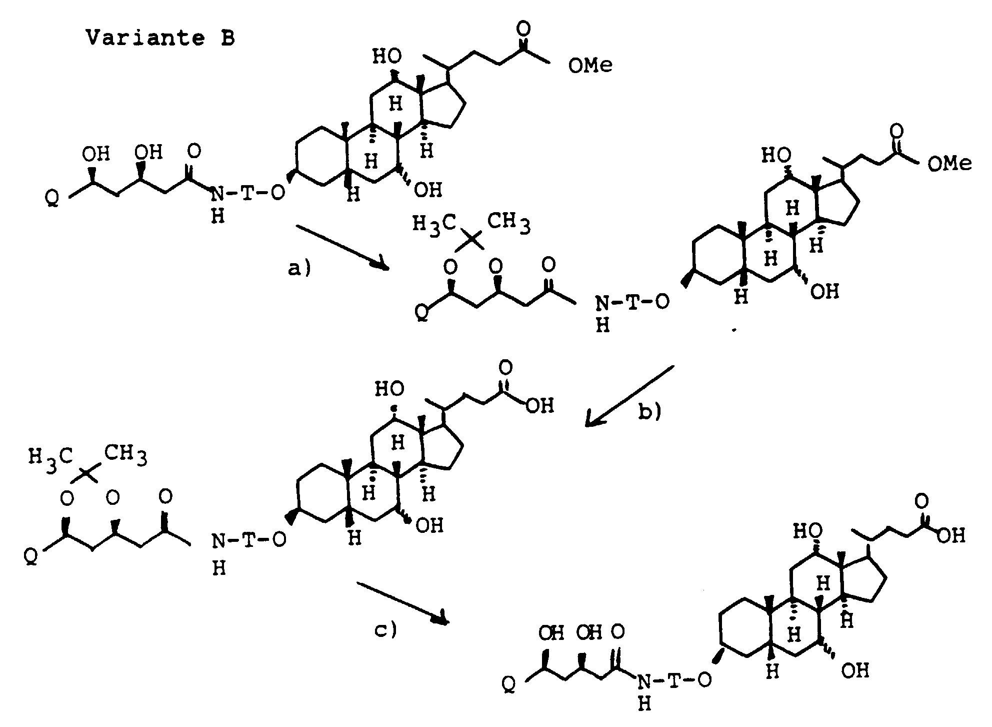

### Beispiel 80

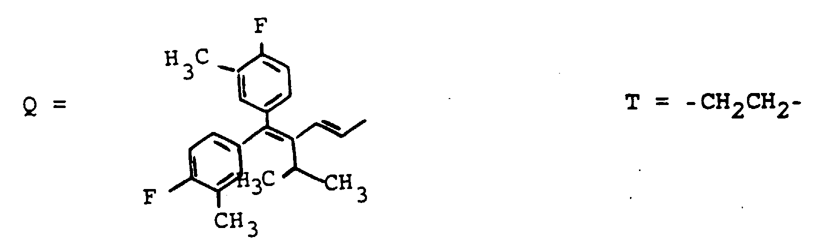

a) 400 mg (0.45 mmol) Beispiel 70 und ein Kristall p-Toluolsulfonsäure wurden in 20 ml Aceton/Dimethoxypropan 1:1 gelöst und bgei Zimmertemperatur gerührt. Nach 15 min wurde das Lösungsmittel eingedampft und der Rückstand auf Kieselgel chromatographiert (Chloroform/Methanol 19:1).
   Ausbeute 390 mg (0.42 mol, 93 %) Acetonid.
   C₅₆H₇₉F₂NO₈ (931), MS (FAB 3-NBA/LiCl): 938 (M+Li⁺).
b) 390 mg (0.42 mmol) Acetonid wurden in 20 ml Ethanol gelöst und mit 5 ml 1N wäßriger Natronlauge versetzt. Nach 5h Rühren bei Zimmertemperatur wurde mit 2N Salzsäure neutralisiert und mit Essigester extrahiert (3x). Die vereinigten organischen Phasen wurden mit MgSO₄ getrocknet und eingedampft. Das Rohprodukt wurde ohne weitere Reinigung umgesetzt.
c) Das Rohprodukt aus b) wurde in 20 ml THF gelöst und mit 5 ml 2N Salzsäure versetzt. Nach 2h Rühren wurden 100 ml Wasser zugegeben und mit Essigester extrahiert (3x). Die vereinigten organischen Phasen wurden mit MgSO₄ getrocknet und eingedampft. Flashchromatographie auf Kieselgel (Dichlormethan/Methanol 9:1) ergab 250 mg (0.29 mmol, 68%) "Beispiel 80c".
   C₅₂H₇₃F₂NO₈ (877), MS (FAB, 3-NBA/LiCl): 884 (M+Li⁺).

### Beispiele 81 - 88

Analog zu 79 oder 80 wurden die "Beispiele 81-88" aus 71-78 erhalten.

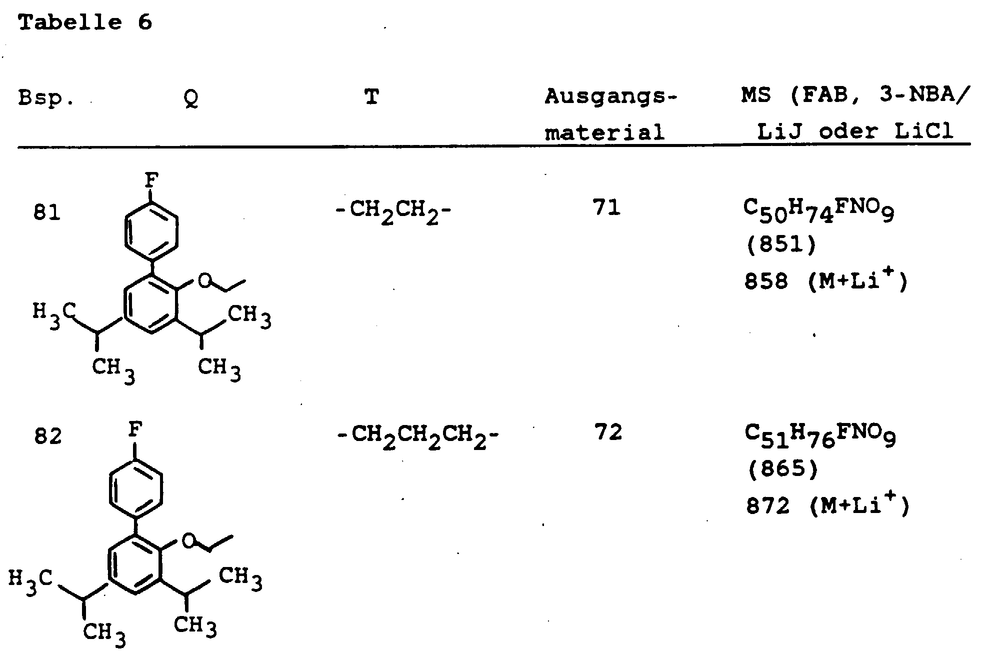

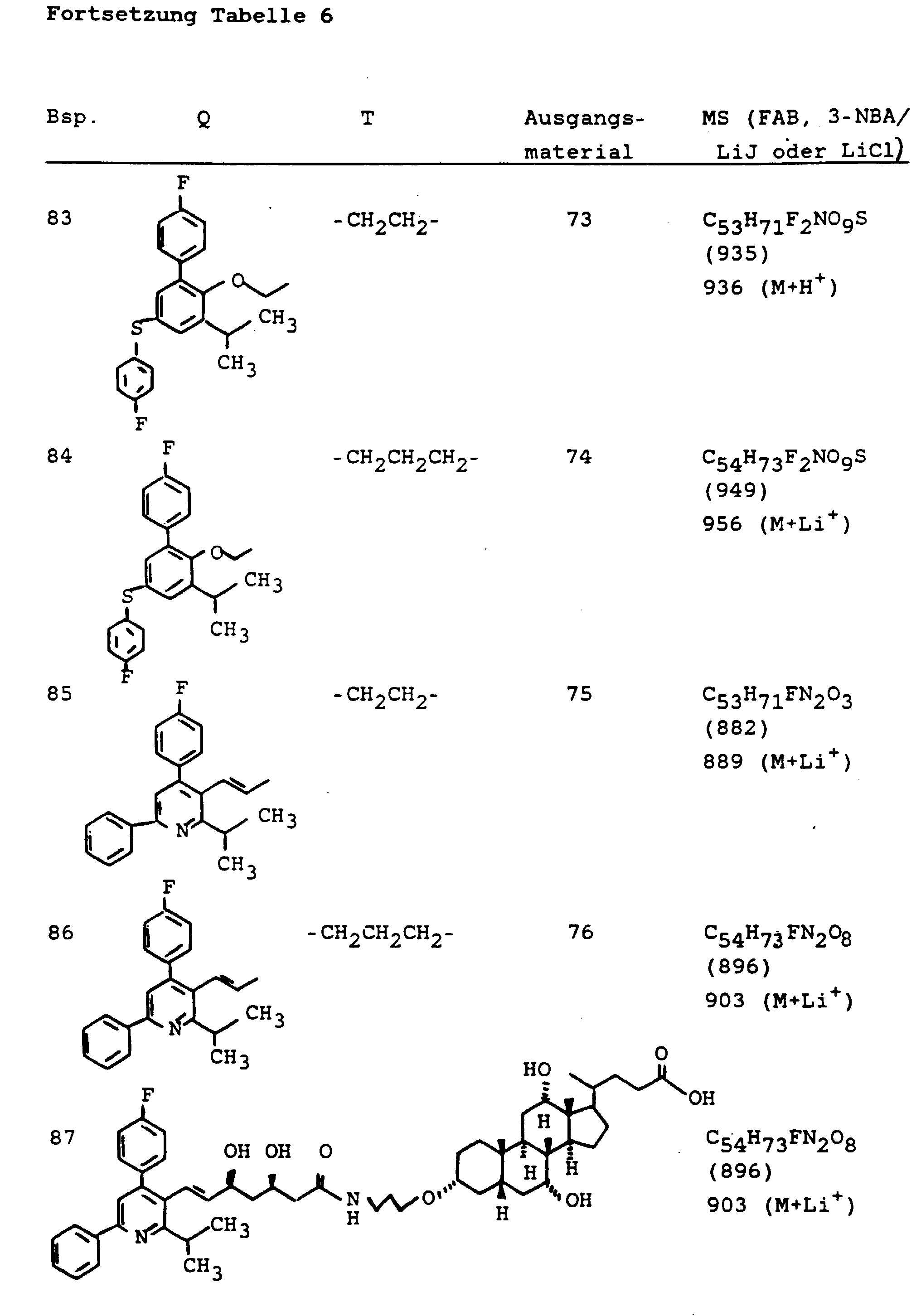

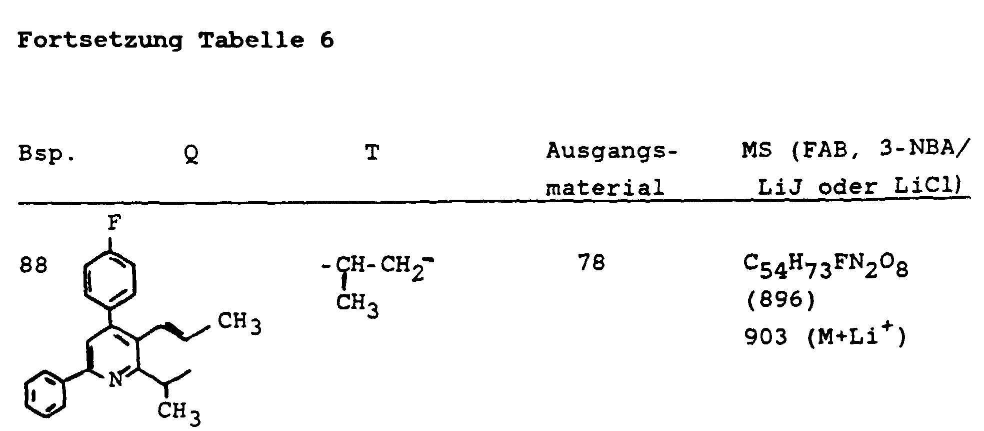

### Beispiele 89-98

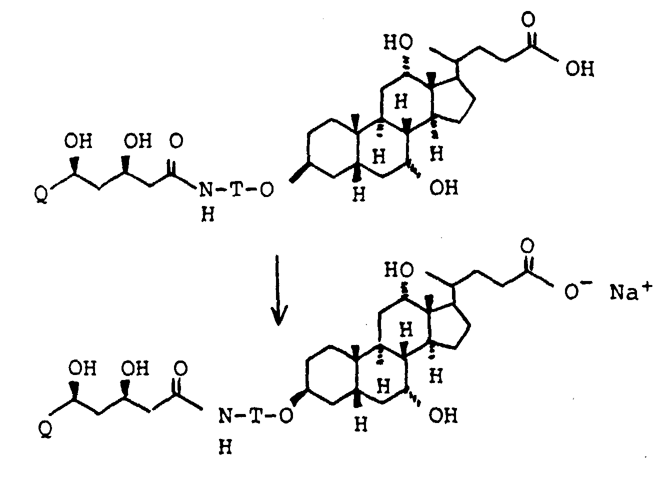

### Beispiel 89

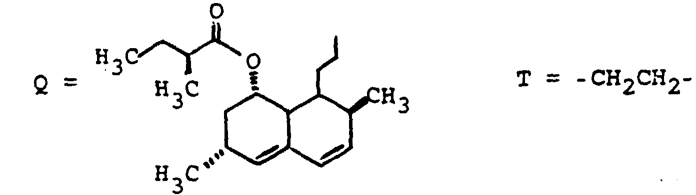

150 mg (0.175 mmol) Beispiel 79 wurden in 10 ml Ethanol gelöst und mit 1.75 ml 0.1 N wäßriger Natronlauge versetzt. Nach 10 min Rühren bei Zimmertemperatur wird das Lösungsmittel eingedampft. Ausbeute 150 mg (quant.) Natriumsalz "Beispiel 89".

### Beispiele 90 - 98

Analog zu 89 wurden die "Beispiele 90-98" erhalten

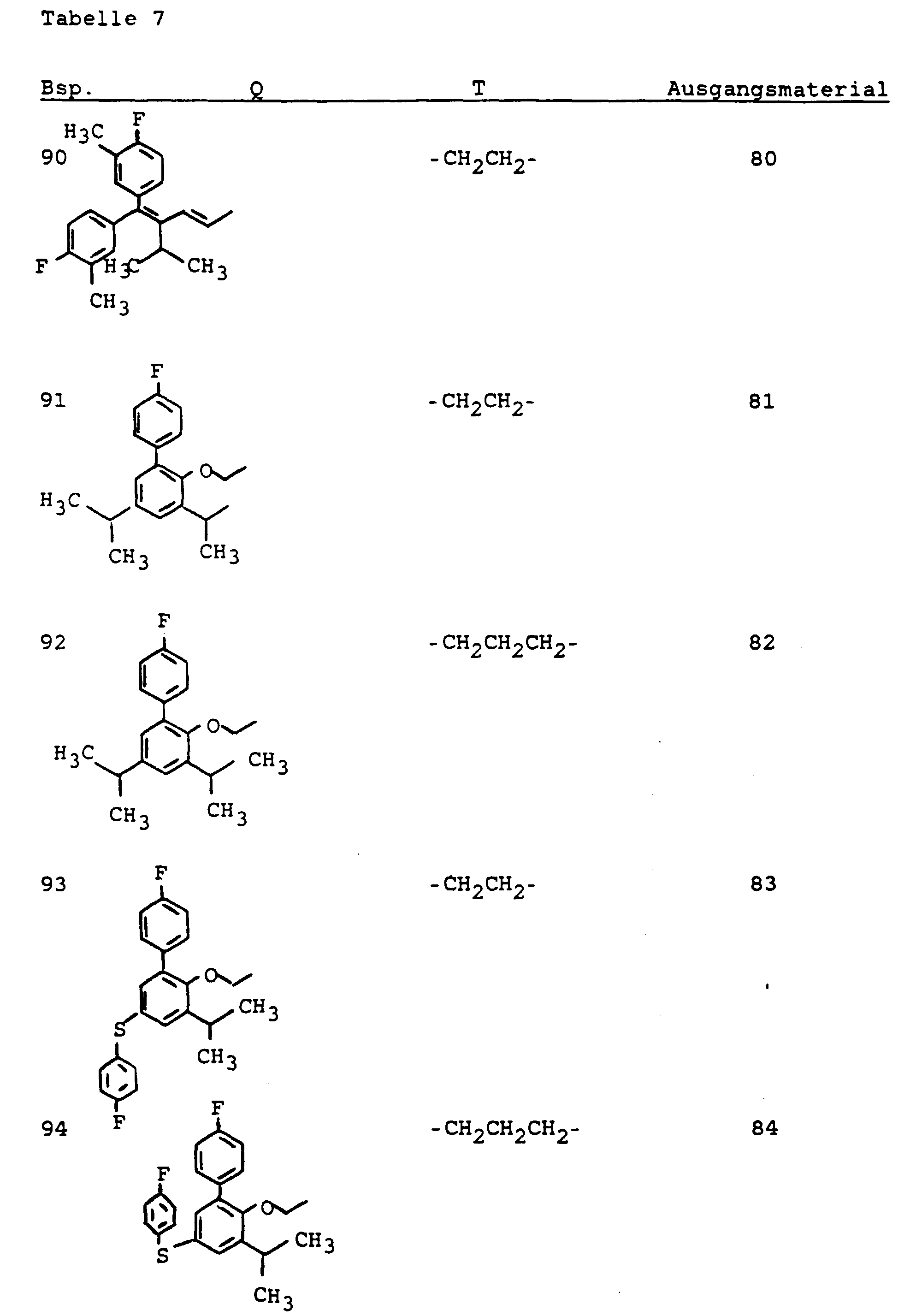

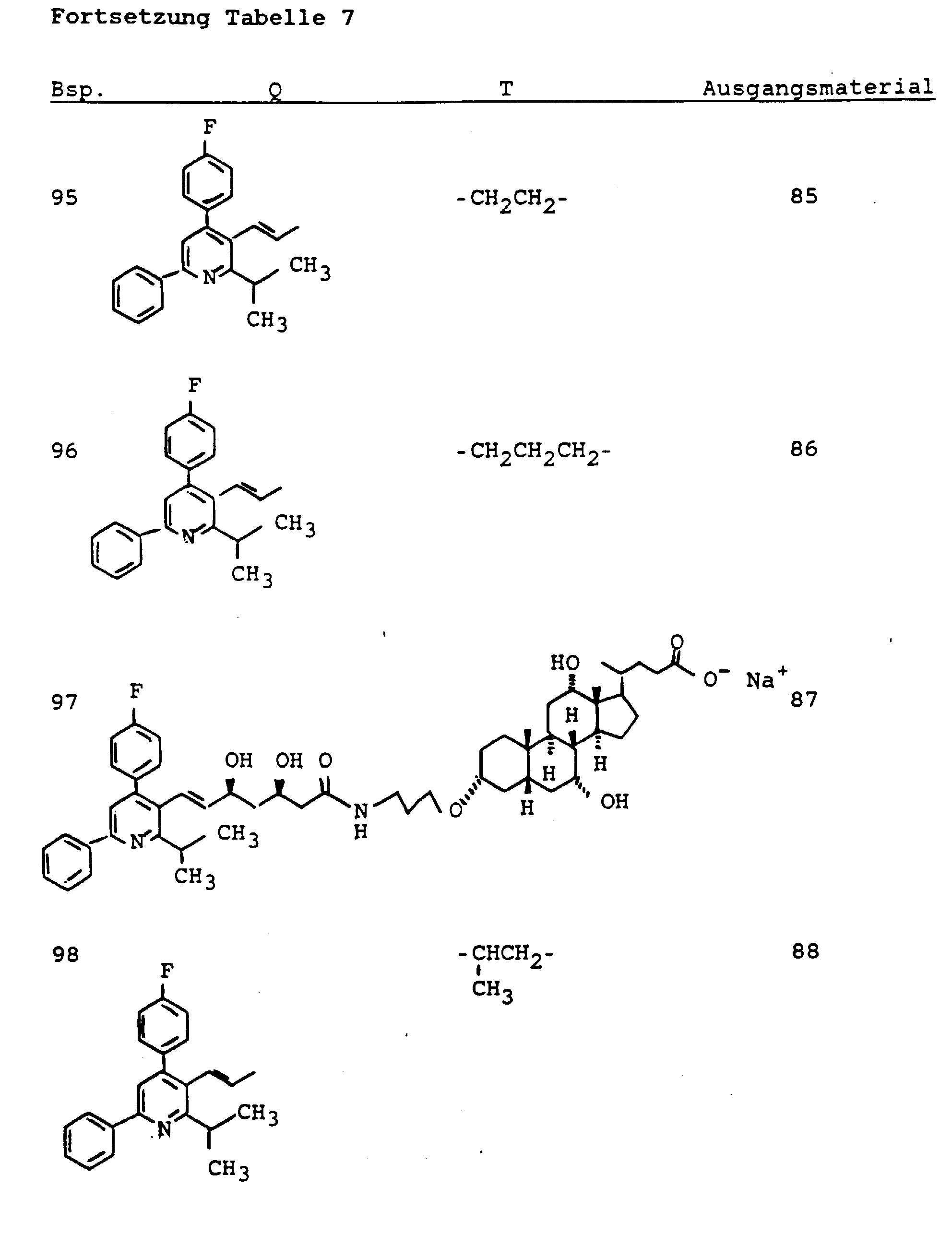

### Beispiele 99-108

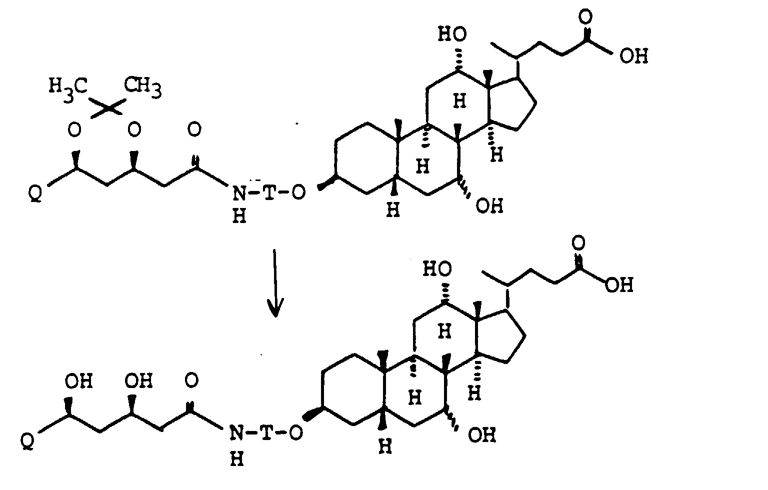

### Beispiel 99

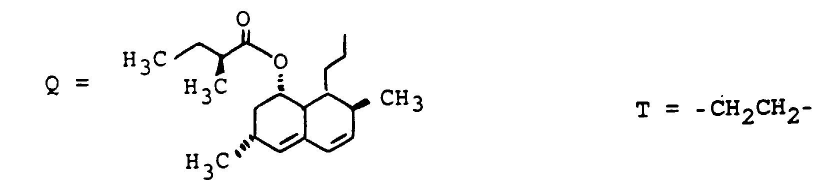

400 mg (0.45 mmol) Acetonid aus 69 (s. Beispiele 79-88, Variante B) wurden in 40 ml absolutem THF gelöst, mit 0.12 ml (0.90 mmol) Triethylamin versetzt und auf 0°C gekühlt. Es wurden 0.07 ml (0.68 mmol) Chlorameisensäureethylester zugegeben und 15 min gerührt. Anschließend wurde eine Lösung von 100 mg (1.3 mmol) Glycin in 12 ml 0.1 N wäßriger Natronlauge zugetropft. Es wurde auf Zimmertemperatur erwärmt und noch 1h gerührt. Das Gemisch wurde auf Wasser gegossen, mit 2N Salzsäure angesäuert und mit Essigester extrahiert (3x). Die vereinigten organischen Phasen wurden mit MgSO₄ getrocknet und eingedampft. Der Rückstand wurde in 50 ml THF gelöst, mit 10 ml 1N Salzsäure versetzt und 4h bei Zimmertemperatur gerührt. Es wurde auf Wasser gegossen, mit Essigester extrahiert (3x), die vereinigten organischen Phasen mit MgSO₄ getrocknet und eingedampft. Flashchromatographie auf Kieselgel (Chloroform/Methanol 7:3) ergab 230 mg (0.25 mmol, 56 %) "Beispiel 99"
C₅₂H₈₄N₂O₁₁ (912), MS (FAB, 3-NBA/LiCl): 919 (M+Li⁺)

### Beispiele 100 - 108

Analog zu 99 wurden die "Beispiele 100-108" erhalten.

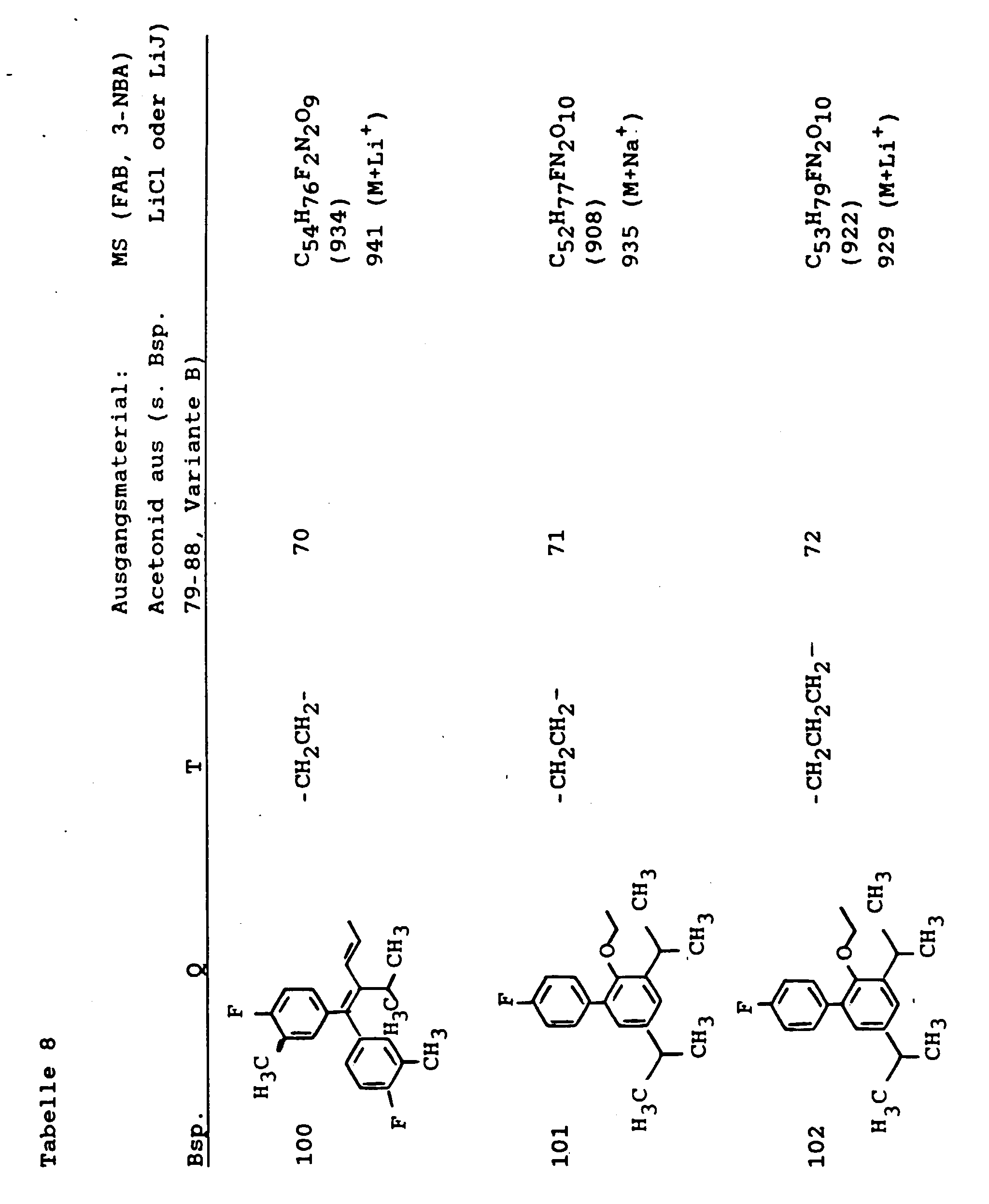

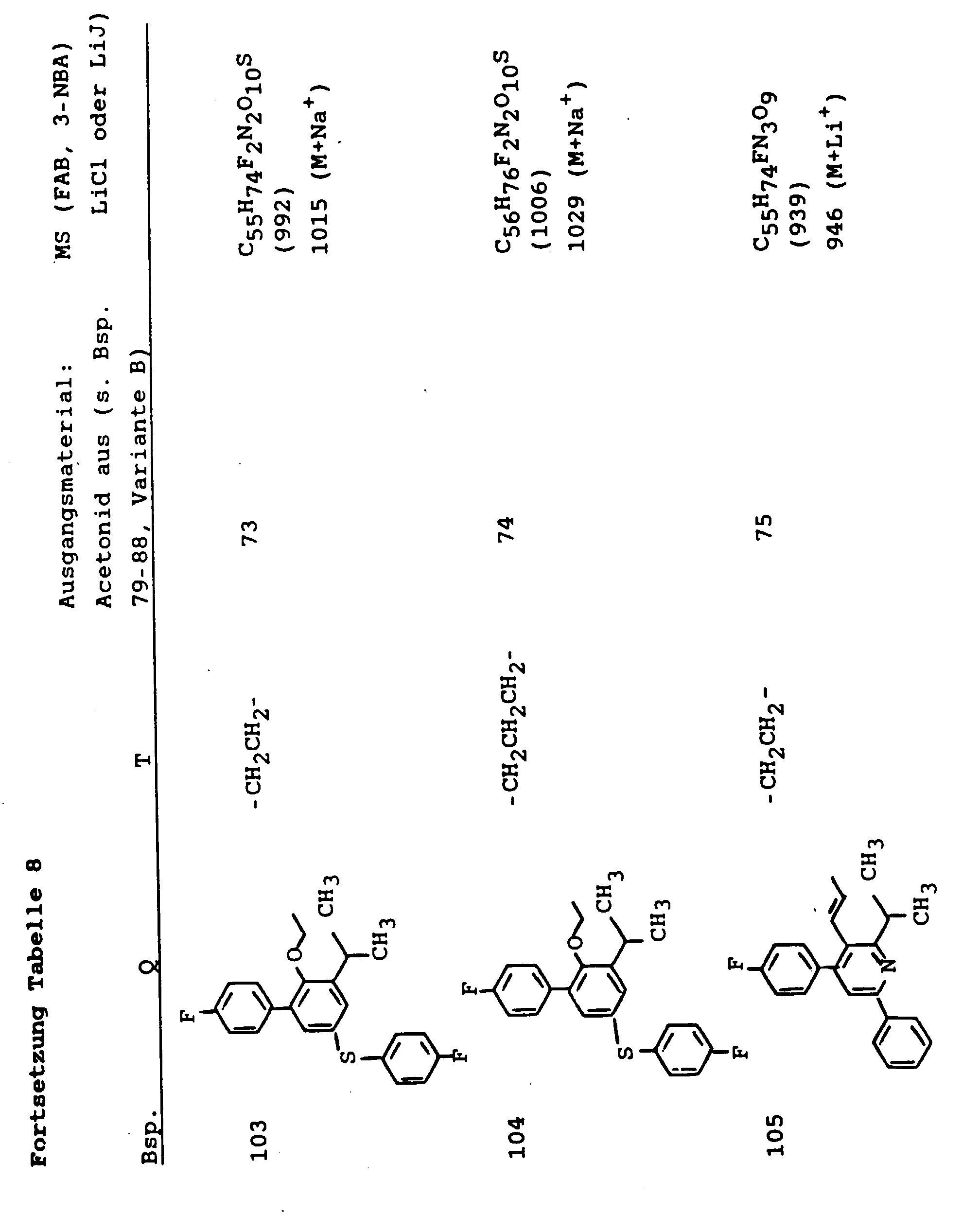

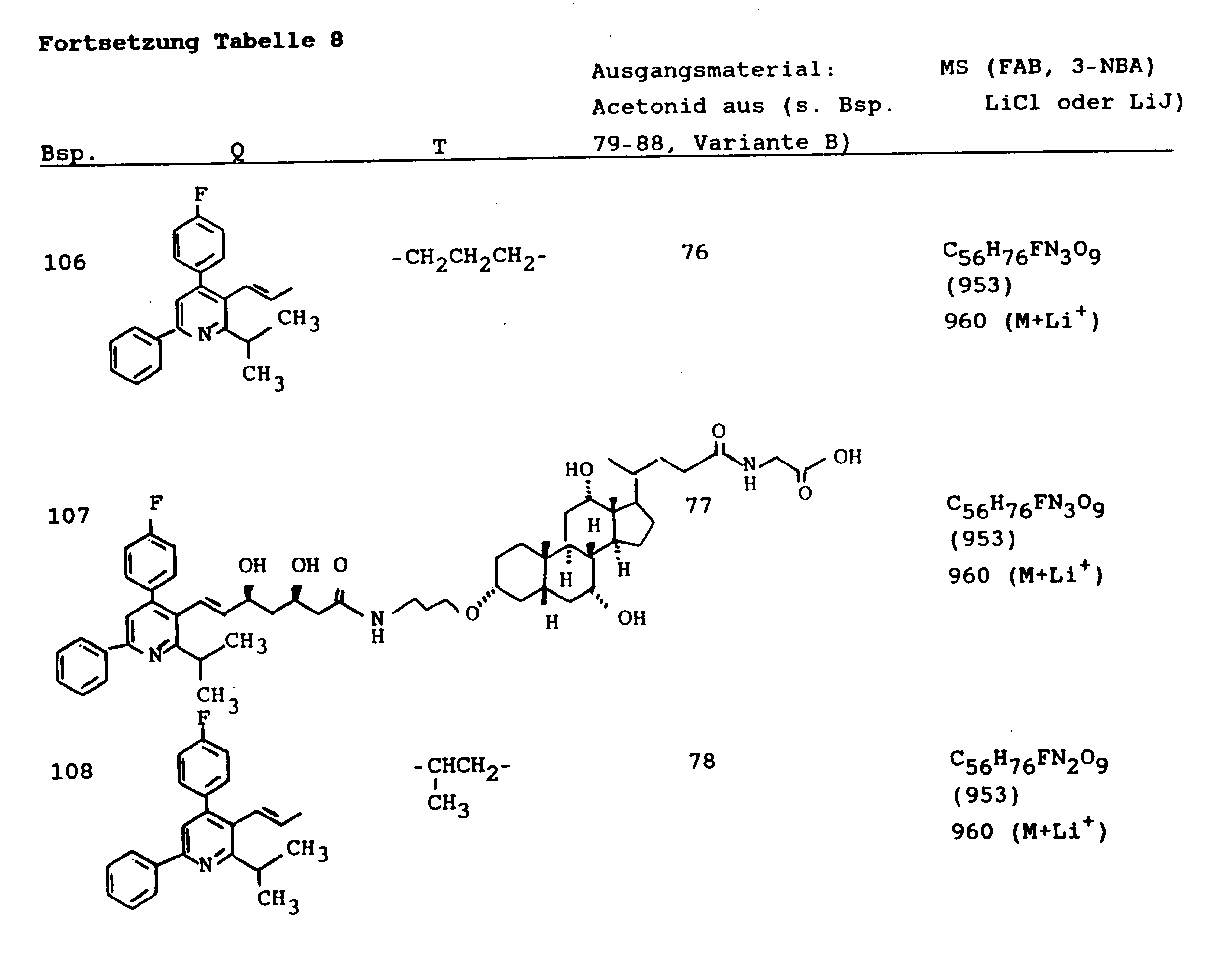

Analog zu den Beispielen 89-98 wurden aus den Beispielen 100-108 ihre Na-Salze hergestellt

### Beispiele 109-118

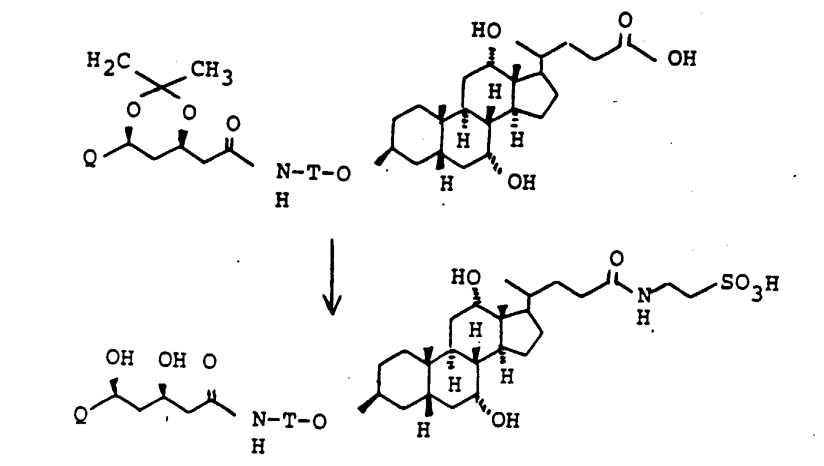

### Beispiel 109

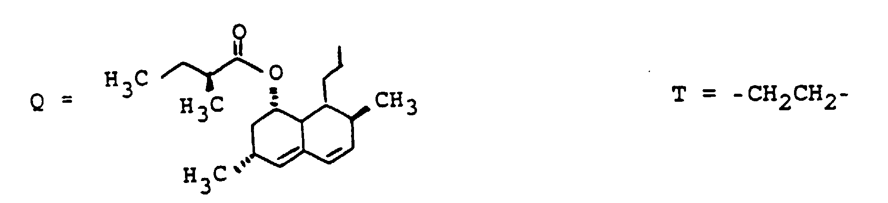

400 mg (0.45 mmol) Acetonid aus 69 (s. Beispiele 79-88, Variante B) wurden in 40 ml absolutem THF gelöst, mit 0.12 ml (0.90 mmol) Triethylamin versetzt und auf 0°C gekühlt. Es wurden 0.07 ml (0.68 mmol) Chlorameisensäureethylester zugegeben und 15 min gerührt. Anschließend wurde eine Lösung von 160 mg (1.3 mmol) Taurin in 12 ml 0.1 N wäßiger Natronlauge zugetropft. Es wurde auf Zimmertemperatur erwärmt und noch 1h gerührt. Das Gemisch wurde auf Wasser gegossen, mit 2N Salzsäure angesäuert und mit Essigester extrahiert (3x). Die vereinigten organischen Phasen wurden mit MgSO₄ getrocknet und eingedampft. Der Rückstand wurde in 50 ml THF gelöst, mit 10 ml 1N Salzsäure versetzt und 4h bei Zimmertemperatur gerührt. Es wurde auf Wasser gegossen, mit Essigester extrahiert (3x), die vereinigten organischen Phasen mit MgSO₄ getrocknet und eingedampft.
Flashchromatographie auf Kieselgel (Dichlormethan/Methanol 7:3) ergab 270 mg (0.28 mmol, 62 %) "Beispiel 109".
C₅₂H₈₆N₂O₁₂S (962), MS (FAB, 3-NBA/LiCl): 969 (M+Li⁺).

### Beispiele 110 - 118

Analog zu 109 wurden die "Beispiele 110-118" erhalten

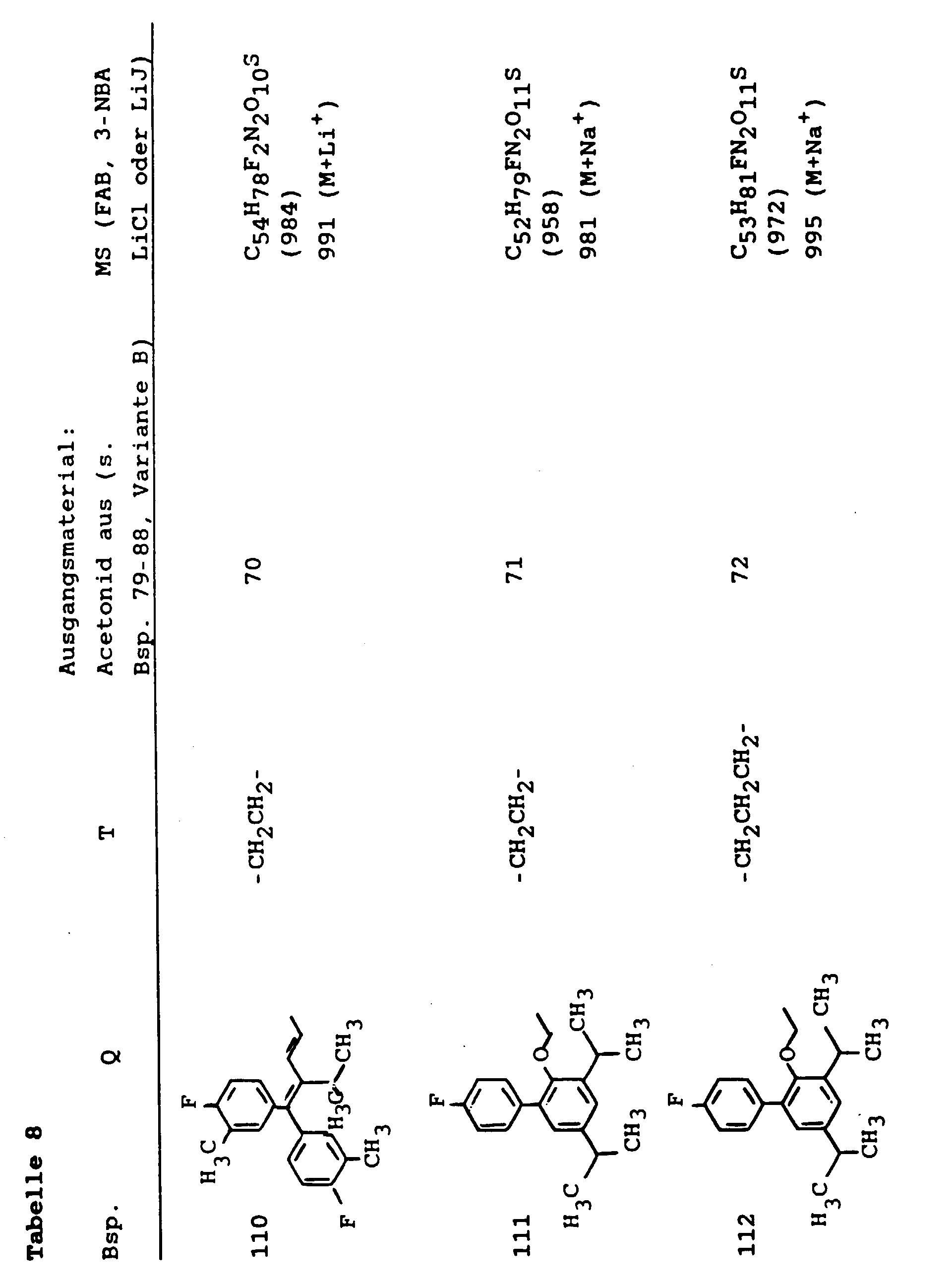

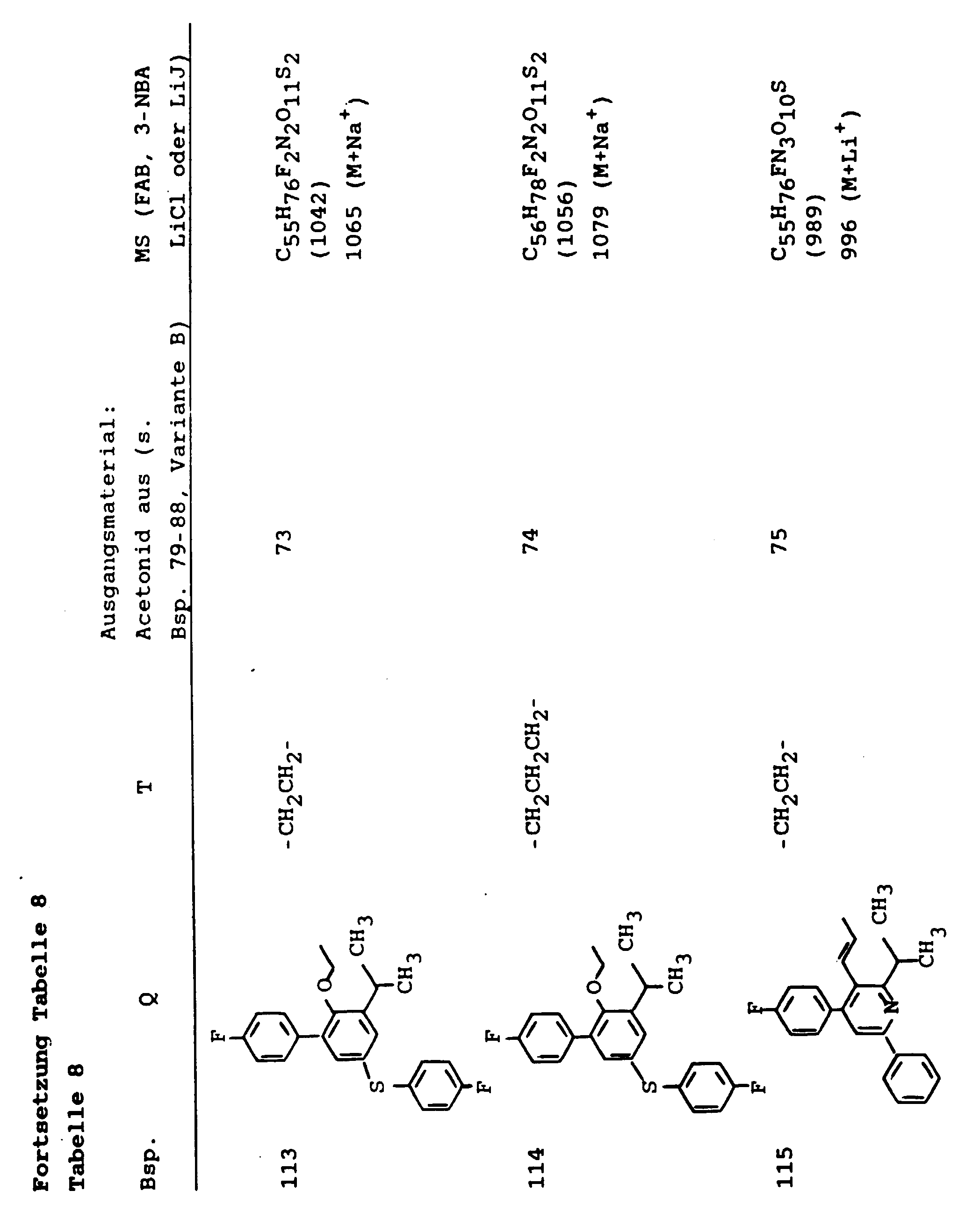

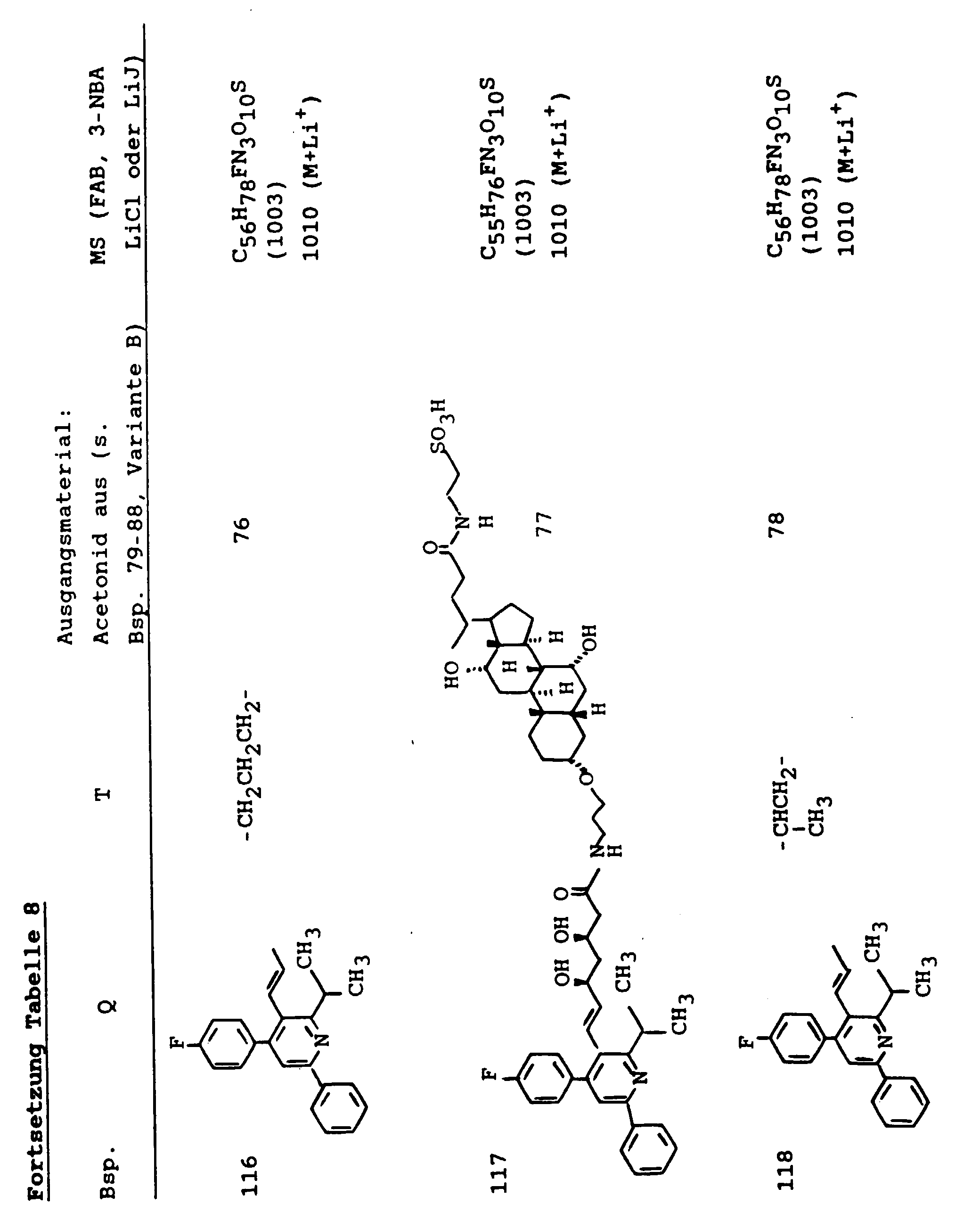

Analog zu den Beispielen 89-98 wurden aus den Beispielen 109-118 ihre Na-Salze hergestellt.
W - X - G, W = Modellpeptid (D-Alanyl-Peptide)

### Herstellung von Modellpeptiden zur Kupplung an Gallensäuren

Die für die Kupplung an Beispiel 21 eingesetzten N-terminalgeschützten D-Alanyl-Peptide (Schutzgruppe z.B. Benzyloxycarbonyl bzw. Boc: tert-Butyloxycarbonyl) bzw. deren Aktivester werden nach in der Peptidchemie allgemein üblichen Methoden (s. z.B. Houben-Weyl, Bände 15/1 und 15/2) hergestellt und charakterisiert.

Die N-terminal geschützten Oligo-D-Alanyl-Peptide werden entweder in Form ihrer Aktivester, z.B. als N-Hydroxysuccinimid (OSu)- bzw. 1-Hydroxybenzotriazol (OBt)-Ester, oder mit Hilfe von Kondensationsreagenzien (z.B. Dicyclohexylcarbodiimid) unter Zusatz eines racemisierungshemmenden Reagenzes, beispielsweise N-Hydroxysuccinimid (HOSu) bzw. 1-Hydroxybenzotriazol (HOBt) mit der Aminofunktion des Gallensäurederivats verknüpft.

Nachfolgend werden die Peptidschutzgruppe sowie die Methylestergruppe des Gallensäurederivats entfernt, wobei durch die hier verwendete orthogonale Schutzgruppen-Strategie - die N-terminale Schutzgruppe kann z.B. durch Hydrierung (Z) oder acidolytisch (Boc) abgespalten werden, während die Methylester-Gruppe des Gallensäurederivats verseift wird - auch die Synthese von teilgeschützten Gallensäure-Peptid-Konjugaten möglich ist.

Die jeweiligen Zwischenprodukte sowie das Endprodukt werden i.a. säulenchromatographisch gereinigt und dünnschichtchromatographisch sowie mittels ¹H-NMR-Spektroskopie charakterisiert.

### Beispiel 119-121

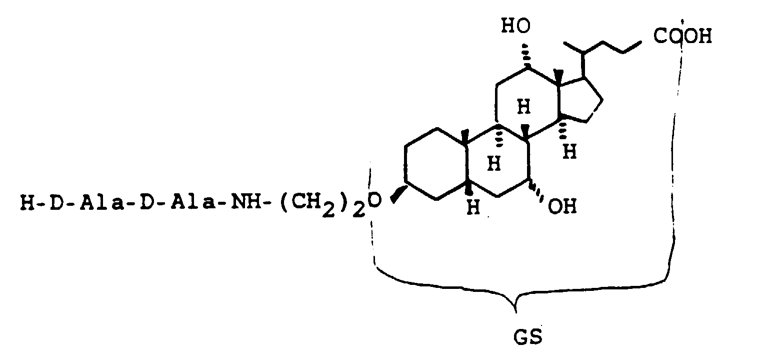

### Beispiel 119

### Z-D-Ala-D-Ala-NH-(CH₂)₂-GS-OCH₃

Man löst 485 mg (1.04 mmol) Beispiel 21 und 467 mg (1.14 mmol) Z-D-Ala-D-Ala-OSu in 10 ml Dichlormethan und rührt 1.5 h bei Zimmertemperatur. Nach Entfernen des Lösungsmittels im Vakuum erhält man 930 mg festen Rückstand, der über eine Kieselgelsäule (50x2 cm, Matrex Silicagel 70-200 µm) chromatographiert wird. Eluent: Dichlormethan/Methanol/Essigsäure 85:10:5.

Die Fraktionen 3 und 4 enthalten das gewünschte Produkt; Fraktion 4 enthält zusätzlich noch N-Hydroxysuccinimid, welches nach Entfernen des Lösungsmittels durch Verreiben des Rückstands mit Wasser bzw. stark verdünnter Salzsäure entfernt werden kann.

Nach Vereinigung beider Fraktionen erhält man 450 mg (57 %) "Beispiel 119" als weißen Feststoff.
R_{f} (Dichlormethan/Methanol/Essigsäure 85:10:5): 0.76.
R_{f} (n-Butanol/Essigsäure/Wasser 40:40:10): 0.89.

### Beispiel 120

### H-D-Ala-D-Ala-NH-(CH₂)₂-GS-OCH₃

280 mg (0.37 mmol) Z-D-Ala-D-Ala-NH-(CH₂)₂-GS-OCH₃ werden in 5 ml Methanol gelöst. Man spült das Reaktionsgefäß mehrere Male mit Inertgas, gibt 28 mg Hydrierkatalysator (Palladium/Aktivkohle, 10 %) zu und hydriert 3h bei Zimmertemperatur. Nach der angegebenen Zeit ist laut Dünnschichtchromatographie (Dichlormethan/Methanol/Essigsäure 85:10:5) kein Edukt mehr vorhanden. Die Reaktionsmischung wird membranfiltriert (Schleicher u. Schuell, RC 58, 0.2 µm), das Filtrat im Rotationsverdampfer eingedampft, der Rückstand zweimal mit Diethylether versetzt und erneut im Rotationsverdampfer eingedampft. Man erhält 225 mg (98 %) weiß-gelben pulvrigen Feststoff, der in Methanol aufgenommen und 10 min unter Zusatz von Aktivkohle bei Zimmertemperatur gerührt wird. Nach Filtration, Verdampfen des Lösungsmittels i.Vak. und Trocknen erhält man 190 mg (83 %) "Beispiel 120" als weißes Pulver.
R_{f} (n-Butanol/Essigsäure/Wasser 40:40:10): 0.55.

### Beispiel 121

### H-D-Ala-D-Ala-NH-(CH₂)₂-GS-OH

Man löst 120 mg (0.19 mmol) H-D-Ala-D-Ala-NH-(CH₂)₂-GS OCH₃ in 2 ml Methanol, tropft bei Zimmertemperatur 2 ml 0.1 N wäßrige Natriumhydroxid-Lösung zu und rührt 4h bei gleicher Temperatur. Anschließend stellt man die Reaktionslösung mit 2N wäßriger Salzsäure auf pH 3 und dampft im Rotationsverdampfer bis zur Trockne ein.

Man verreibt den Rückstand in wenig Ethanol und filtriert von unlöslichem Material ab. Das Filtrat wird zur Trockne gebracht jeweils einmal mit Ether und Pentan verrieben, filtriert und getrocknet. Man erhält 107 mg "Beispiel 121" als weißen Feststoff (enthält noch ca. 10 % Natriumchlorid).
Ausbeute: 85 %
R_{f} (n-Butanol/Essigsäure/Wasser 40:40:10): 0.54.

In Analogie zu Beispiel 121 wurde Beispiel 122 erhalten.

### Beispiel 122

### Z-D-Ala-D-Ala-NH-(CH₂)₂-GS-OH

Z = Benzyloxycarbonyl

### Beispiel 123

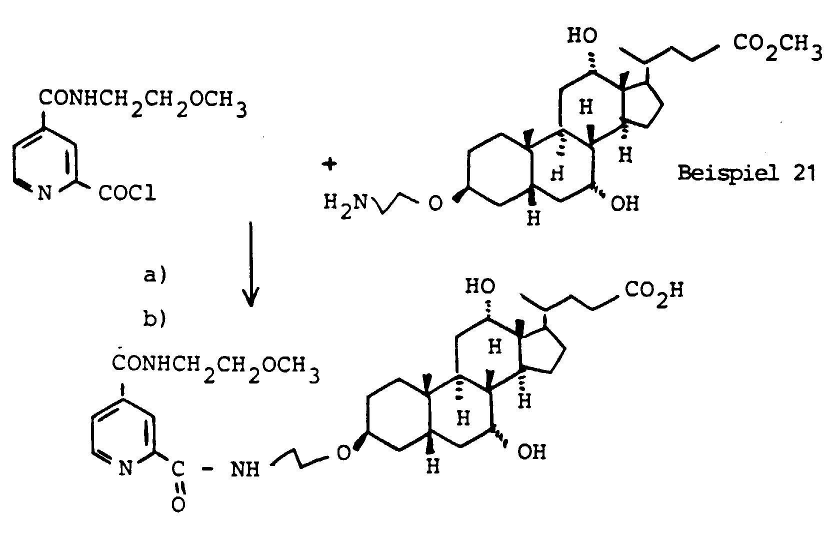

a) Zu 500 mg (2.23 mmol) Säurechlorid (hergestellt aus der entsprechenden Carbonsäure durch Umsetzung mit Thionylchlorid/katalytische Menge DMF, 2h Rückfluß), in 25 ml Methylenchlorid wurden bei 0°C 1.04 g (2.23 mmol) Beispiel 21 in 5 ml Methylenchlorid/1 ml Triethylamin getropft. Anschließend ließ man 2h bei Zimmertemperatur rühren. Das Reaktionsgemisch wurde auf Wasser gegossen und die wäßrige Phase mit Methylenchlorid extrahiert (3x). Die vereinigten organischen Phasen wurden mit gesättigter NaHCO₃-Lösung gewaschen (lx) und getrocknet (MgSO₄). Abziehen des Lösungsmittels und Chromatographie auf Kieselgel (Essigester/Methanol = 15:1) ergab 314 mg.
b) 100 mg (1.49 mmol) des nach a) erhaltenen Produktes wurden in 20 ml Ethanol gelöst und mit 4 ml 1 N NaOH über Nacht gerührt. Es wurden 4 ml Salzsäure zur Neutralisation zugegeben und mit Essigester extrahiert. Die vereinigten organischen Phasen wurden mit gesättigter Natriumchloridlösung gewaschen und getrocknet (MgSO₄). Abziehen des Lösungsmittels ergab 89.1 mg "Beispiel 123".
   C₃₆H₅₅N₃O₈ (657); MS (FAB, 3-NBA/LiJ): 664 (M+Li⁺).

### Beispiel 124

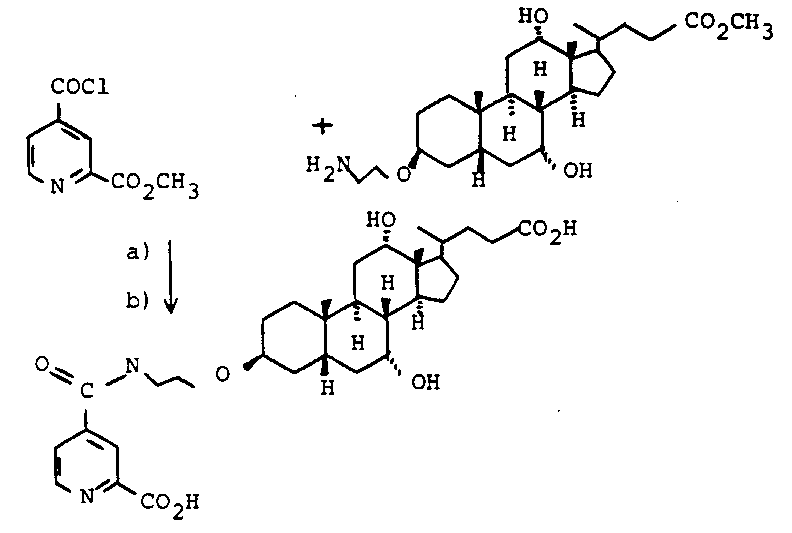

Analog zu Beispiel 123 wurde "Beispiel 124" erhalten.
C₃₃H₄₈N₂O₈ (600); MS (FAB, 3-NBA/LiCl): 607 (M+Li⁺), 613 (M+2Li-H)⁺, 619 (M+3Li-2H)+.

Herstellungsvorschrift der Lactonkomponente für Beispiel 71 nach DE 38 19 999:

### Synthese von 4(R)-Hydroxy-6(S)-[(2,4-diisopropyl-6-p-fluorphenyl)-phenoxymethyl]tetrahydro-2H-pyran-2-on

### Stufe 1

### 2,4-Diisopropylphenol

Das Gemisch aus 145 g (0,65 Mol) 3,5-Diisopropyl-2-hydroxybenzoesäure (XI), 540 ml (588 g, 4,55 Mol) Chinolin und 7,5 g (0,024 Mol) Kupferchromit (2CuO·Cr₂O₃) wird 2 Stunden bei 190°C (225°C Außentemperatur) gerührt. Man kühlt auf ca. 10°C, säuert unter weiterer Kühlung mit ca. 1 1 halbkonzentrierter Salzsäure auf pH 1 bis 2 an, extrahiert mit Toluol und wäscht das Extrakt mit 2 N Salzsäure, dann mit Wasser und dann mit NaHCO₃-Lösung. Man trocknet, filtriert, engt ein und destilliert im Hochvakuum. Man erhält 105 g der Titelverbindung XIII als hellgelbes Öl, Sdp. 81 bis 84°C/0,2 Torr
- ¹H-NMR(CDCl₃):: δ 1,20 (6H,d); 1,25 (6H,d); 3,00 (2H,2xhept.); 4,10 (1H,s,br); 6,50-7,00 (3H,m)

### Stufe 2

### 2,4-Diisopropyl-6-bromphenol

Zur 95°C warmen Lösung von 102,3 g (0,57 Mol) 2,4-Diisopropylphenol in 900 ml Eisessig gibt man 1 g Eisenpulver, dann tropfenweise 101 g (32,2 ml, 0,63 Mol) Brom innerhalb 90 Minuten. Man rührt noch 1 Stunde bei 100°C, kühlt ab, verteilt das Reaktionsgemisch zwischen Toluol und Wasser und wäscht die Toluolphase mit NaHCO₃-Lösung. Man trocknet, filtriert, engt ein und destilliert den Rückstand im Hochvakuum. Man erhält 125 g der Titelverbindung als hellgelbes Öl, Sdp. 85°C/0,15 Torr
- ¹H-NMR(CDCl₃):: δ 1,20 (6H,d); 1,25 (6H,d), 2,80 (1H,hept.); 3,25 (1H,hept.); 5,33 (1H,s); 6,87-7,20 (2H,m)
- MS (70eV):: m/e = 256/258 (M⁺), 241/243 (M⁺-CH₃)

### Stufe 3

### 1-Benzyloxy-2,4-diisopropyl-6-brombenzol

Die Suspension von 166,5 g (1,2 Mol) Kaliumcarbonat in 124 g (0,48 Mol) des obigen Bromphenols, 91,52 g (0,72 Mol) Benzylchlorid und 2 1 2-Butanon wird 24 Stunden zum Rückfluß erhitzt.
Man kühlt ab, saugt den anorganischen Feststoff ab, engt das Filtrat im Vakuum ein und verteilt den Rückstand zwischen Toluol und Wasser. Die Toluolphase wird mit gesättigter Kochsalzlösung gewaschen, getrocknet, filtriert und eingeengt. Der Rückstand wird mit Cyclohexan/Toluol 9:1 über Kieselgel chromatographiert. Man erhält 155 g der Titelverbindung als farbloses Öl.
Geringe Reste Benzylchlorid werden im Hochvakuum entfernt. Man kann die Reinigung auch durch Destillation erreichen (Sdp. 150 °C/0,15 Torr).
- ¹H-NMR(CDCl₃):: δ 1,18 (6H,d); 1,22 (6H,d), 2,80 (1H,hept.); 3,32 (1H,hept.); 4,90 (2H,s); 6,93-7,60 (7H,m)
- MS (70eV):: m/e = 346/348 (M⁺), 267, 254/256, 91

### Stufe 4

### 1-Benzyloxy-2,4-diisopropyl-6-p-fluorphenyl-benzol

Man stellt die Grignard-Verbindung aus 48,62 g (0,14 Mol) des Bromids aus Stufe 3 und 3,53 g (0,147 Mol) Mg-Spänen in 120 ml absolutem THF her (∼ 60°C, 1 Stunde).
Diese Grignard-Lösung wird rasch zur Lösung von 31,08 g (0,14 Mol) 4-Fluorjodbenzol und 3,23 g (2,8 mMol)
Tetrakis(triphenylphosphin)palladium (O) in 140 ml absolutem THF gegeben. Die Innentemperatur steigt innerhalb von 15 Minuten auf 55 bis 60°C. Nach 7 Minuten bildet sich ein Niederschlag. Man rührt 1 Stunde bei 50 bis 58°C, läßt über Nacht bei Raumtemperatur stehen, verteilt zwischen Ether und 1 N Salzsäure, wäscht die Etherphase mit 1 N Salzsäure, dann mit Wasser und dann mit gesättigter NaHCO₃-Lösung. Man trocknet, filtriert und engt ein. Bei Bedarf reinigt man das Produkt durch Chromatographie mit Cyclohexan/Toluol 4:1 über Kieselgel oder durch Destillation (Sdp. 180 °C/0,3 Tor). Man erhält 49,3 g der Titelverbindung als farblosen Feststoff, Schmp. 65 bis 67°C
- ¹H-NMR(CDCl₃):: δ 1,30 (12H,d); 2,95 (1H,hept.); 3,45 (1H,hept.); 4,40 (2H,s); 6,90-7,80 (11H,m)
- MS (CI):: m/e = 363 (M+H⁺), 362 (M⁺), 285, 263

### Stufe 5

### 2,4-Diisopropyl-6-p-fluorphenyl-phenol

Zur Lösung von 49,3 g (0,136 Mol) des Benzylethers aus Stufe 4 in 1 l Essigester und 100 ml Eisessig gibt man 4 g 10 % Pd auf Kohle und schüttelt 20 Minuten in einer Wasserstoffatmosphäre (lebhafte H₂-Aufnahme). Man filtriert den Katalysator ab, engt ein, nimmt den Rückstand mehrmals in Toluol auf und engt jeweils im Vakuum ein. Man erhält 34,4 g der Titelverbindung als farbloses Öl,
Sdp. 115 °C/0,1 Torr.
- ¹H-NMR(CDCl₃,270MHz):: δ 1,25 (6H,d); 1,29 (6H,d); 2,87 (1H,hept.); 3,31 (1H,hept.); 4,95 (1H,s,br); 6,88(1H,d); 7.08 (1H,d) 7,18 (2H,m); 7,45 (2H,m).
- MS (70eV):: m/e = 272 (M⁺), 257 (M⁺-CH₃)

### Stufe 6

### 6(S)-{(2,4-Diisopropyl-6-p-fluorphenyl)-phenoxymethyl}-3,4,5,6-tetrahydro-2(R,S)-methoxy-4(R)-(t-butyldiphenylsilyloxy)-2H-pyran

Zur Suspension von 27.6 g (0,2 Mol) Kaliumcarbonat und einer Spatelspitze Hydrochinon in 250 ml abs. DMSO gibt man 27,2 g (0,1 Mol) des Phenols aus Stufe 5. Man rührt 1 Std. bei Raumtemperatur und gibt dann die Lösung von 56 g (0,11 Mol) 6(S)-Jod-methyl-3,4,5,6-tetrahydro-2(R,S)-methoxy-4(R)-(t-butyldiphenylsilyoxy)-2-H-pyran (Herstellung siehe EP-A 0 216 127, R₇ = t-Butyldiphenylsilyl) in 250 ml abs. DMSO zu. Man rührt 4 Std. bei 50-55 °C Innentemperatur. DC (Kieselgel, 1. Entwicklung mit Cyclohexan/Essigester 9:1, 2. Entwicklung mit Cyclohexan/Essigester 15:1) zeigt vollständigen Umsatz des Jodids (R_{f} 0,5), etwas restliches Ausgangsphenol (R_{f} 0,7), hauptsächlich Produkt der Formel V (R_{f} 0,6) an. Man läßt abkühlen und verteilt das Reaktionsgemisch zwischen Ether und halbgesättigter Kochsalzlösung. Die wäßrige Phase wird nochmals mit Ether extrahiert. Die vereinigten organischen Phasen werden mit Kochsalzlösung gewaschen, über MgSO₄ getrocknet, filtriert und eingeengt. Das Rohprodukt wird mit Toluol/Cyclohexan 2:1; dann 100 % Toluol, dann Toluol/Essigester 30:1 über Kieselgel chromatographiert. Man erhält 51 g der Titelverbindung als farbloses Harz.
- ¹H-NMR(CDCl₃):: δ 1,10 (9H,s); 1,28 (12H,d), 1,4-2,2 (4H,m); 2,93 (2H,2xhept.); 3,40 (2H,m); 3,52 (3H,s); 3,97-4.40 (2H,qui+m); 4,87 (1H,dd); 6,87-7,90 (16H,m)
- MS (CI):: m/e = 654 (M⁺), 597 (M⁺-tert.-Bu), 539, 519, 323, 283, 135, 127

### Stufe 7

### 6(S)-{2,4-Diisopropyl-6-p-fluorphenyl)-phenoxymethyl}-3,4,5,6-tetrahydro-2-(R,S)-hydroxy-4(R)-(t-butyldiphenylsilyloxy)-2H-pyran

Die Lösung von 40,2 g (61,4 mMol) des Laktolethers aus Stufe 6 in 3 1 THF, 3 1 Wasser und 4,2 1 Eisessig wird 24 Std. bei 80-85 °C (Außentemperatur) gerührt. Die Lösungsmittel werden im Vakuum entfernt und der Rückstand 3 mal mit Toluol im Vakuum abgeraucht. Chromatographie mit Cyclohexan/Essigester 12:1 durch 2 1 Kieselgel gibt 33,4 g (Ausbeute 85 %) der Titelverbindung als farbloses amorphes Pulver.
MS (FAB): m/e = 640 (M⁺), 519, 367, 323, 283, 271, 257

### Stufe 8

### 6(S)-{2,4-Diisopropyl-6-p-fluorphenyl)-phenoxymethyl}-3,4,5,6-tetrahydro-4(R)-(t-butyldiphenylsilyloxy)-2H-pyran-2-on

Zur Lösung von 33,4 g (52,1 mMol) des Lactols aus Stufe 7 und 19,25 g (52,1 mMol) Tetrabutylammoniumjodid in 2,5 1 absolutem Methylenchlorid gibt man unter Rühren und Kühlung 46,9 g (208,4 mMol) N-Jodsuccinimid. Man rührt unter Lichtausschluß unter Stickstoff 1 Stunde bei 10°C, 20 Stunden bei Raumtemperatur. Man wäscht die Reaktionslösung mit Wasser, dann 2mal mit NaHSO₃-Lösung, dann mit gesättigter NaCl-Lösung, trocknet, filtriert und engt ein. Der Rückstand wird in wenig Methylenchlorid gelöst und mit Cyclohexan/Essigester 92:8 durch Kieselgel filtriert. Man erhält 32,1 g der Titelverbindung als farbloses Harz.
- ¹H-NMR(CDCl₃,270MHz):: δ 1,06 (9H,s); 1,23 (6H,d); 1,26 (6H,d); 1,59 (2H,m); 2,41 (1H,dd); 2,59 (1H,dm); 2,90 (1H,hept.); 3,36 (1H,hept.); 3,48 (2H, AB von ABX); 4,29 (1H,qui); 4,80 (1H,m); 6,96 (1H,d); 7,03 (2H,m); 7,10 (1H,d); 7,36-7,52 (8H,m); 7,58-7,73 (4H,m)
- MS (70eV,70°C):: m/e = 638 (M⁺), 581 (M⁺-tert.-Bu), 539 (581 - Propen), 283, 199

### Stufe 9

### 4(R)-Hydroxy-6(S)-[(2,4-diisopropyl-6-p-fluorphenyl)-phenoxymethyl]-tetrahydro-2H-pyran-2-on

Zur Lösung aus 31,0 g (48,5 mMol) der Silylverbindung aus Stufe 8 in 1.5 1 Tetrahydrofuran (über bas. Al₂O₃ filtriert) gibt man 11,65 g (194 mMol) Eisessig, gefolgt von 45,92 g (145,5 mMol) Tetrabutylammoniumfluorid-Trihydrat. Man rührt 20 Std. bei Raumtemperatur. Die Lösungsmittel werden im Vakuum entfernt und der Rückstand wird sofort zwischen Ether und Wasser verteilt. Die wäßrige Phase wird noch zweimal mit Ether extrahiert. Die vereinigten organischen Phasen werden mit gesättigter Kochsalzlösung gewaschen, über MgSO₄ getrocknet, filtriert und eingeengt.
Der Rückstand wird in Toluol aufgenommen und im Vakuum eingeengt. Das Rohprodukt wird mit Cyclohexan/Essigester 1:1 durch 2 kg Kieselgel chromatographiert.
Man erhält 15,7 g (Ausbeute 81 %) der Titelverbindung als farblosen Feststoff, Schmp. 145-147 °C.
- ¹H-NMR(CDCl₃,270MHz):: δ 1,25 und 1,27 (12H,2xd); 1,67 (1H,s,br.); 1,76 (1H,dtd); 1,87 (1H,ddd); 2,58 (1H,ddd); 2,69 (1H,dd); 2,91 (1H,hept); 3,39 (1H,hept), 3,54 (2H,AB von ABX); 4,38 (1H,qui), 4,68 (1H,m); 6,97 (1H,d); 7,10 (3H,d+m); 7,51 (2H,m)
MS (FAB): m/e = 400 (M⁺), 257

Herstellungsvorschrift für die Lactonkomponente für Beispiel 73 nach DE 39 29 913

### Stufe 1

### 2-Brom-6-isopropyl-phenol

198,1 ml (3,85 Mol) Brom wurden bei -5 bis 0°C zur Lösung von 470 g Natriumhydroxid in 2 l Wasser getropft. Das Gemisch wurde noch 10 Min. bei dieser Temperatur gerührt. Die entstandene Natriumhypobromid-Lösung wurde bei -5 bis 0°C zur Lösung von 464 g einer 40 %igen wäßrigen Dimethylamin-Lösung (4,11 Mol) in 50 ml Wasser getropft. Das Gemisch wurde noch 30 Min. gerührt, dann wurde die organische Phase abgetrennt und die wäßrige Phase mit 2 mal 750 ml Methylenchlorid extrahiert. Die vereinigten organischen Phasen wurden kurz über Magnesiumsulfat getrocknet und filtriert. Das Filtrat wurde bei -10°C zur Lösung von 500 g (3,67 Mol) ortho-Isopropylphenol in 900 ml Methylenchlorid getropft. Nach Zugabe von ca. 2/3 des Filtrats bildete sich ein Feststoff und das Reaktionsgemisch wurde viskos und schwer rührbar. 500 ml Methylenchlorid wurden bei -10°C zugegeben und das Gemisch wurde eine weitere Stunde gerührt. Der Feststoff wurde abgesaugt, mit wenig kaltem Methylenchlorid gewaschen, in 1,5 l 2N Schwefelsäure suspendiert und bei Raumtemperatur gerührt, bis sich sämtlicher Feststoff in ein Öl umgewandelt hatte. Die organische Phase wurde abgetrennt, die wäßrige Phase mit Methylenchlorid extrahiert. Die vereinigten organischen Phasen wurden mit Kochsalzlösung gewaschen, getrocknet und das Lösungsmittel wurde im Vakuum entfernt. Der Rückstand wurde im Wasserstrahlvakuum durch eine 30 cm Vigreux-Kolonne destilliert.
391,7 g (1,82 Mol) farbloses Öl, Sdp. 122-124°C/21 Torr;
Ausbeute 49,6 %
- NMR (60 MHz) :: δ = 1,20 (d, 6H, CH₃), 3,23 (sept., 1H, CH), 5,42 (s, 1H, OH), 6,4-7,2 (m, 3H, arom.-H).

### Stufe 2

### 2-(p-Fluorphenyl)-6-isopropyl-phenol

**a)** Zu 18,7 g (0,77 Mol) Magnesiumspänen wurden drei Jodkristalle gegeben und die Zugabestelle mit einem Heißluftgerät (®Fön) erhitzt, bis Joddämpfe im Kolben sichtbar waren. Es wurde auf Raumtemperatur abgekühlt und 20 ml absol. THF zugegeben. 131,3 g (0,75 Mol) p-Bromfluorbenzol wurden in einen 500 ml Tropftrichter gefüllt und davon ca. 2 ml in den Reaktionskolben gegeben. Die braune Farbe des Reaktionsgemischs verschwand rasch und es erfolgte starke Wärmeentwicklung zum Rückfluß. Sofort wurden weitere 50 ml absol. THF zum Reaktionsgemisch gegeben und das p-Bromfluorbenzol im Tropftrichter mit 200 ml THF verdünnt. Diese Lösung wurde dann so zugetropft, daß ein gelinder Rückfluß aufrecht erhalten wurde. Anschließend wurde das Reaktionsgemisch noch 1 Stunde unter Rückfluß gekocht und dann auf 50°C abgekühlt.
**b)** In einem zweiten Kolben wurde aus der Lösung von 52,0 g (0,24 Mol) 2-Brom-6-isopropyl-phenol in 150 ml absol. THF mittels 20 minütigem Durchleiten von Stickstoff der gelöste Sauerstoff vertrieben. 1,7 g (1,5 mMol) Tetrakis(triphenylphosphin)-palladium(O) wurden unter Minimierung des Sauerstoffkontakts zugegeben.

Dann wurde die Grignard-Lösung aus Stufe a) mittels einer Doppelkanüle ("®Flex-Needle", Firma Aldrich) unter Stickstoffdruck in diese Lösung transferiert, wobei eine Wärmeentwicklung auftrat. Die Geschwindigkeit des Transfers wurde so gewählt, daß ein gelinder Rückfluß aufrecht erhalten wurde. Anschließend wurde noch 6 Stunden zum Rückfluß erhitzt. Das Reaktionsgemisch wurde abgekühlt und auf 500 g Eis/100 ml konz. Salzsäure gegossen. Die organische Phase wurde abgetrennt und die wäßrige Phase wurde mit 3 x 100 ml Ether extrahiert. Die vereinigten organischen Phasen wurden mit 100 ml gesättigter Kochsalzlösung gewaschen, getrocknet und die Lösungsmittel abgezogen. Der Rückstand wurde im Pumpenvakuum durch eine 30 cm-Vigreux-Kolonne destilliert. Nach einem Vorlauf (30-65°C/0,2 Torr) destillierte das reine Produkt (Sdp. 107-109°C/0,5 Torr) als farbloses Öl, das in der Vorlage und z.T. auch schon in der Destillationsbrücke kristallisierte (Schmp. 44-46°C). Um ein Verstopfen der Brücke zu vermeiden, wurde diese auf ca. 50°C temperiert. Ausbeute 37,8 g Titelverbindung (164 mMol); 68,4 % d. Th. GC-Analyse (30 m fused silica-Säule DB-5 "Polydiphenyldimethylsiloxan", Schichtdicke 0,25 µm, Innendurchmesser 0,32 mm, 180°C, Inj. 240°C, 1 bar H₂): tᵣₑₜ: 4,46 Min; Reinheit > 99,9 %.
- NMR (270 MHz):: δ =1,28 (d, 6H, CH₃); 3,32 (sept., 1H, CH); 5,08 (s, 1H, OH); 6,9-7,5 (m, 7H, arom.-H).
- MS (DCI, Isobutan):: m/e = 231 (M+H⁺), 230 (M⁺), 215 (M⁺-CH₃)

### Stufe 3

### 2-(p-Fluorphenyl)-4-thiocyanato-6-isopropyl-phenol

Die Suspension von 70,9 g (838 mMol, 5,0 Äquivalenten) Natriumthiocyanat in 200 ml Methanol wurde 20 Min. bei Raumtemperatur gerührt. 40,0 g (173,8 mMol, 1,0 Äquiv.) 2-(p-Fluorphenyl)-6-isopropyl-phenol wurden zugegeben und das Gemisch wurde 20 Minuten gerührt. 14,32 ml (277,8 mMol, 1,6 Äquiv.) Brom wurden in 50 ml Methanol gelöst (exotherm) und diese Lösung wurde bei 15-20°C während 20 Minuten zu obiger Reaktionslösung getropft. Das Reaktionsgemisch färbte sich gelb und das Phenol löste sich vollständig. Das Reaktionsgemisch wurde 30 Min. gerührt. DC (Toluol/Cyclohexan 1:1) zeigte vollständigen Umsatz des Ausgangsmaterials (R_{f}=0,54) an. Neben der Titelverbindung (R_{f}=0,32) entstand als Verunreinigung nur eine geringe Menge der entsprechenden para-Brom-Verbindung, die mit dem Ausgangsmaterial cochromatographiert (R_{f}=0,54), aber aufgrund anderer Anfärbung unterschieden werden kann. Das Reaktionsgemisch wurde auf 400 g Eis/400 ml 2 N Salzsäure gegossen und mit 4 x 200 ml Toluol extrahiert. Die Extrakte wurden mit wäßriger Natriumsulfit-Lösung gewaschen, filtriert, mit gesättigter Kochsalzlösung gewaschen, getrocknet und im Vakuum eingeengt.

Der zurückbleibende gelbe Feststoff wurde in 500 ml heißem Cyclohexan gelöst und es wurden 5 g Aktivkohle zugesetzt. Danach wurde 5 Minuten unter Rückfluß gekocht und die heiße SUspension im Vakuum filtriert. Die abgesaugte Aktivkohle wurde mit 20 ml heißem Cyclohexan nachgewaschen. Das fast farblose Filtrat kühlte langsam ab und wurde dann noch 12 Std. auf 10°C gekühlt.

Die farblosen Kristalle (Titelverbindungen) wurden abgesaugt und im Vakuum getrocknet. 47,6 g (165,7 m Mol) Ausbeute entsprech. 95,3 %; Schmp.: 94,5-96 °C.
- NMR (60 MHz):: δ = 1,26 (d, 6H, CH₃), 3,32 (sept., 1H, CH); 5,46 (s, 1H, OH); 7,0-7,6 (m, 6H, arom.-H).
- MS (DCI, Isobutan) :: m/e = 288 (M+H⁺), 272 (M⁺-CH₃), 261

### Stufe 4

### 2-(p-Fluorphenyl)-4-(p-fluorphenylthio)-6-isopropyl-phenol

Eine THF-Lösung (100 ml) von p-Fluorphenylmagnesiumbromid [aus 3,11 g (128 mMol) Magnesium und 22,0 g (13,8 ml, 126 mMol) p-Bromfluorbenzol] wurde wie in Stufe 2 hergestellt. Die Lösung von 6,04 g (21 mMol) 2-(p-Fluorphenyl)-4-thiocyanato-6-isopropyl-phenol (aus Stufe 3) in 50 ml THF wurde bei 50°C zugetropft und noch 2 Stunden bei 40-50°C gerührt. Das Gemisch wurde abgekühlt und auf 500 ml eiskalte 2N Salzsäure gegossen. Es wurde dreimal mit 200 ml Ether wurde dreimal mit 200 ml Ether extrahiert. Die vereinigten Extrakte wurden mit Kochsalzlösung gewaschen, getrocknet und das Lösungsmittel im Vakuum entfernt.

Das zurückbleibende Öl (Titelverbindung) (7,5 g, 21 mMol, Ausbeute ∼ 100 %) war gemäß DC (Cyclohexan/Ethylacetat 9:1) und ¹H-NMR rein.
- NMR (60 MHz):: δ =1,25 (d, 6H, CH₃); 3,31 (sept., 1H, CH); 5,22 (s, 1H, OH); 6,8-7,8 (m, 10H, arom.-H)
- MS (DCI, Isobutan):: m/e = 357 (M+H⁺); 356 (M⁺)

### Stufe 5

### (3R,5S)-6-Methylsulfonyloxy-3,5-O-isopropyliden-3,5-dihydroxyhexansäure-tert.-butylester

Zur Lösung von 175,7 g (676 mMol) (3R,5S)-6-Hydroxy-3,5-O-isopropyliden-3,5-dihydroxyhexansäure-tert.-butylester (siehe EPA 0 319 847) in 1,7 l absol. Methylenchlorid und 1,7 l absol. Pyridin tropfte man bei 0-5°C 116,2 g (1,01 Mol, 1,5 Äquiv.) Methansulfonsäurechlorid. Man rührte 90 Min. unter Eiskühlung, engte dann das Reaktionsgemisch bei 30°C im Vakuum ein und entfernte die Hauptmenge des restlichen Pyridins nach Aufnehmen in Toluol durch Abziehen im Vakuum. Der Rückstand wurde in Toluol aufgenommen und zweimal mit Wasser, einmal mit gesättigter Natriumhydrogencarbonat-Lösung, einmal mit gesättigter Natriumchloridlösung gewaschen, dann getrocknet, filtriert und im Vakuum eingeengt. Das zurückbleibende Öl kristallisierte bei Raumtemperpatur innerhalb weniger Minuten praktisch vollständig. Die Kristalle wurden abgesaugt, auf der Nutsche zerstoßen, mit kaltem Petrolether gewaschen und im Vakuum getrocknet.

Man erhielt 192,0 g (568 mMol) farblosen Feststoff, Schmp. 75-76°C. Einengen des Filtrats, Absaugen der Kristalle und Waschen mit wenig kaltem Petrolether lieferte weitere 34,8 g (103 mMol) leicht verunreinigtes Produkt, Schmp. 69-73°C. Gesamtausbeute an Titelverbindung: 226,8 g (671 mMol, 99,3 %).
- NMR (270 MHz, CD₂Cl₂):: δ = 1,18-1,33 (m, 1H, CH₂, axial), 1,36 (s, 3H, CH₃), 1,42 (s, 9H, tert.-Bu), 1,46 (s, 3H, CH₃), 1,56 (dt, 1H, CH₂, äquat.), 2,36 (AB-Teil von ABX-System, 2H, CH₂), 3,03 (s, 3H, CH₃-SO₂), 4,09-4,23 (m, 3H, OCH₂ und O-CH), 4,24-4,37 (m, 1H, OCH).
- MS (DCI-Isobutan):: m/e= 283 (M+H⁺ - >= )

### Stufe 6

### {2,2-Dimethyl-4(S)-[(2-p-fluorphenyl-4-p-fluorphenylthio-6isopropyl)-phenoxymethyl]-6(R)-tert.-butoxycarbonylmethyl}-1,3-dioxolan

Zur Lösung von 4,0 g (11,2 mMol) 2-(p-Fluorphenyl)-4-p-fluorphenylthio)-6-isopropyl-phenol (Stufe 4) in 25 ml trockenem Hexamethylphosphorsäuretriamid (HMPT) gab man 2,02 g (14,6 mMol, 1,3 Äquiv.) gepulvertes Kaliumcarbonat und ca. 10 mg Kronen-Ether 18-Crown-6 (Firma Aldrich). Man rührte die Suspension 20 Min. bei Raumtemperatur, gab dann 4,55 g (13,5 mMol, 1,2 Äquiv.) (3R,5S)-6-Methylsulfonyloxy-3,5-O-isopropyliden-3,5-dihydroxyhexansäure-tert.-butylester (Stufe 5) hinzu und rührte 2 Tage bei 75-80°C.
Das Reaktionsgemisch färbte sich dunkel und wurde dickflüssiger. Es wurde in 200 ml wäßrige Natriumdihydrogenphosphat-Lösung gegossen und mehrmals mit Ether extrahiert. Die vereinigten Extrakte wurden mit gesättigter Kochsalzlösung gewaschen, getrocknet und im Vakuum eingeengt und ergaben 8,64 g eines bräunlichen Öls.
Säulenchromatographie (Cyclohexan/Ethylacetat 10:1 plus 1 o/oo Triethylamin) gab 4,96 g (8,28 mMol, 74,0 % Ausbeute) eines blaßgelben, zähen Öls (Titelverbindung).
- NMR (270 MHz, C₆D₆):: δ = 0,98-1,07 (m, 2H, CH₂), 1,19 + 1,20 (2xd, 6H, CH(CH₃)₂), 1,38 (s, 9H, tert.-Bu), 1,39 + 1,41 (2xs, 6H, OC(CH₃)₂), 2,12 (dd, 1H, CH₂CO₂), 2,42 (dd, 1H, CH₂CO₂), 3,27 (dd, 1H, O-CH₂), 3,37 (dd, 1H, O-CH₂), 3,65 (sept., 1H, CH(CH₃)₂), 3,65-3,76 (m, 1H, O-CH), 4,10-4,21 (m, 1H, O-CH), 6,60+6,82 (AA'BB'-System, 4H, arom.-H), 7,12-7,18 (m, 2H, arom.-H), 7,22-7,29 (m, 3H, arom.-H), 7,45 (d, 1H, arom-H)
- MS (DCI, Isobutan):: m/e = 598 (M⁺), 543 (M + H⁺ - >= ), 485.

### Stufe 7

### 3(R),5(S)-Dihydroxy-6-[(2-p-fluorphenyl-4-p-fluorphenylthio-6-isopropyl)-phenoxy]-hexansäure-tert.-butylester

Die Lösung von 4,47 g (7,47 mmol) des Acetonids aus Stufe 6 in 50 ml Tetrahydrofuran, 50 ml Ethanol und 5 ml 2 N Salzsäure wurde 16 Std. bei Raumtemperatur gerührt. DC (Cyclohexan/Ethylacetat 1:1) zeigte fast quantitative Umwandlung des Ausgangsmaterials (R_{f} = 0,78) ins Produkt (R_{f} = 0,59) an. Das Reaktionsgemisch wurde in wäßrige Natriumhydrogencarbonat-Lösung geschüttet und mehrmals mit Ether extrahiert. Die Extrakte wurden mit gesättigter Kochsalzlösung gewaschen, getrocknet und im Vakuum eingeengt. Der Rückstand (4,46 g bräunliches Öl) wurde durch Säulenchromatographie (Cyclohexan/Ethylacetat 2:1) gereinigt und ergab 3,37 g (6,03 mMol) Titelverbindung als farbloses Öl (Ausbeute 80,8 %).
- NMR (270 MHz, C₆D₆):: δ = 1,1 - ca. 1,4 (m, partiell verdeckt durch starke Singuletts, 2H, CH₂), 1,18 (d, 6H, CH(CH₃)₂, 1,31 (s, 9H, tert.-Bu), 2,00 (dd, 1H, CH₂-CO₂), 2,13 (dd, 1H, CH₂-CO₂), 3,15 (s, breit, 1H, OH), 3,36 (AB-Teil von ABX-Systemen, 2H, OCH₂), 3,52 (s, breit, 1H, OH), 3,56 (sept., 1H, CH(CH₃)₂, 3,76-3,96 (m, 2H, 2x CHOH), 6,61+6,79 (AA'BB'-System, 4H, arom.-H), 7,14-7,27 (m, 5H, arom.-H), 7,45 (d, 1H, arom.-H).
- MS (FAB, 3-NBA):: m/e = 558 (M⁺), 519, 503 (M⁺ - >= + H⁺), 356 (M⁺ des Phenolbausteins).

### Stufe 8

### 4(R)-Hydroxy-6(S)-[(2-p-fluorphenyl-4-p-fluorphenylthio-6-isopropyl)-phenoxymethyl]-3,4,5,6-tetrahydro-2H-pyran-2-on

Zur Lösung von 5,59 g (10,0 mMol) des tert.-Butylesters (Stufe 7) in 20 ml Methylenchlorid wurden 5 ml Trifluoressigsäure zugetropft. Das Reaktionsgemisch wurde 2 Std. bei Raumtemperatur gerührt. DC (Cyclohexan/Ethylacetat 1:1) zeigte quantitative Umwandlung des tert.-Butylesters (R_{f}=0,37) zum Lakton (R_{f}=0,12) und geringfügigen unpolareren Verunreinigungen an. Das Reaktionsgemisch wurde mit Natriumhydrogencarbonat-Pulver abgestumpft, dann mit Natriumcarbonat-Pulver neutral gestellt, anschließend in Wasser geschüttet und mehrmals ausgeethert. Die vereinigten organischen Phasen wurden mit Natriumhydrogencarbonat-Lösung, dann mit Kochsalzlösung gewaschen, getrocknet, filtriert und eingeengt. Der Rückstand wurde mit Cyclohexan/Ethylacetat 1:1 durch eine Kieselgelsäule chromatographiert und ergab 3,88 g (8,0 mMol, Ausbeute 80 %) eines farblosen Feststoffs (Titelverbindung), Schmp. 170-172°C.
- NMR (270 MHz):: δ =1,30+1,32 (2xd, 6H, CH(CH₃)₂), 1,72-1,94 (m, 3H, CH₂ und OH), 2,67 (AB-Teil von ABX-System, 2H, CH₂CO₂), 3,47 (sept., 1H, CH(CH₃)₂), 3,59 (AB-Teil von ABX-System, 2H, OCH₂), 4,40 (m, 1H, CH-OH), 4,72 (m, 1H, CH-OCO), 6,80-7,55 (m, 1OH, arom.-H).
- MS (DCI, Isobutan):: m/e = 484 (M⁺), 467 (M⁺-OH),

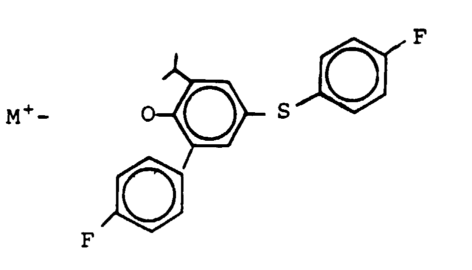

### Pharmakologische Daten Organselektive Wirkung von Pharmakon-Gallensäurekonjugaten

Chlorambucil, [4-(Bis-N-(2'-chlorethyl)-aminophenyl]buttersäure, ist ein Zytostatikum zur Behandlung maligner Tumoren. Chlorambucil wird hauptsächlich über die Nieren ausgeschieden. Durch kovalente Kopplung von Chlorambucil an Gallensäuren wird eine leberspezifische Wirkung des Chlorambucils erreicht, wie durch folgende Experimente belegt wird.

### 1. Wechselwirkung von Chlorambucil-Gallensäurekonjugaten mit Bindeproteinen für Gallensäuren in den Leberzellen

Gallensäuren werden aus dem Pfortaderblut von den Leberparenchymzellen aufgenommen und wiederum in die Galle ausgeschieden. Der Transport der Gallensäuren durch Membranen auf der Blut- und Gallenseite der Leberzelle, der Transport von Gallensäuren in die verschiedenen Zellorganellen wie Mitochondrien, endoplasmatisches Retikulum etc. und der Transport im Zytoplasma der Leberzelle erfolgt durch verschiedene spezifische Bindeproteine für Gallensäuren. Gallensäuren binden auch an regulatorische Proteine und die Enzyme des Gallensäuremetabolismus.

Diese physiologisch relevanten Bindeproteine für Gallensäuren wurden durch die Methode der Photoaffinitätsmarkierung molekular identifiziert und charakterisiert. Das Prinzip dieser Methode besteht darin, daß ein photoreaktives radioaktiv markiertes Gallensäurederivat analog zu den natürlichen Gallensäuren an die physiologischen Bindeproteine bindet. Durch Bestrahlung mit UV-Licht werden in diesem Derivat hochreaktive chemische Intermediate wie Carbene, Nitrene oder Radikale erzeugt, die aufgrund ihrer hohen Reaktivität durch Addition an Doppelbindungen oder Insertion in Einfachbindungen sofort mit den Bindeproteinen reagieren und kovalent an diese binden. Durch diese kovalente Verknüpfung des radioaktiven Gallensäurederivats mit dem Bindeprotein werden dann auch die Bindeproteine radioaktiv markiert und können nach einer Auftrennung der'verschiedenen Proteine einer Leberzelle z.B. durch Elektrophorese identifiziert und ihr Molekulargewicht bestimmt werden.

Wird eine solche Photoaffinitätsmarkierung in Gegenwart eines Stoffes X' durchgeführt, der ebenfalls an die gallensäurebindenden Proteine bindet, so wird X' mit dem radioaktiven Gallensäurederivat um die Bindung an die gallensäurebindenden Proteine konkurrieren; die radioaktive Markierung der entsprechenden Bindeproteine wird damit in Gegenwart von X' geringer werden. Ist X' hingegen ein Stoff, der nicht an die gallensäurebindenden Proteine bindet, so wird die Markierung der gallensäurebindenden Proteine durch die Gegenwart von X' nicht verringert werden.

3,6 x 10⁶ frisch isolierte Hepatozyten aus Rattenleber (ca. 3 mg Protein) in 1,5 ml Puffer I (118 mM NaCl, 4,74 mM KCl, 0.59 mM KH₂PO₄, 0.59 mM Na₂HPO₄ x 2 H₂O, 1,185 mM MgCl₂ x 6 H₂O, 24,87 mM NaHCO₃, 1,25 mM CaCl₂, 5,5 mM D-Glucose, pH 7,35. Puffer 1 h mit Carbogen (95 % 0₂/5 % CO₂) begast) wurden bei 37°C min im Dunkeln mit 1,25 µM (25 µCi) (7,7-Azo-3α-12α-dihydroxy-5β[3β-³H]cholan-24-oyl)-2-aminoethansulfonsäure in Abwesenheit oder in Anwesenheit von 250 µM "Beispiel 3" inkubiert und anschließend bei 350 nm in einem Rayonet RPR-100 Photoreaktor mit 16 RPR 3500 Å-Lampen 5 min bestrahlt. Anschließend wurde die Zellsuspension mit 10 ml eiskaltem Puffer I verdünnt und die Zellen durch 3-minütige Zentrifugation bei 1000 x g sedimentiert. Die Zellen wurden in 10 ml Puffer I erneut resuspendiert und wiederum durch Zentrifugation sedimentiert. Der Niederschlag wurde in 600 µl Tris/HCl-Puffer (pH 6,8)/2% Natriumdodecylsulfat (SDS)/ 5 % 2-Mercaptoethanol/10 % Glyzerin/0,001 % Bromphenolblau aufgenommen, 5 min auf 90°C erhitzt und 20 min bei 40 000 x g zentrifugiert. Der klare Überstand mit den solubilisierten Proteinen wurde weiterverwendet. Jeweils 100 µl (500 µg Protein) wurden auf diskontinuierliche SDS-Plattengele (200 x 170 x 2,7 mm, Gesamtacrylamidkonzentration 12 %) aufgetragen und die Proteine durch Elektrophorese bei 50 V Spannung aufgetrennt. Nach der Elektrophorese wurden die Gele in 12,5 %iger Trichloressigsäurelösung fixiert und mit 0,08 % Serva Blau R 250-Lösung in 25 % Ethanol/8 % Essigsäure/67 % Wasser gefärbt. Nach dem Entfärben wurden die Gele 20 min in 1 M Natriumsalizylat-Lösung in 70 % Methanol equilibriert und anschließend getrocknet. Die Verteilung der radioaktiv markierten Proteine wurde mit der Methode der Fluorographie durch Auflegen der getrockneten Gele auf Kodak-X-Omat AR Röntgenfilme und Exposition bei -80°C für 14 d bestimmt. Die geschwärzten Filme wurden durch Densitometrie vermessen. Die Abnahme der Markierung der verschiedenen gallensäurebindenden Proteine durch die Gegenwart von "Beispiel 3" während der Photoaffinitätsmarkierung wird in % bezogen auf die Kontrolle angegeben.

| **Molekulargewicht der gallensäurebindenden Leberzellproteine** | **% Hemmung gallensäurebindenden Proteine durch 250 µM "Beispiel 3"** |
|---|---|
| 67 000 (Albumin) | 90,5 |
| 54 000 (Transportprotein der Membran) | 89,6 |
| 48 000 (Transportprotein der Membran) | 88,8 |
| 43 000 (Zytoskelett-Protein) | 88,1 |
| 36 000 (Transportprotein im Zytoplasma) | 98,1 |
| 33 000 (Bindeprotein in Mitochondrien) | 93,2 |

### 2. Alkylierung von Proteinen der Leberzellen durch "Beispiel 5"

Frisch isolierte Hepatozyten (2 x 10⁶) in 2 ml Puffer 1 wurden bei 37°C mit 30 µCi "Beispiel 5" inkubiert. Nach 10, 30, 40 und 60 min wurden jeweils 500 µl Zellsuspension (500 µg Protein) abgenommen und mit 10 ml eiskaltem Puffer 1 verdünnt. Nach 3-minütiger Zentrifugation bei 1000 x g wurde der Niederschlag in 10 ml Puffer 1 resuspendiert und die Suspension erneut zentrifugiert. Die Zellniederschläge wurden in je 100 µl 62,5 mM Tris/HCl-Puffer (pH 6,8)/2 % SDS/5 % 2-Mercaptoethanol/10 % Glyzerin/0,001 % Bromphenolblau aufgelöst, 30 min bei 48 000 x g zentrifugiert und die klaren Überstände auf diskontinuierliche SDS-Plattengel (200 x 170 x 2,7 mm) aufgetragen. Nach der elektrophoretischen Auftrennung wurden die Gele fixiert und gefärbt. Zur Bestimmung der Verteilung der Radioaktivität wurden die einzelnen Gelbahnen in 2 mm-Abschnitte aufgeteilt, die Proteine durch Inkubation mit 0,5 ml mit Biolute S verdaut, und nach Zugabe von 5 ml Szintillator Quickszint 501 wurden die Proben in einem Flüssigkeitszintillationsmeßgerät vermessen.

| **Inkubationszeit (min)** | **Molekulargewichte markierter Leberproteine (in kDa)** | **Radioaktivität in markierten Leberproteinen (in cpm)** |
|---|---|---|
| 0 | / | < 30 |
| 10 | 48, 67 | 48: 408; 67: 102 |
| 30 | 122, 54, 48, 42, 33, 28 | 122: 1064; 54: 1811 48: 1375; 42: 1777 33: 1116; 28: 2190 |
| 40 | 122, 60, 54, 48, 42, 33, 28 | 122: 1549; 54: 2385 48: 2420; 42: 2650 33: 1474; 28: 2682 |
| 60 | 122, 54, 48, 42 35, 33, 28 | 122: 997; 54: 1949 48: 2074; 42: 1725 28: 2163 |

"Beispiel 5" führt zu einer radioaktiven Markierung von Proteinen der Leberzelle, sowohl in Membranen als auch im Zytoplasma und in Zellorganellen. Neben den bekannten physiologisch relevanten gallensäurebindenden Proteinen (54, 48, 33 kDa) werden auch andere Proteine der Leberzelle (z.B. 122, 35, 28 kDa) markiert.

Diese Experimente zeigen, daß die alkylierende für die pharmakologische Wirkung relevante Eigenschaft des Chlorambucils auch nach Kopplung des Wirkstoffs an Gallensäuren erhalten ist. Die gefundene radioaktive Markierung rührt von einer Markierung von Proteinen durch das intakte Chlorambucil-Gallensäurekonjugat her, da die radioaktive Markierung im Gallensäureteil lokalisiert ist.

Erfindunggemäße Verbindungen W-X-G werden also von den Leberzellen aufgenommen und können Proteine alkylieren. Die Eigenschaften sowohl des Chlorambucilmoleküls (alkylierende Wirkung) als auch der Gallensäure (Benutzung spezifischer Transportwege für Gallensäure) sind in W-X-G vereint.

### 3. Metabolisierung von "Beispiel 5" durch frisch isolierte Hepatozyten

Frisch isolierte Hepatozyten (1,8 x 10⁶) in 750 µl Puffer I wurden mit 5,5 µCi "Beispiel 5" bei 37°C inkubiert. Nach 10, 20, 30, 40 und 60 min wurden jeweils 100 µl der Zellsuspension abgenommen, mit 10 ml eiskaltem Puffer I verdünnt und 5 min bei 1000 x g zentrifugiert. Der Zellniederschlag wurde 2 x mit je 100 µl Chloroform/Methanol-Lösung (1/1, v/v) versetzt zur Extraktion der Gallensäure. Die organischen Extrakte wurden in einem N₂-Strom eingedampft, die Rückstände in je 20 µl Dioxan aufgenommen und auf HPTLC-Dünnschichtplatten aufgetragen. Nach dem Entwickeln des Chromatogramms mit n-Butanol/Essigsäure/Wasser (9/1/2, v/v/v) als Laufmittel wurde die Platte getrocknet, mit 1 M Natriumsalicylat-Lösung in Methanol besprüht und nach dem Trocknen auf einen Kodak-X-Omat AR Röntgenfilm gelegt. Nach 1 Woche Exposition bei -80°C wurde der Film entwickelt und die Verteilung der Radioaktivität durch Densitometrie bestimmt. Die Verteilung der Radioaktivität auf die einzelnen Metabolite ist in % angegeben.

| Inkubationszeit (min) | "Beispiel 5" R_{f}=0,49 | Metabolit 1 R_{f}=0,32 | Metabolit 2 R_{f}=0,36 | Metabolit 3 R_{f}=0,21 |
|---|---|---|---|---|
| 10 | 31,34 | 33,79 | 38,03 | 5,62 |
| 20 | 26,51 | 29,49 | 32,25 | 11,74 |
| 30 | 23,76 | 29,88 | 34,18 | 12,16 |
| 40 | 13,78 | 33,52 | 40,93 | 11,75 |
| 60 | 11,00 | 33,7 | 45,7 | 10,3ù |
| Metabolit 1: (3α,12β-dihydroxy-5β[12α-³H]cholan-24-oyl)-2-aminoethansulfonsäure Metabolit 2: (3α,12α-dihydroxy-5β[12β-³H]cholan-24-oyl)-2-aminoethansulfonsäure Metabolit 3: nicht identifiziert | | | | |

"Beispiel 5" wird von den Leberzellen in das Zellinnere aufgenommen und gelangt zu den intrazellulären Orten des Metabolismus. Das Chlorambucil-Gallensäurekonjugat wird schnell hydrolytisch gespalten in den Wirkstoff freies Chlorambucil und Gallensäure, Chlorambucil wird also in der Form von Gallensäurekonjugaten in Zellen mit Gallensäuretransportsystemen gebracht, um dort pharmakologisch wirken zu können.

An Gallensäuren gekoppelte Zytostatika sind damit zur Behandlung von Malignomen und deren Metastasen von solchen Zellen, welche die Fähigkeit zur Aufnahme von Gallensäuren haben, insbesondere geeignet.

### 4. Interaktion von Gallensäurederivaten mit dem intestinalen Gallensäuretransportsystem im terminalen Dünndarm

### 4.1. Präparation von Bürstensaummembranvesikeln aus dem Ileum von Kaninchen

Die Präparation von Bürstensaummembranvesikeln aus den Darmzellen des Dünndarms erfolgte mit der sog. Mg²⁺-Präzipitationsmethode. Männliche Neuseeland-Kaninchen (2-2,5 kg Körpergewicht) wurden durch intravenöse Injektion von 0,5 ml T-61^{R} getötet. Der Dünndarm wurde entnommen und mit eiskalter physiologischer Kochsalzlösung gespült. Die terminalen 3/10 des Dünndarms (gemessen in oral rectaler Richtung, d.h. das terminale Ileum, welches das aktive Na⁺-abhängige Gallen-säuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/ 300 mM Mannit, 5 mM EGTA/10 mg/l Phenylmethylsulfonylfluorid/1 mg/l Trypsin-Inhibitor v. Sojabohnen (32 U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, BRD) 3 Minuten bei 75 % max. Leistung unter Eiskühlung homogenisiert. Nach Zugabe von 3 ml 1 M MgCl₂-Lösung (Endkonzentration 10 mM) ließ man exakt 1 Minute bei O°C stehen. Durch Zugabe von Mg²⁺ aggregieren die Zellmembranen und präzipitieren mit Ausnahme der Bürstensaummembranen. Nach einer 15-minütigen Zentrifugation bei 3 000 x g (5000 rpm, SS-34-Rotor) wird der Niederschlag verworfen und der Überstand, der die Bürstensaummembranen enthält, 30 Minuten bei 26 700 x g (15 000 rpm, SS-34-Rotor) zentrifugiert. Der Überstand wurde verworfen, der Niederschlag in 60 ml 12 mM Tris/HCl-Puffer (ph 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M MgCl2-Lösung und 15-minütiger Inkubationszeit bei O°C wurde erneut 15 Minuten bei 3 000 x g zentrifugiert. Der Überstand wurde anschließend nochmals 30 Minuten bei 46 000 x g (15 000 rpm, SS-34-Rotor) zentrifugiert. Der Niederschlag wurde in 30 ml 10 mM Tris/Hepes-Puffer (pH 7,4) / 300 mM Mannit aufgenommen und durch 20 Hübe in einem Potter Elvejhem Homogenisator bei 1 000 rpm homogen resuspendiert. Nach 30-minütiger Zentrifugation bei 48 000 x g (20 000 rpm, SS-34-Rotor) wurde der Niederschlag in 0,5 bis 2 ml Tris/Hepes-Puffer (pH 7,4) / 280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196°C in 4 mg Portionen in flüssigem Stickstoff aufbewahrt.

### 4.2. Hemmung der Na⁺-abhängigen [³H]Taurocholat-Aufnahme in Bürstensaummembranvesikel des Ileums

Die Aufnahme von Substraten in Bürstensaummembranvesikel wurde mittels der sog. Membranfiltrationstechnik bestimmt. 10 µl der Vesikelsuspension (100 µg Protein) wurden als Tropfen an die Wand eines Polystyrolinkubationsröhrchens (11x70 mm) pipettiert, welches das Inkubationsmedium mit den entsprechenden Liganden enthielt (90 µl). Das Inkubationsmedium enthielt 0,75/µl = 0,75 µCi [³H(G)]-Taurocholat (spezifische Aktivität: 2,1 Ci/mMol), /0,5 µl 10 mM Taurocholat/8,75 µl Natrium-Transport-Puffer (10mM Tris/Hepes, (pH 7,4)/100 mM Mannit/100 mM NaCl) (Na-T-P) bzw. 8,75 µl Kalium-Transport-Puffer (10 mM Tris/Hepes (pH 7,4)/100 mM Mannit/100 mM KCl) (K-T-P) und 80 ul der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 um, 4 mm⌀, Millipore, Eschborn, B.R.D.) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 min) wurde der Transport durch Zugabe von 1 ml eiskalter Stoplösung (10 mM Tris/Hepes, (pH 7,4)/150 mM KCl) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, BRD) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, BRD) aufgelöst und die Radioaktivität durch Flüssigkeits-Szintillationsmessung in einem Meßgerät TriCarb 2500 (Canberra Packard GmbH, Frankfurt, BRD) gemessen. Die gemessenen Werte wurden nach Eichung des Gerätes mit Hilfe von Standardproben und nach Korrektur evtl. vorhandener Chemilumineszenz als dpm (Decompositions per minute) erhalten.

Die Kontrollwerte wurden jeweils in Na-T-P und K-T-P ermittelt. Die Differenz zwischen der Aufnahme in Na-T-P und K-T-P ergab den Na⁺-abhängigen Transportanteil. Als IC₅₀ Na⁺ wurde diejenige Konzentration an Inhibitor bezeichnet, bei der der Na⁺-abhängige Transportanteil um 50 - bezogen auf die Kontrolle - gehemmt war; entsprechendes gilt für die Angaben der IC₂₅ und IC₇₅-Werte.

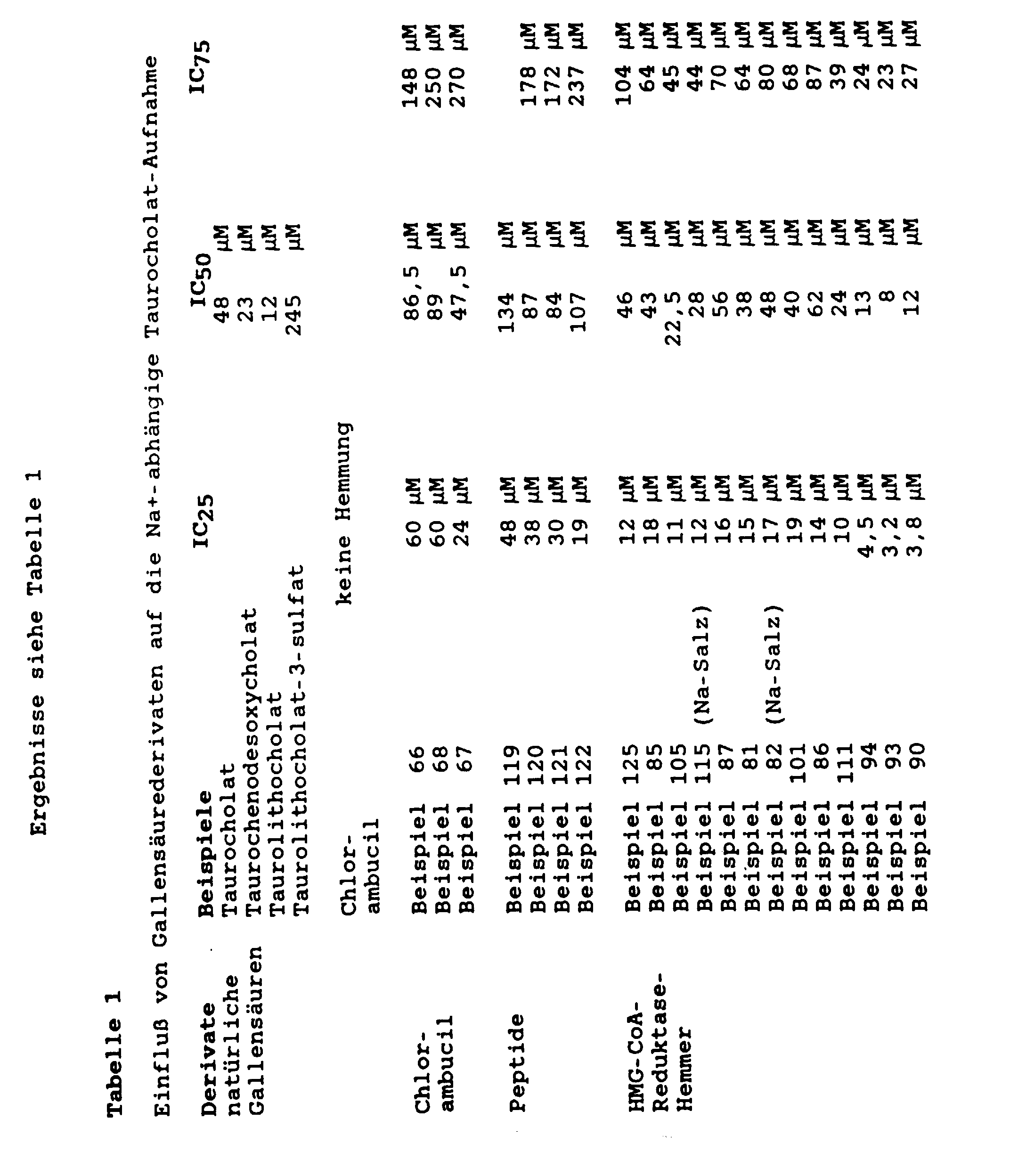

### 5. Aufnahme von Gallensäurederivaten durch die Leber aus Pfortaderblut und Ausscheidung in die Galle

Zur Untersuchung einer selektiven Aufnahme von Gallensäurederivaten in die Leber und ihrer hepatotropen Wirkung wurden narkotisierten Ratten die entsprechende Derivate als Bolus in eine Mesenterialvene injiziert und die Ausscheidung dieser Derivate sowie der entsprechenden Wirkstoffe bzw. Wirkstoff-metabolite in die Galle analysiert.

### 5.1. In vivo perfundierte Leber

Männliche Spraque-Dawley-Ratten (300 - 500 g Körpergewicht) wurden vom Futter abgesetzt und erhielten Wasser ad libitum. In Urethannarkose (5 ml/kg i.m. Urethan 30 %) wurde nach Tracheotomie das Abdomen durch Medianschnitt eröffnet. Es wurde der Pylorus ligiert und der Gallengang durch Vorschieben eines Polyäthylenschlauches (d = 0,05 mm) bis vor die Leber kanüliert und die Galle drainiert. Das Sekretvolumen wurde in 15-minütigen Abständen gemessen. Nach einem 60-minütigen Vorlauf wurden die Substanzen (0,5 ml einer 1 mM Lösung in 0,9 % NaCl Lösung) während 30 sec in eine Mesenterialvene appliziert und die Galle in vorgegebenen Zeitintervallen (2,4,6,8,10,15, 20,30,40,50 60,70,80,90,100,110 120 min) gesammelt. Zunächst wurde das Gallensäurederivat appliziert, nach einer 2-stündigen Sammelperiode wurde der entsprechende Wirkstoff appliziert. 120 min nach Testsubstanzgabe wurde das Blasenvolumen gemessen und der Harn ebenfalls gesammelt. Die Galle- und Harnproben wurden während des Versuchs auf Eis gelagert und anschließend tiefgefroren.

### 5.2. Analyse von Gallensäurederivaten in der Galle

Je 10 µl Gallenprobe wurden mit Microcaps (Firma Drummond, Broomall, USA) auf DC Platten (10 x 20 cm, Kieselgel 60 spezial Schichtdicke 0,25 mm, Nr. 37581 Firma Riedel-de Haen, Seelze, BRD) aufgetragen und die Dünnschichtplatten an der Luft 20' getrocknet.

Die Platten wurden in folgenden Laufmitteln entwickelt:

| Substanz | Laufmittel |
|---|---|
| Chlorambucil | Chloroform/Methanol (10/1, v/v) |
| Beispiel 67 | Butanol/Eisessig/Wasser (9/2/1, v/v) |
| Beispiel 125 K⁺-Salz | Butanol/Eisessig/Wasser (9/2/1, v/v) |
| Beispiel 85 | Butanol/Eisessig/Wasser (9/2/1, v/v) |
| Beispiel 87 | Chloroform/Methanol (3/1, v/v) |
| Beispiel 86 | Chloroform/Methanol (3/1, v/v) |

Die Bestandteile der Laufmittel wurden von den üblichen Herstellern im höchstmöglichen Reinheitsgrad bezogen. Nach dem Entwickeln und Trocknen der Platten wurde die Verteilung der Wirkstoffe und der Gallensäurederivate, bzw. ihrer Metabolite durch Densitometrie bestimmt (Densitometer CD 50, DESAGA; Heidelberg). Die Detektion erfolgte im Reflexionsmodus bei linearer Abtastung bei den Wellenlängen 252 nm (Chlorambucil/Chlorambucil-Konjugat) bzw. 260 nm (restl. Substanzen)

Für die Auswertung der Chromatogramme wurde die relative Intensität der Gallensäurederivate, der Wirkstoffe bzw. der Wirkstoffmetabolite auf einen Chromatogramm in Flächeneinheiten angegeben.

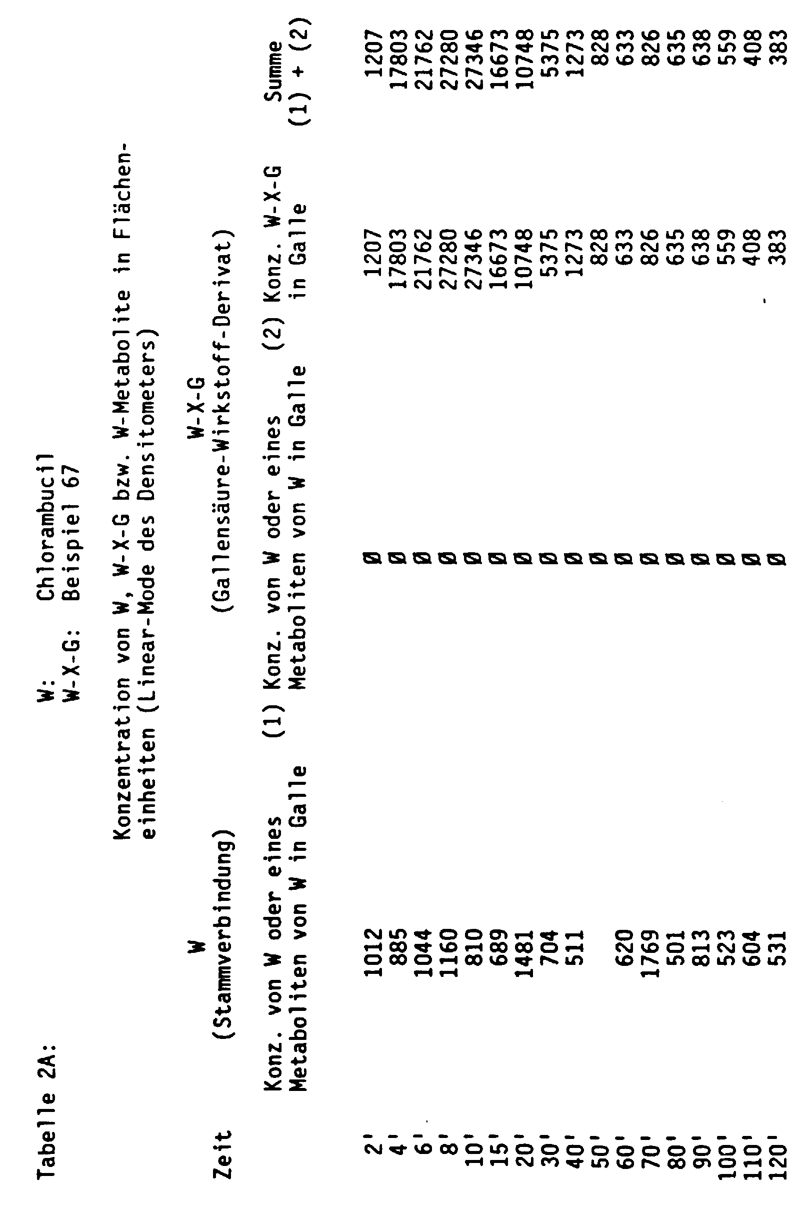

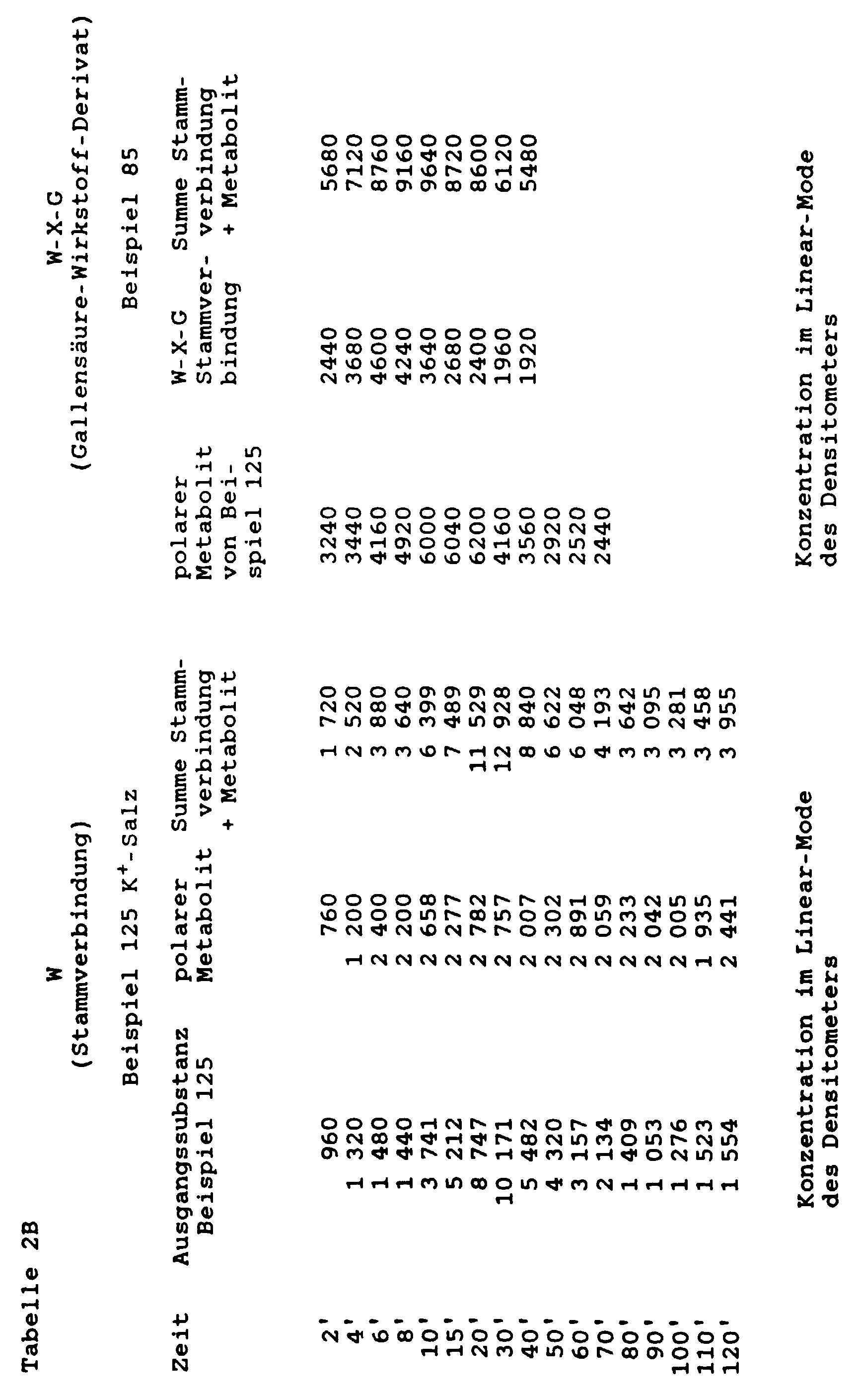

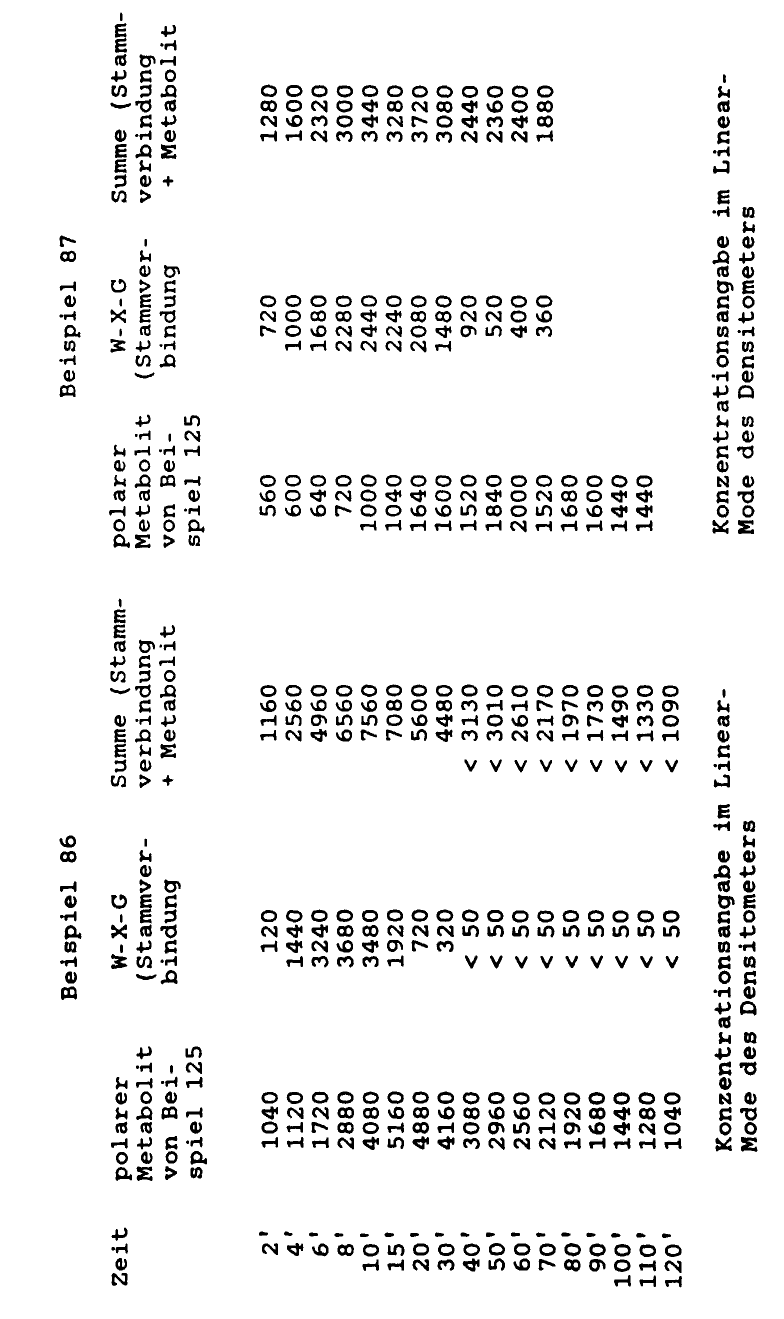

## Patentansprüche

1. Gallensäurederivate der Formel I
W-X-G I
bestehend aus
einem modifizierten Gallensäurerest G: 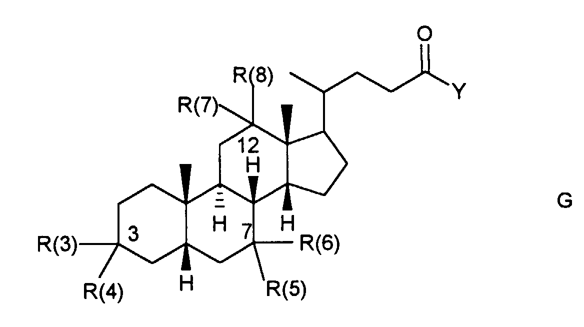 in welcher R(3) - R(8) gleich oder verschieden sind und folgende Bedeutung haben:
eine Bindung zu W-X-, wobei insgesamt bis zu zwei W-X-Einheiten gebunden sein können,
R(3) und R(4), R(5) und R(6), R(7) und R(8) jeweils gemeinsam der Sauerstoff einer Carbonylgruppe, 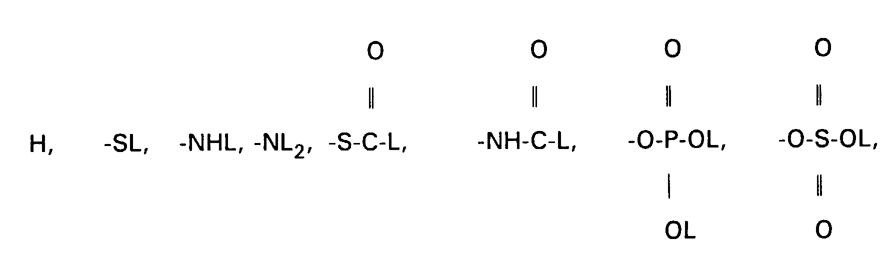 -L, wobei L bedeutet:
H, einen gesättigten oder ungesättigten Alkylrest mit 1 - 10 C-Atomen, der verzweigt oder unverzweigt ist, Cycloalkyl mit 3 - 8 C-Atomen, einen Phenylrest (der unsubstituiert oder 1 - 3-fach substituiert ist mit F, Cl, Br, (C₁-C₄)-Alkyl oder (C₁-C₄)-Alkoxy, einen Benzylrest, der unsubstituiert oder 1 - 3-fach substituiert ist mit F, Cl, Br, (C₁-C₄)-Alkyl oder (C₁-C₄)-Alkoxy;
und in der Y folgende Bedeutung hat
-OH oder
eine über die Aminogruppe gebundene Aminosäure oder Aminosulfonsäure und deren Alkali- und Erdalkalisalze, -OKa, wobei Ka ein Kation bedeutet wie z. B. ein Alkali oder Erdalkaliion oder auch ein quartäres Ammoniumion;
einem Verbindungsglied X, das eine direkte Bindung oder ein Zwischenglied X ist: 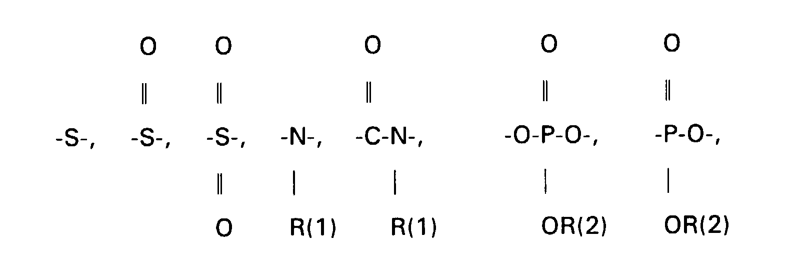 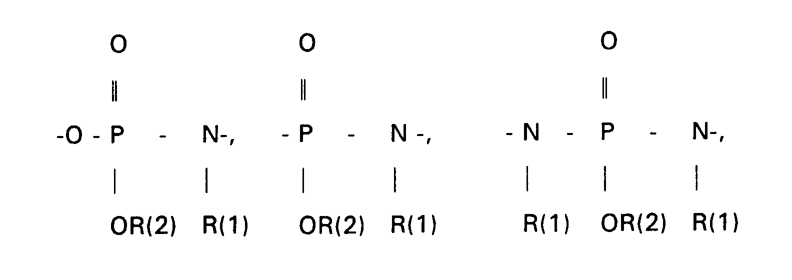 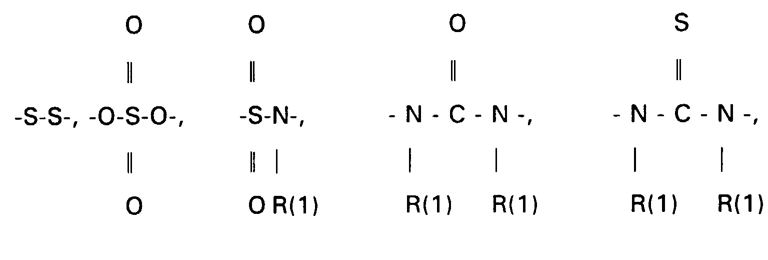 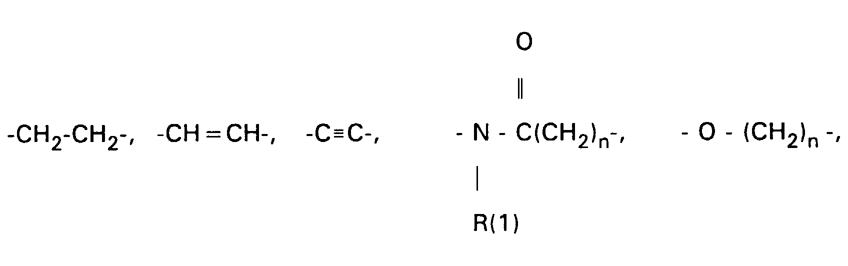 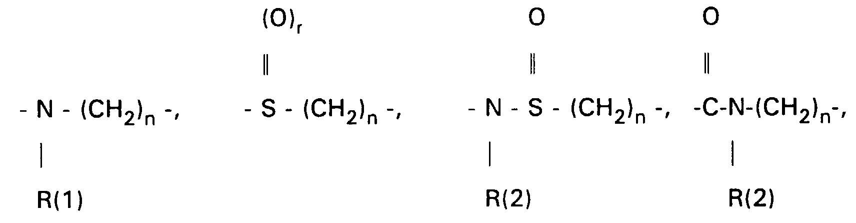 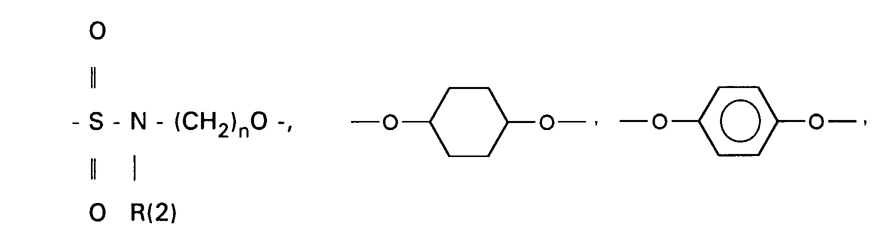 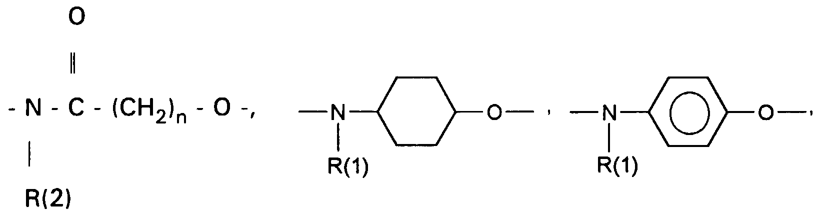 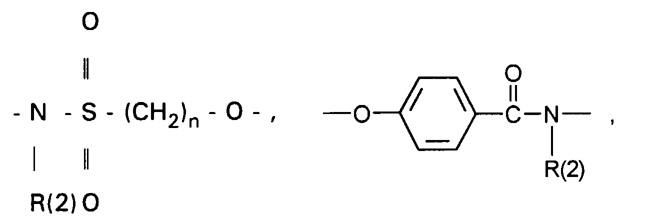 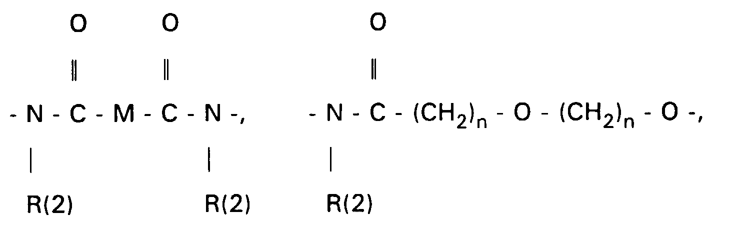 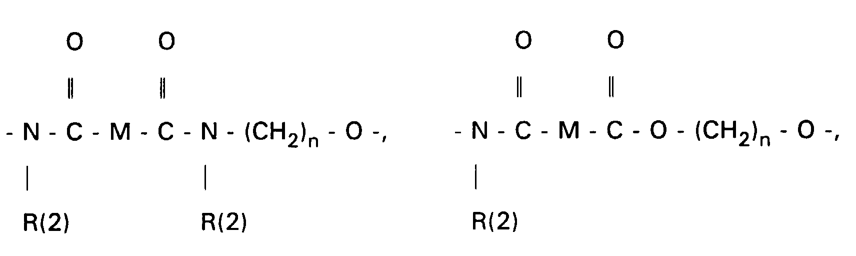 worin
R(1) = H, (C₁-C₈)-Alkyl; die Gruppe 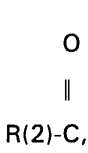 Phenyl, Benzyl, unsubstituiert oder 1 - 3-fach substituiert mit F, Cl, Br, (C₁-C₄)-Alkyl oder Alkoxy mit 1 - 4 C-Atomen
R(2) = H, (C₁-C₈)-Alkyl, Phenyl, Benzyl, unsubstituiert oder 1 - 3-fach substituiert mit F, Cl, Br, (C₁-C₄)-Alkyl oder Alkoxy mit 1 - 4 C-Atomen
m = 0-6
n = 1-16
p = 1, 2, 3
r = 0 - 2 und
M = -(CH₂)ₘ-, -(C=C)ₚ-, -C≡C-, 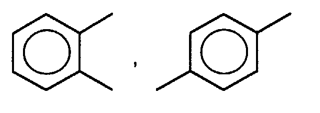
und einem Wirkstoffteil W, der ein Peptid, ein Antibiotikum z. B. ein β-Lactam-Antibiotikum, eine antivirale Substanz, ein Krebsmittel wie z. B. Chlorambucil, ein Leberschutzmittel, ein Antihyperlipidämikum z. B. ein HMG-CoA Reductase-Hemmer, ein Diuretikum, ein Blutdrucksenker, ein Renininhibitor oder ein Prolylhydroxylase-Hemmer ist,
wobei eine Verbindung der Formel 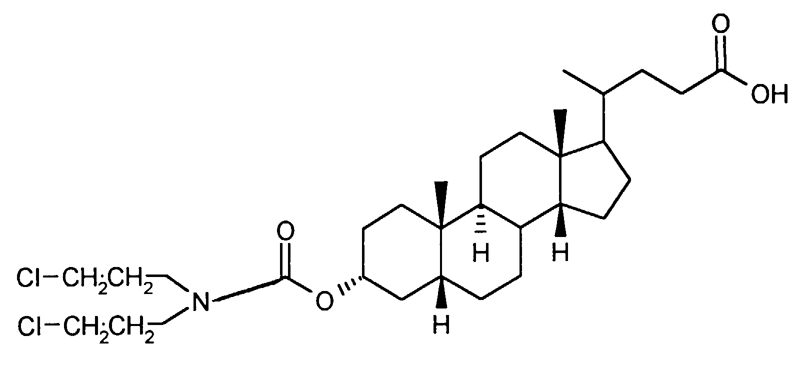 ausgenommen ist.

2. Verbindung I nach Anspruch 1, dadurch gekennzeichnet, daß die Reste folgende Bedeutung haben:
G 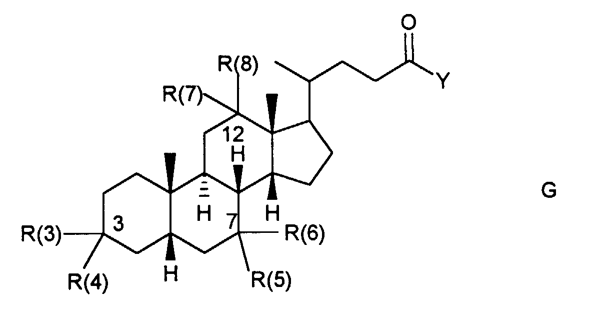 mit R(3) - R (8) gleich oder verschieden und folgende Bedeutung haben: eine Bindung zu W-X-, wobei bis zu zwei W-X-Einheiten gebunden sein können, 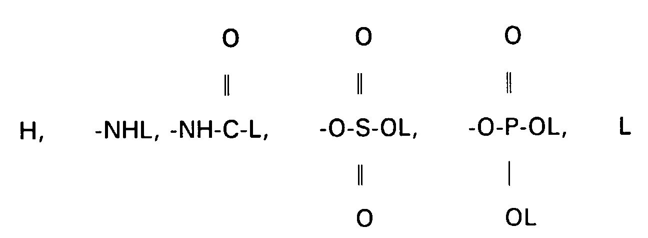 wobei L bedeutet:
H, einen gesättigten Alkylrest mit 1 - 6 C-Atomen, der verzweigt oder unverzweigt sein kann, einen Phenylrest, der unsubstituiert oder 1 - 3 fach substituiert ist mit F, Cl, Br, (C₁-C₄)-Alkyl oder (C₁-C₄)-Alkoxy
Y: -OH,
-OKa, wobei Ka ein Alkali oder Erdalkalikation oder ein Ammoniumion bedeutet 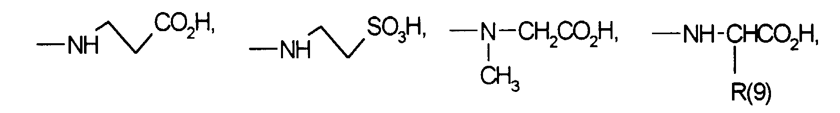 wobei R(9) bedeutet
Methyl, Isopropyl, Isobutyl, 2-Butyl, Benzyl, 4-Hydroxybenzyl, Hydroxymethyl, 1-Hydroxyethyl, H₃CSCH₂CH₂-, HO₂CCH₂-, HO₂CCH₂CH₂-
das Verbindungsglied X, wobei G und W an beliebigen Enden von X gebunden sein können, 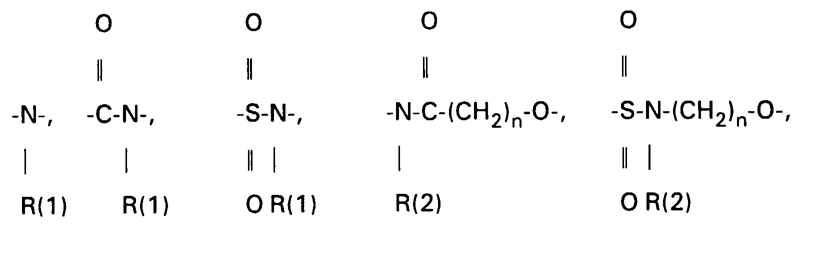 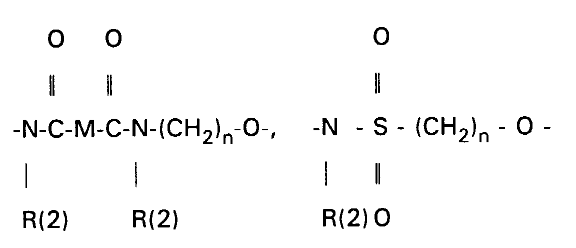 mit
R(1) = H, (C₁-C₈)-Alkyl, 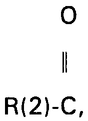 Phenyl, Benzyl, wobei die aromatischen Ringe unsubstituiert oder 1 - 3 fach substituiert sind mit F, Cl, Br, (C₁-C₄)-Alkyl und (C₁-C₄)-Alkoxy;
mit R(2) = H, (C₁-C₈)-Alkyl, Phenyl, Benzyl; jeweils unsubstituiert oder 1 - 3 fach substituiert mit F, Cl, Br, (C₁-C₄)-Alkyl oder Alkoxy mit 1 - 4 C-Atomen
n = 1 - 16 und
M = -CH₂-CH₂-,
-CH = CH-
W
ein Peptid, ein Antibiotikum z. B. ein β-Lactam-Antibiotikum, eine antivirale Substanz, ein Krebsmittel wie z. B. Chlorambucil, ein Leberschutzmittel, ein Antihyperlipidämikum z. B. einen HMG-CoA Reductase-Hemmer, ein Diuretikum, einen Blutdrucksenker, einen Renininhibitor, einen Prolylhydroxylase-Hemmer, wobei eine Verbindung der Formel 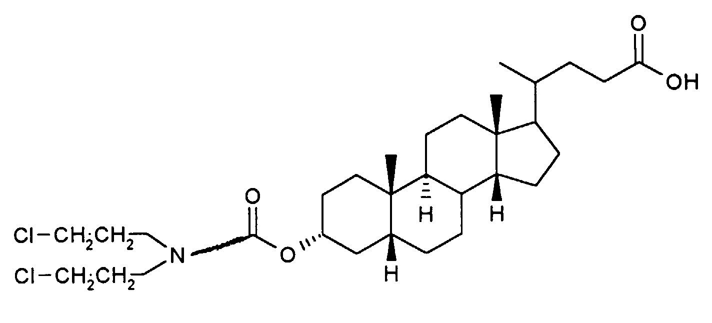 ausgenommen ist.

3. Verfahren zum Herstellen einer Verbindung I nach Anspruch 1, dadurch gekennzeichnet, daß man
a) im Falle von X = eine direkte Bindung geeignete reaktionsfähige Formen von G und W nach bekannten Verfahren miteinander zur Reaktion bringt oder
b) im Falle von X = ein Zwischenglied
α) reaktionsfähige Formen von G-X mit W bzw.,
β) reaktionsfähige Formen von W-X mit G nach bekannten Verfahren miteinander zur Reaktion bringt.

4. Verbindung I nach Anspruch 1 zur Verwendung als Arzneimittel.

5. Verwendung einer Verbindung I nach Anspruch 1 zur Herstellung eines Arzneimittels.

6. Verbindung XXII
SG-X-G XXII
in der
SG gleich H oder eine Schutzgruppe ist,
G 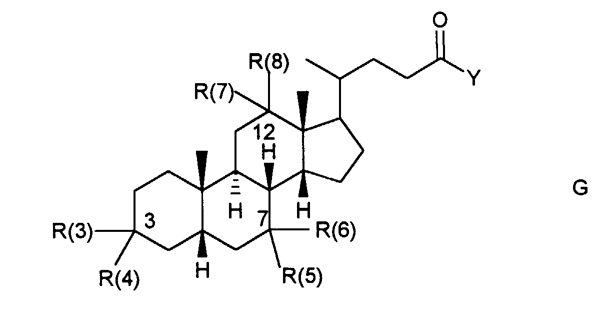 in welcher R(3) - R (8) gleich oder verschieden sind und folgende Bedeutung haben:
eine Bindung zu SG-X-, wobei insgesamt bis zu zwei SG-X-Einheiten gebunden sein können,
R(3) und R(4), R(5) und R(6), R(7) und R(8) jeweils gemeinsam der Sauerstoff einer Carbonylgruppe, 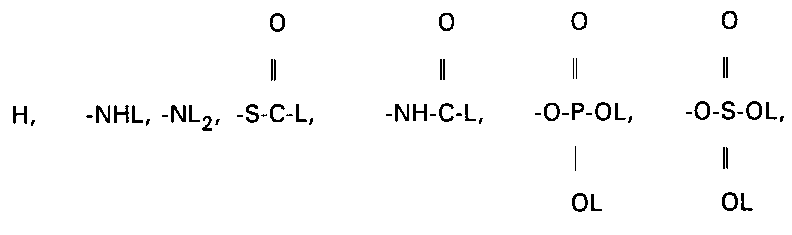 -L, wobei L bedeutet:
H, einen gesättigten oder ungesättigten Alkylrest mit 1 - 10-C-Atomen, der verzweigt und unverzweigt ist, Cycloalkyl mit 3 - 8 C-Atomen, einen Phenylrest (der unsubstituiert oder 1 - 3fach substituiert ist mit F, Cl, Br, (C₁-C₄)-Alkyl oder (C₁-C₄)-Alkoxyl), einen Benzylrest (der unsubstituiert oder 1 - 3 fach substituiert ist mit F, Cl, Br, (C₁-C₄)-Alkyl oder (C₁-C₄)-Alkoxyl),
und in der Y folgende Bedeutung hat:
-OH oder eine über die Aminogruppe gebundene Aminosäure oder Aminosulfonsäure und deren Alkali- und Erdalkalisalze, -OKa, wobei Ka ein Kation bedeutet wie z. B. ein Alkali oder Erdalkaliion oder auch ein quartäres Ammoniumion,
das Verbindungsglied X 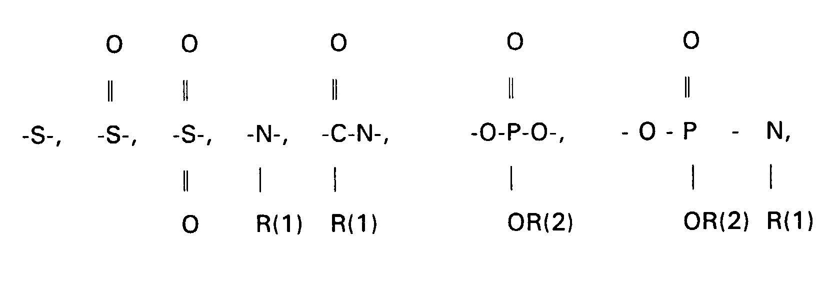 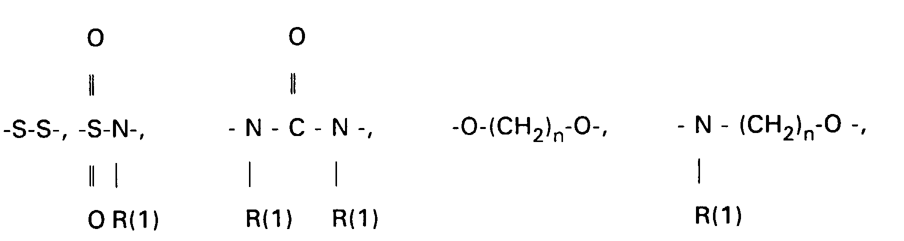 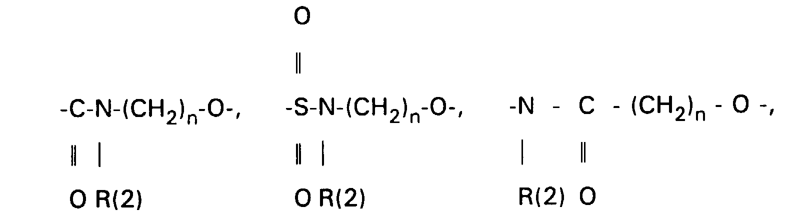 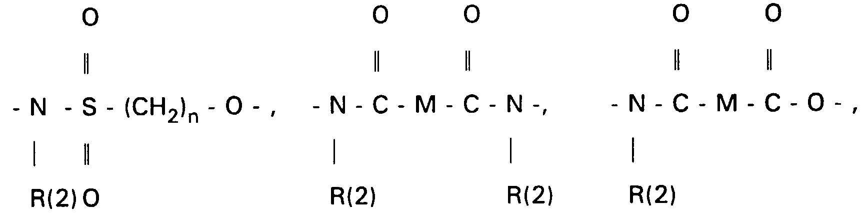 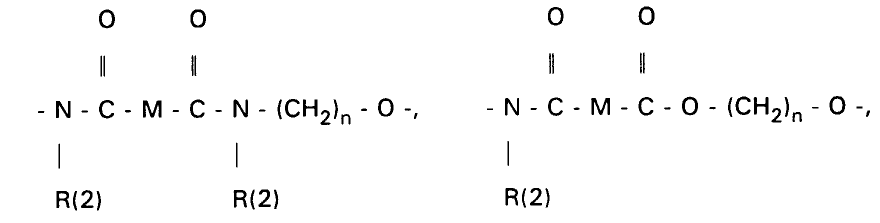 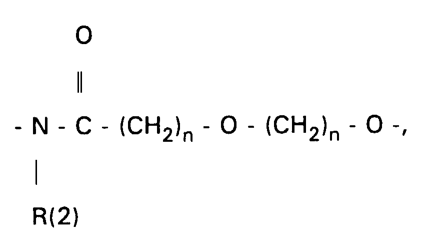 mit
R(1) = H, (C₁-C₈)-Alkyl; die Gruppe 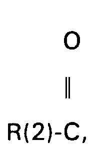 Phenyl, Benzyl, unsubstituiert oder im Kern 1 - 3 fach substituiert mit F, Cl, Br, (C₁-C₄)-Alkyl, Alkoxy mit 1 - 4 C-Atomen;
R(2) = H, (C₁-C₈)-Alkyl, Phenyl, Benzyl; jeweils unsubstituiert oder 1 - 3 fach substituiert mit F, Cl, Br, (C₁-C₄)-Alkyl oder Alkoxy mit 1 - 4 C-Atomen;
n = 1 - 16;
M = -(CH₂)ₘ- mit m = 2.

7. Verbindung XXII nach Anspruch 6, in welcher bedeuten
SG Wasserstoff,
X gleich -O(CH₂)₂₋₁₀-O-
-HN-(CH₂)₂₋₁₀-O-,
-O(CH₂)₂₋₄-O(CH₂)₂₋₄-O-,
-HN-(CH₂)₂₋₄-O(CH₂)₂₋₄-O-.

8. Verbindung XXII nach Anspruch 6 oder 7, in welcher bedeuten
SG Wasserstoff
X gleich -O(CH₂)₂₋₄-O- ,
-HN-(CH₂)₂₋₁₀-O-.

## Claims

1. A bile acid derivative of the formula I
W-X-G I
consisting of
a modified bile acid radical G: 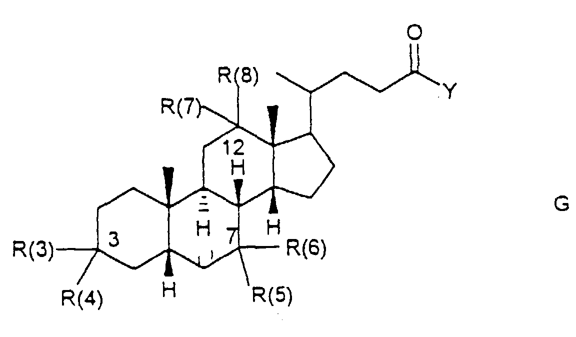 in which R(3) - R(8) are identical or different and have the following meaning:
a bond to W-X-, where a total of up to two W-X-units can be bonded,
R(3) and R(4), R(5) and R(6), R(7) and R(8) are each together the oxygen of a carbonyl group, 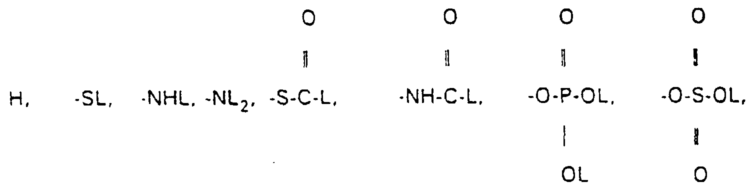 -L, where L is:
H, a saturated or unsaturated alkyl radical having 1 - 10 carbon atoms, which is branched or unbranched, cycloalkyl having 3 - 8 carbon atoms, a phenyl radical which is unsubstituted or substituted 1 - 3 times by F, Cl, Br, (C₁-C₄)-alkyl or (C₁-C₄)-alkoxy, a benzyl radical which is unsubstituted or substituted 1 - 3 times by F, Cl, Br, (C₁-C₄)-alkyl or (C₁-C₄)-alkoxy;
and in which Y has the following meaning:
-OH or
an amino acid bonded via the amino group or aminosulfonic acid and its alkali metal and alkaline earth metal salts, -OCat, where Cat is a cation such as, for example, an alkali metal or alkaline earth metal ion or alternatively a quaternary ammonium ion;
a connecting link X, which is a direct bond or an intermediate member X: 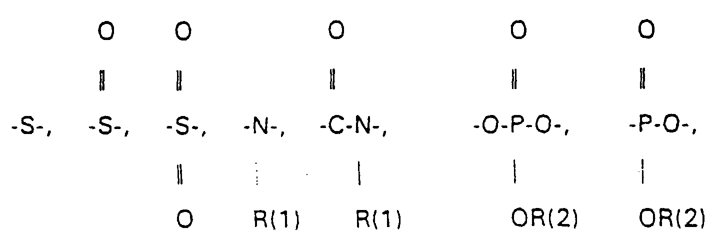 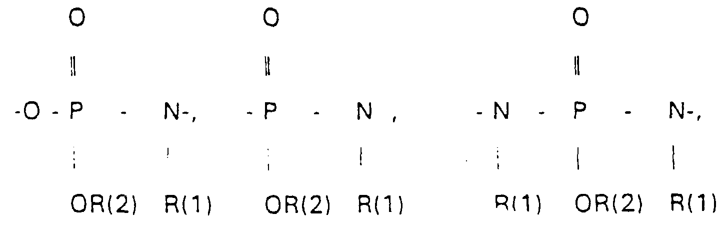 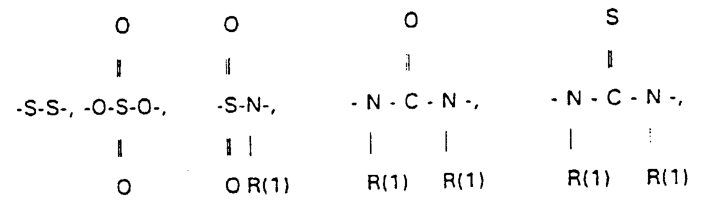 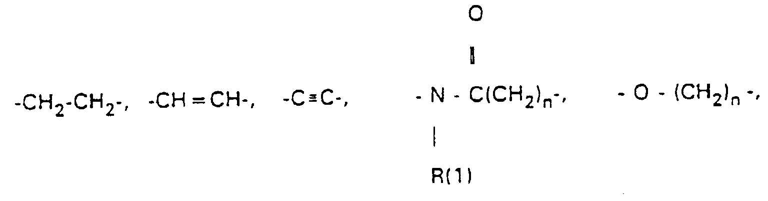 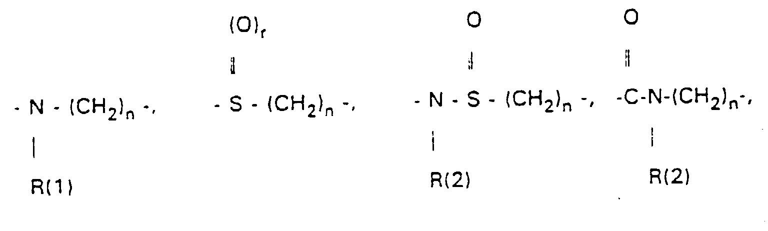 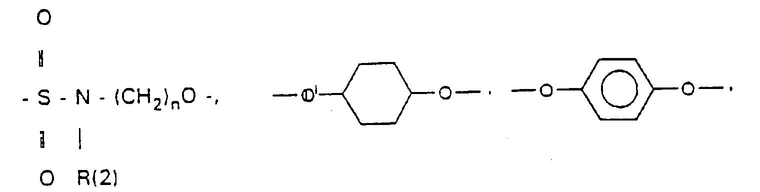 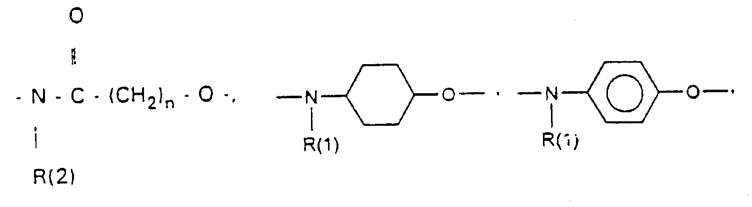 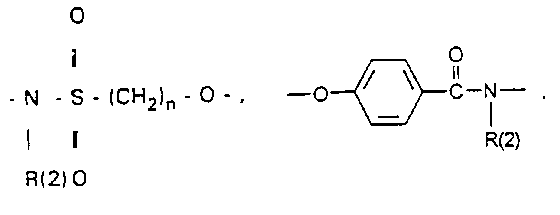 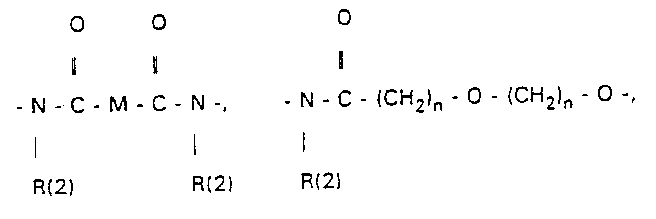 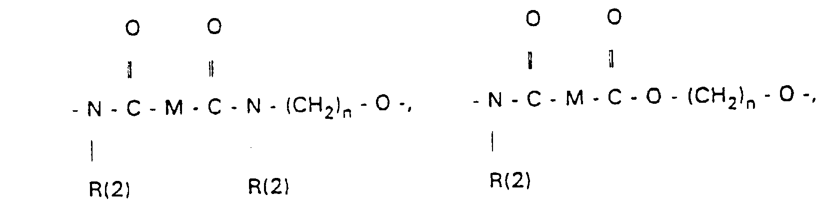 in which
R(1) = H, (C₁-C₈)-alkyl ; the group 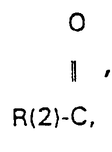 phenyl, benzyl, unsubstituted or substituted 1 - 3 times by F, Cl, Br, (C₁-C₄)-alkyl or alkoxy having 1 - 4 carbon atoms
R(2) = H, (C₁-C₈)-alkyl, phenyl, benzyl, unsubstituted or substituted 1 - 3 times by F, C1, Br, (C₁-C₄)-alkyl or alkoxy having 1 - 4 carbon atoms
m = 0 - 6
n = 1 - 16
p = 1, 2, 3
r = 0 - 2 and
M = -(CH₂)m-, -(C=C)ₚ-, -C≡C-, 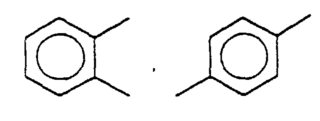 and an active compound moiety W, which is a peptide, an antibiotic, e.g. a β-lactam antibiotic, an antiviral substance, an anticancer agent such as, for example, chlorambucil, a hepatoprotective agent, an antihyperlipidemic, e.g. an HMG-CoA reductase inhibitor, a diuretic, a hypotensive, a renin inhibitor or a prolyl hydroxylase inhibitor,
a compound of the formula 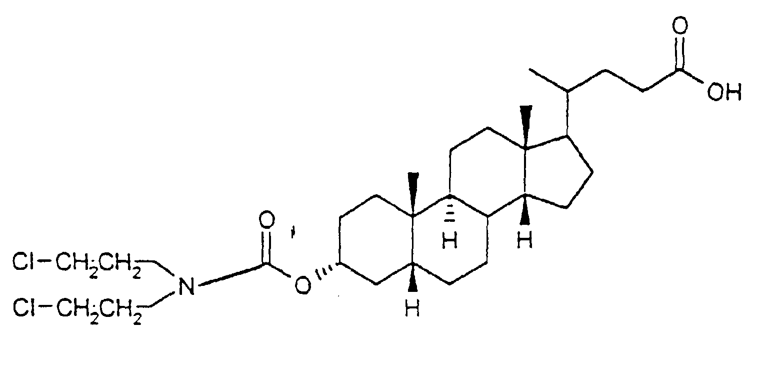 being excluded.

2. A compound I as claimed in claim 1, wherein the radicals have the following meaning:
G is 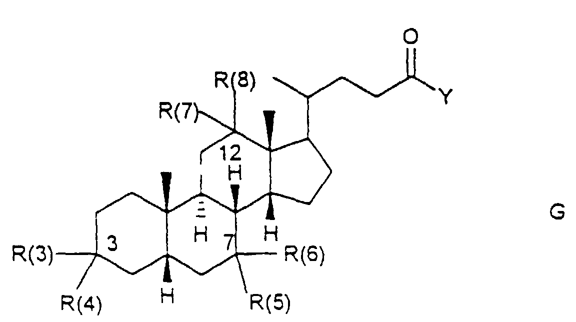 where R(3) - R(8) are identical or different and have the following meaning:
a bond to W-X-, where up to two W-X- units can be bonded, 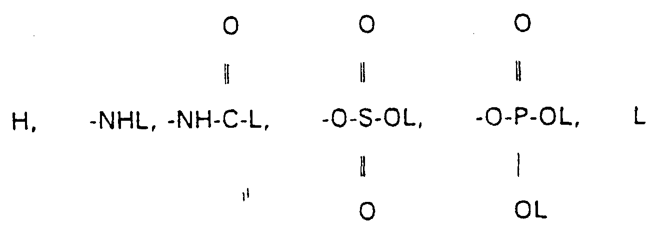 where L is:
H, a saturated alkyl radical having 1 - 6 carbon atoms, which can be branched or unbranched, a phenyl radical which is unsubstituted or substituted 1 - 3 times by F, Cl, Br, (C₁-C₄)-alkyl or (C₁-C₄)-alkoxy
Y is: -OH,
-OCat, where Cat is an alkali metal or alkaline earth metal cation or an ammonium ion 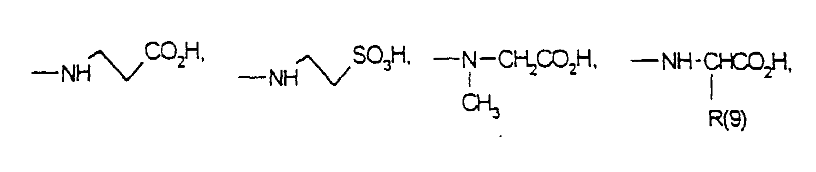 where R(9) is
methyl, isopropyl, isobutyl, 2-butyl, benzyl, 4-hydroxybenzyl, hydroxymethyl, 1-hydroxyethyl, H₃CSCH₂CH₂-, HO₂CCH₂-, HO₂CCH₂CH₂-
the connecting link X, where G and W can be bonded to any desired end of X, 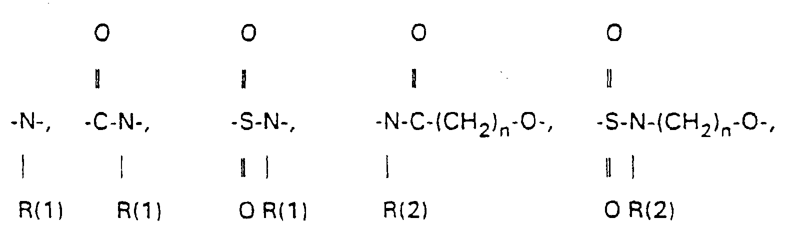 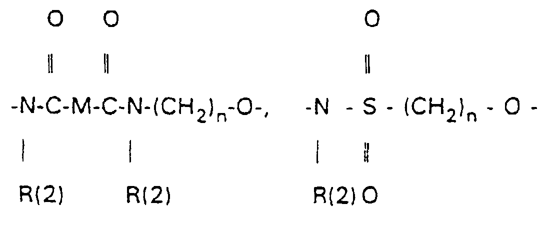
where R(1) = H, (C₁-C₈)-alkyl, 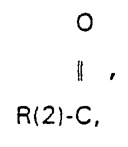 phenyl, benzyl, where the aromatic rings are unsubstituted or substituted 1 - 3 times by F, Cl, Br, (C₁-C₄)-alkyl or (C₁-C₄)-alkoxy;
where R(2) = H, (C₁-C₈)-alkyl, phenyl, benzyl; each unsubstituted or substituted 1 - 3 times by F, Cl, Br, (C₁-C₄)-alkyl or alkoxy having 1 - 4 carbon atoms
n = 1 - 16 and
M = -CH₂-CH₂-,
-CH=CH-
W is
a peptide, an antibiotic, e.g. a β-lactam antibiotic, an antiviral substance, an anticancer agent such as, for example, chlorambucil, a hepatoprotective agent, an antihyperlipidemic, e.g. an HMG-CoA reductase inhibitor, a diuretic, a hypotensive, a renin inhibitor, a prolyl hydroxylase inhibitor, a compound of the formula 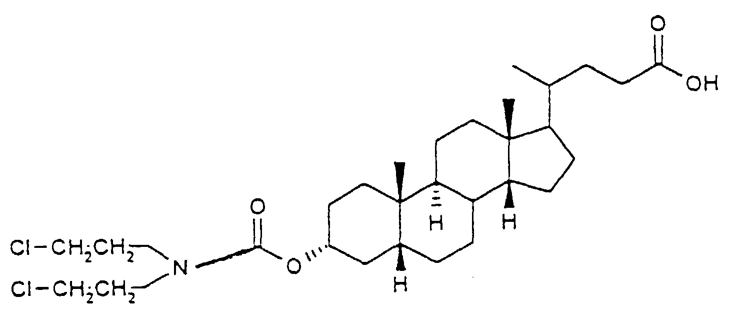 being excluded.

3. A process for preparing a compound I as claimed in claim 1, which comprises
a) if X = a direct bond, reacting suitable reactive forms of G and W with one another according to known processes or
b) if X = an intermediate link,
α) reacting reactive forms of G-X with W or
β) reacting reactive forms of W-X with G with one another according to known processes.

4. A compound I as claimed in claim 1 for use as a pharmaceutical.

5. The use of a compound I as claimed in claim 1 for the production of a pharmaceutical.

6. A compound XXII
SG-X-G XXII
in which
SG is H or a protective group,
G is 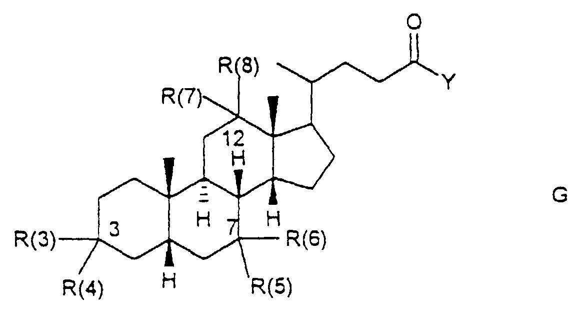 in which R(3) - R(8) are identical or different and have the following meaning:
a bond to SG-X-, where a total of up to two SG-X-units can be bonded,
R(3) and R(4), R(5) and R(6), R(7) and R(8) are each together the oxygen of a carbonyl group, 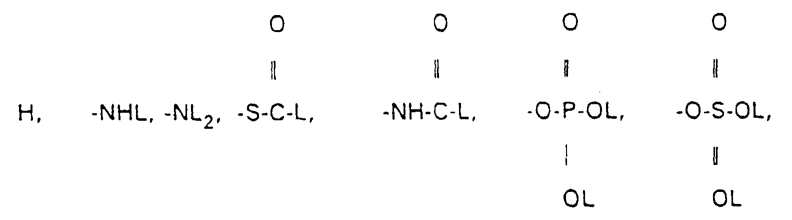 -L, where L is:
H, a saturated or unsaturated alkyl radical having 1 - 10 carbon atoms, which is branched or unbranched, cycloalkyl having 3 - 8 carbon atoms, a phenyl radical (which is unsubstituted or substituted 1 - 3 times by F, Cl, Br, (C₁-C₄)-alkyl or (C₁-C₄)-alkoxy), a benzyl (which is unsubstituted or substituted 1 - 3 times by F, Cl, Br, (C₁-C₄)-alkyl or (C₁-C₄)-alkoxy),
and in which Y has the following meaning:
-OH or an amino acid or aminosulfonic acid bonded via the amino group and their alkali metal and alkaline earth metal salts, -OCat, where Cat is a cation such as, for example, an alkali metal or alkaline earth metal cation or alteratively a quaternary ammonium ion,
the connective link X 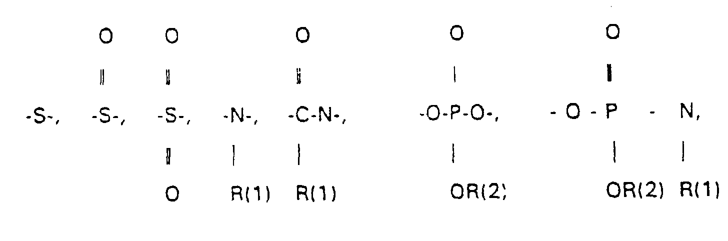 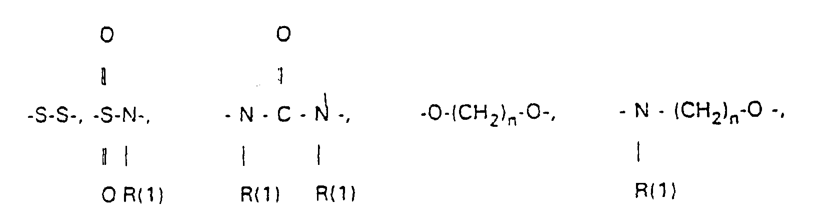 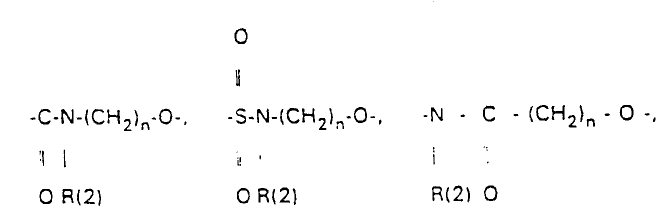 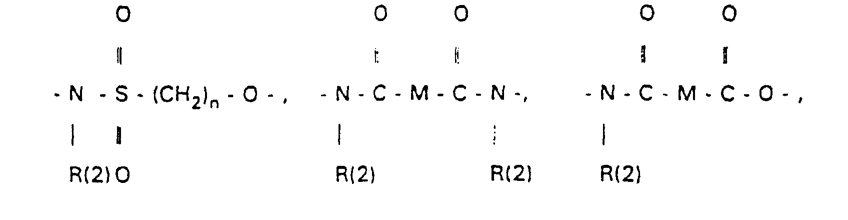 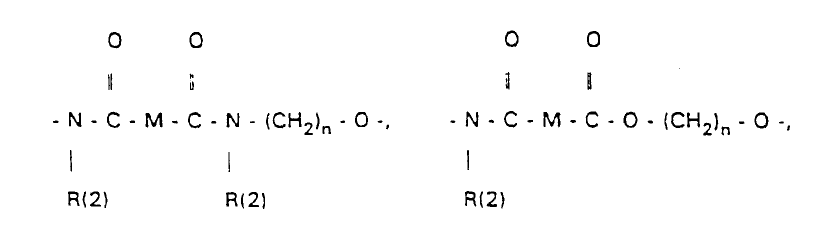 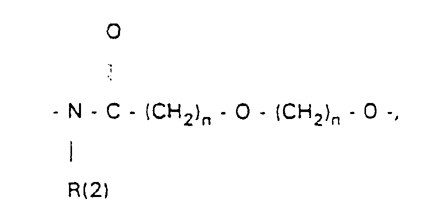 where
R(1) = H, (C₁-C₈)-alkyl; the group 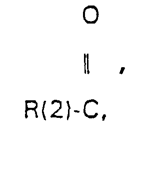 phenyl, benzyl, unsubstituted or substituted 1 - 3 times in the nucleus by F, Cl, Br, (C₁-C₄)-alkyl, alkoxy having 1 - 4 carbon atoms;
R(2) = H, (C₁-C₈)-alkyl, phenyl, benzyl; each unsubstituted or substituted 1 - 3 times by F, Cl, Br, (C₁-C₄)-alkyl or alkoxy having 1 - 4 carbon atoms
n = 1 - 16;
M = -(CH₂)ₘ- where m = 2.

7. A compound XXII as claimed in claim 6, in which
SG is hydrogen,
X is -O(CH₂)₂₋₁₀-O-,
-HN-(CH₂)₂₋₁₀-O-,
-O(CH₂)₂₋₄-O(CH₂)₂₋₄-O-,
-HN-(CH₂)₂₋₄-O(CH₂)₂₋₄-O-.

8. A compound XXII as claimed in claim 6 or 7, in which
SG is hydrogen,
X is -O(CH₂)₂₋₄-O-,
-HN-(CH₂)₂₋₁₀-O-.

## Revendications

1. Dérivés d'acides biliaires de formule I
W-X-G I
constitués
d'un reste d'acide biliaire modifié G: 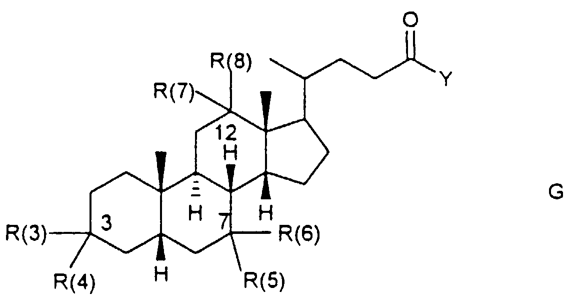 dans lequel les radicaux R(3) à R(8) sont identiques ou différents et ont les significations suivantes:
une liaison à W-X-, au total jusqu'à deux entités W-X pouvant être liées,
R(3) et R(4), R(5) et R(6), R(7) et R(8) représentent dans chaque cas ensemble l'atome d'oxygène d'un groupe carbonyle, 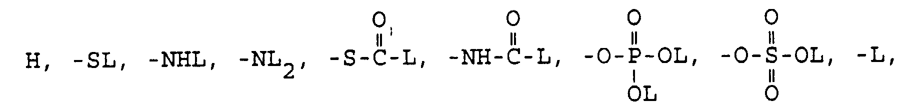 L représentant:
H ou un radical alkyle saturé ou insaturé ayant de 1 à 10 atomes de carbone, qui est ramifié ou non ramifié, ou un radical cycloalkyle ayant de 3 à 8 atomes de carbone, un radical phényle (qui est non substitué ou 1 à 3 fois substitué par un ou des atomes de F, Cl, Br ou groupes alkyle en C₁-C₄ ou alcoxy en C₁-C₄), un radical benzyle, qui est non substitué ou 1 à 3 fois substitué par un ou des atomes de F, Cl, Br ou groupes alkyle en C₁-C₄ ou alcoxy en C₁-C₄;
et dans lequel Y a la signification suivante:
-OH ou un aminoacide ou acide aminosulfonique lié par le groupe amino, et leurs sels alcalins et leurs sels alcalino-terreux, -OKa, Ka représentant un cation, comme par exemple un ion alcalin ou alcalino-terreux, ou bien un ion ammonium quaternaire;
d'un chaînon de liaison X, qui est une liaison directe ou un chaînon intermédiaire X: 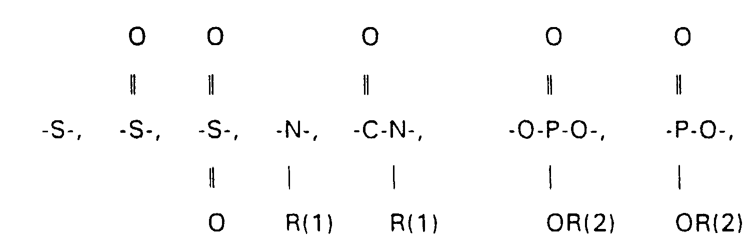 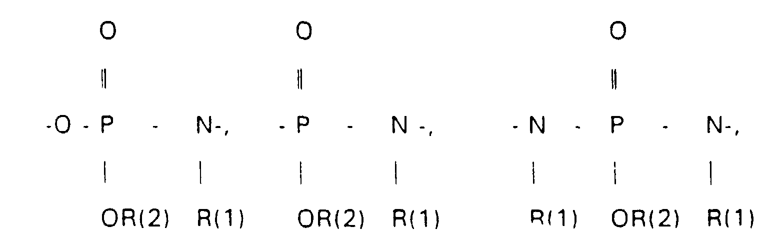 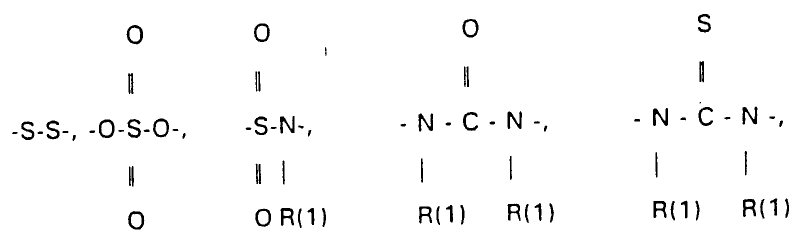 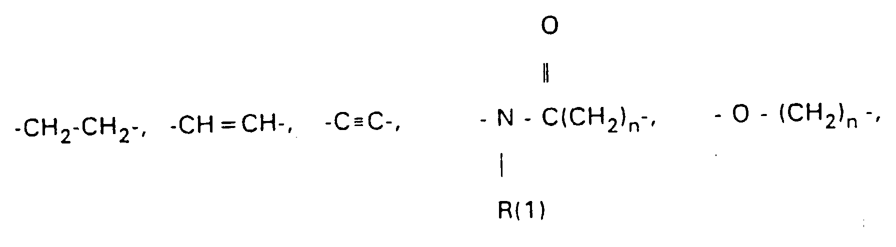 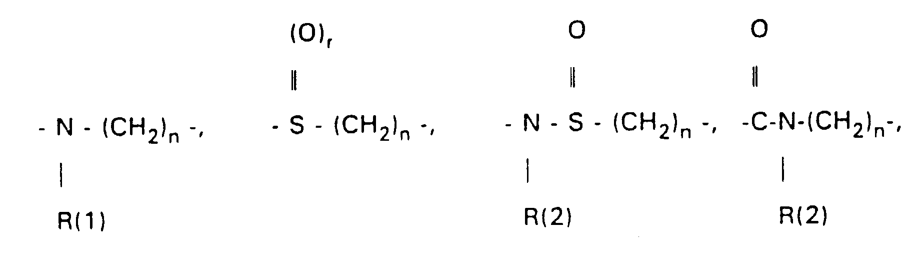 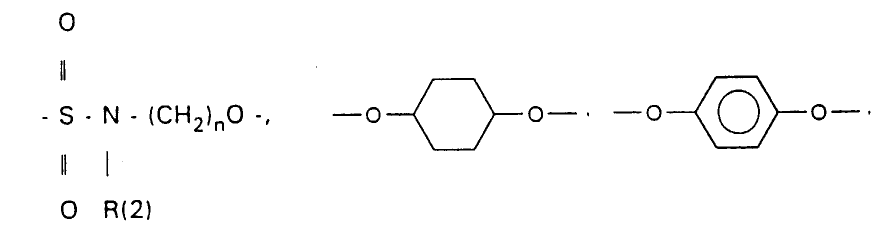 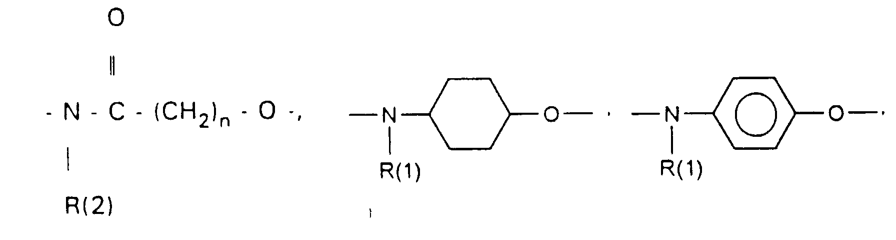 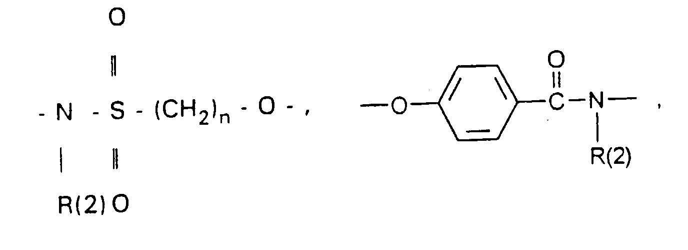 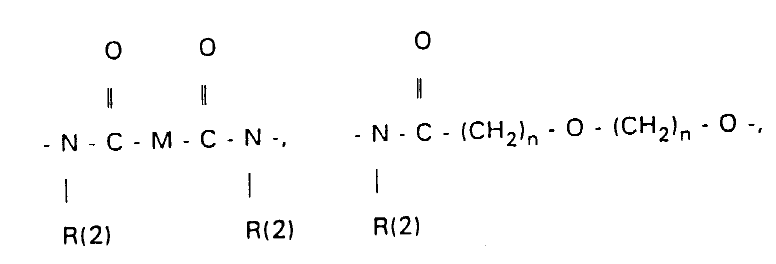 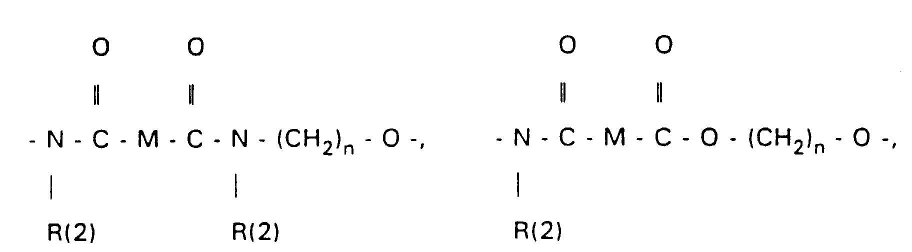 où
R(1) représente H ou un groupe alkyle en C₁-C₈, 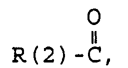 phényle, benzyle, non substitué ou 1 à 3 fois substitué par un ou des atomes de F, Cl, Br ou groupes alkyle en C₁-C₄ ou alcoxy ayant de 1 à 4 atomes de carbone,
R(2) représente H ou un groupe alkyle en C₁-C₈, phényle, benzyle, non substitué ou 1 à 3 fois substitué par un ou des atomes de F, Cl, Br ou groupes alkyle en C₁-C₄ ou alcoxy ayant de 1 à 4 atomes de carbone,
m = 0-6,
n = 1-16,
p = 1, 2, 3,
r = 0-2 et
M = -(CH₂)ₘ-, -(C=C)ₚ-, -C≡C-, 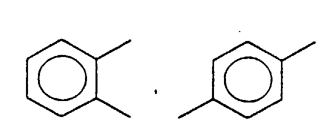
et d'une substance active W qui est un peptide, un antibiotique, par exemple un antibiotique de type β-lactame, une substance antivirale, un agent anticancéreux, comme par exemple le chlorambucil, un agent protecteur du foie, un anti-hyperlipidémiant, par exemple un inhibiteur de HMG-CoA réductase, un diurétique, un hypotenseur, un inhibiteur de la rénine ou un inhibiteur de la prolylhydroxylase,
à l'exclusion du composé de formule

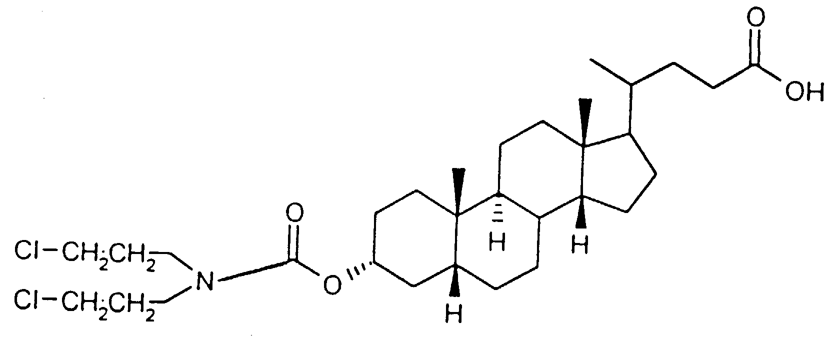

2. Composé I selon la revendication 1, caractérisé en ce que les radicaux ont les significations suivantes:
G est 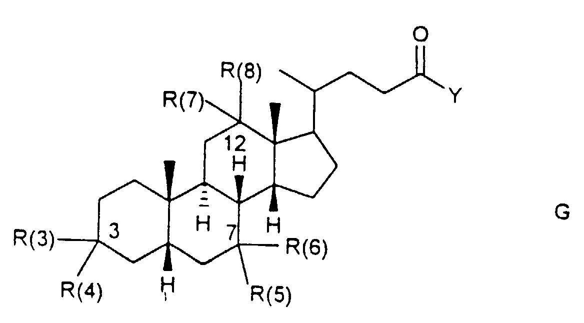 où les radicaux R(3) à R(8) sont identiques ou différents et ont les significations suivantes: une liaison à W-X-, jusqu'à deux entités W-X pouvant être liées, 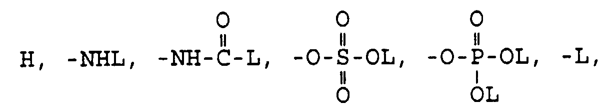 L représentant
H ou un radical alkyle saturé ayant de 1 à 6 atomes de carbone, qui peut être ramifié ou non ramifié, un radical phényle qui est non substitué ou 1 à 3 fois substitué par un ou des atomes de F, Cl, Br ou groupes alkyle en C₁-C₄ ou alcoxy en C₁-C₄,
Y représente -OH ou -OKa, Ka représentant un ion alcalin ou alcalino-terreux ou un ion ammonium, 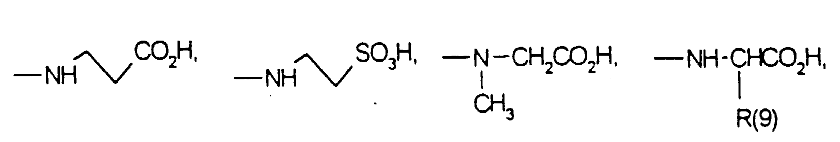 R(9) représentant le groupe méthyle, isopropyle, isobutyle, 2-butyle, benzyle, 4-hydroxybenzyle, hydroxyméthyle, 1-hydroxyéthyle, H₃CSCH₂CH₂-, HO₂CCH₂-, HO₂CCH₂CH₂-, le chaînon de liaison X, G et W pouvant être liés à n'importe quelle extrémité de X, est 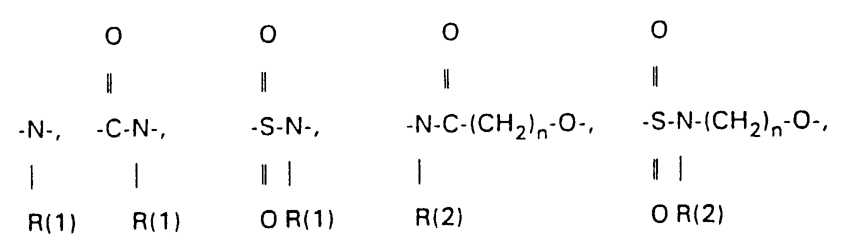 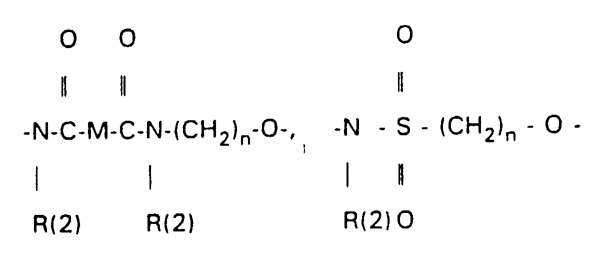 où
R(1) représente H ou un groupe alkyle en C₁-C₈, 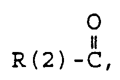 phényle, benzyle, les cycles aromatiques étant non substitués ou 1 à 3 fois substitués par un ou des atomes de F, Cl, Br ou groupes alkyle en C₁-C₄ et alcoxy en C₁-C₄;
R(2) représente H ou un groupe alkyle en C₁-C₈, phényle, benzyle; dans chaque cas non substitué ou 1 à 3 fois substitué par un ou des atomes de F, Cl, Br ou groupes alkyle en C₁-C₄ ou alcoxy ayant de 1 à 4 atomes de carbone;
n = 1-16 et
M = -CH₂-CH₂-, -CH=CH-,
W est un peptide, un antibiotique, par exemple un antibiotique de type β-lactame, une substance antivirale, un agent anticancéreux, comme par exemple le chlorambucil, un agent protecteur du foie, un anti-hyperlipidémiant, par exemple un inhibiteur de HMG-CoA réductase, un diurétique, un hypotenseur, un inhibiteur de la rénine ou un inhibiteur de la prolylhydroxylase,
à l'exclusion du composé de formule

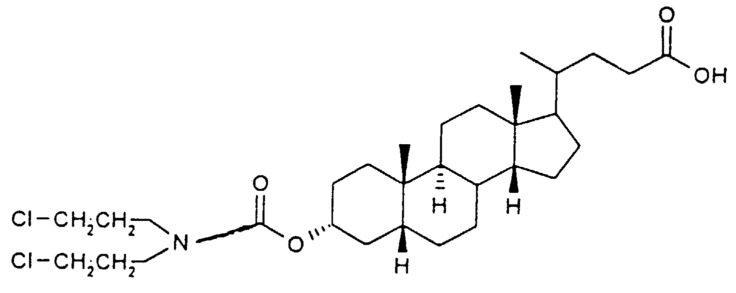

3. Procédé pour la préparation d'un composé I selon la revendication 1, caractérisé en ce que
a) lorsque X est une liaison directe, on fait réagir l'un avec l'autre, selon des procédés connus, des formes réactives appropriées de G et W, ou
b) lorsque X représente un chaînon intermédiaire, on fait réagir l'un avec l'autre, selon des procédés connus,
α) des formes réactives de G-X avec W ou
β) des formes réactives de W-X avec G.

4. Composé I selon la revendication 1, pour utilisation en tant que médicament.

5. Utilisation d'un composé I selon la revendication 1, pour la fabrication d'un médicament.

6. Composé XXII
SG-X-G XXII
dans lequel
SG représente H ou un groupe protecteur,
G est 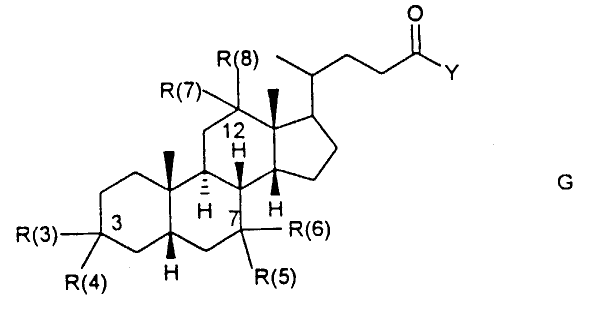 où les radicaux R(3) à R(8) sont identiques ou différents et ont les significations suivantes:
une liaison à SG-X-, au total jusqu'à deux entités SG-X-pouvant être liées,
R(3) et R(4), R(5) et R(6), R(7) et R(8) représentent dans chaque cas ensemble l'atome d'oxygène d'un groupe carbonyle, 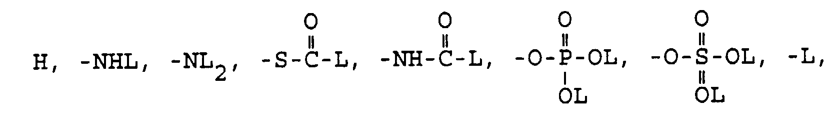 L représentant:
H ou un radical alkyle saturé ou insaturé ayant de 1 à 10 atomes de carbone, qui est ramifié ou non ramifié, un radical cycloalkyle ayant de 3 à 8 atomes de carbone, un radical phényle (qui est non substitué ou 1 à 3 fois substitué par un ou des atomes de F, Cl, Br ou groupes alkyle en C₁-C₄ ou alcoxy en C₁-C₄), un radical benzyle (qui est non substitué ou 1 à 3 fois substitué par un ou des atomes de F, Cl, Br ou groupes alkyle en C₁-C₄ ou alcoxy en C₁-C₄) ;
et dans lequel Y a la signification suivante:
-OH ou un aminoacide ou acide aminosulfonique lié par le groupe amino, et leurs sels alcalins et leurs sels alcalino-terreux, -OKa, Ka représentant un cation, comme par exemple un ion alcalin ou alcalino-terreux, ou bien un ion ammonium quaternaire;
le chaînon de liaison X est 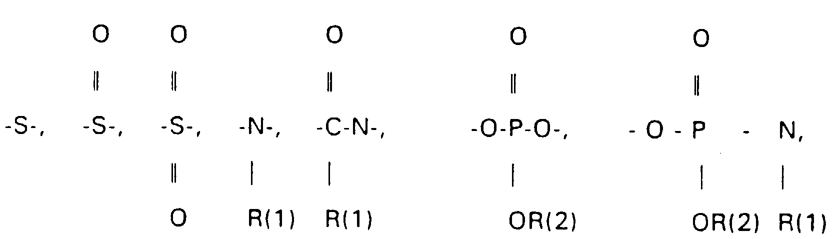 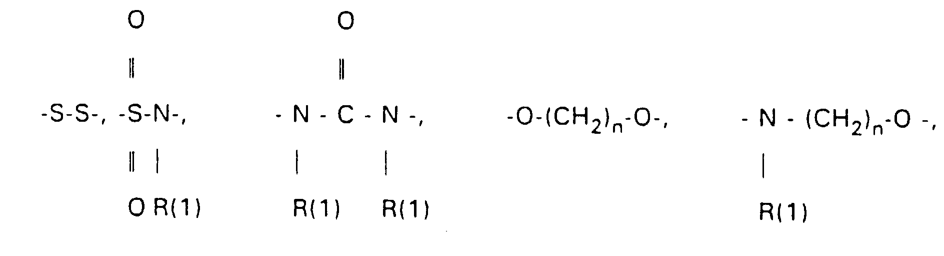 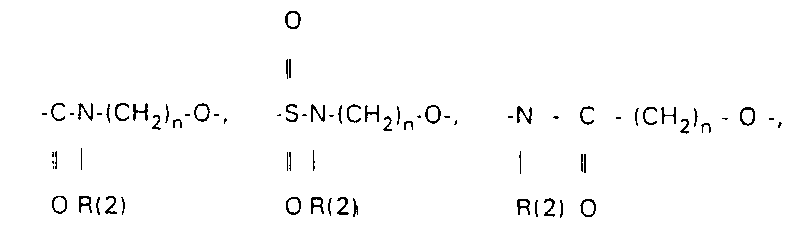 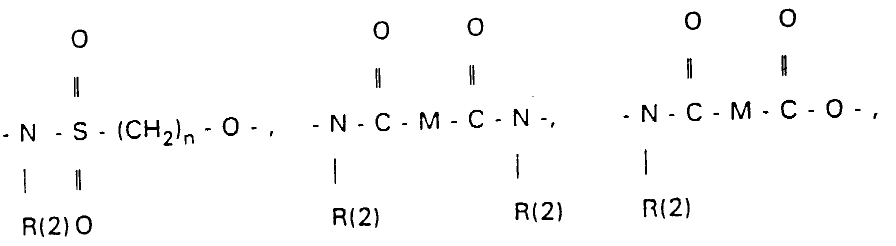 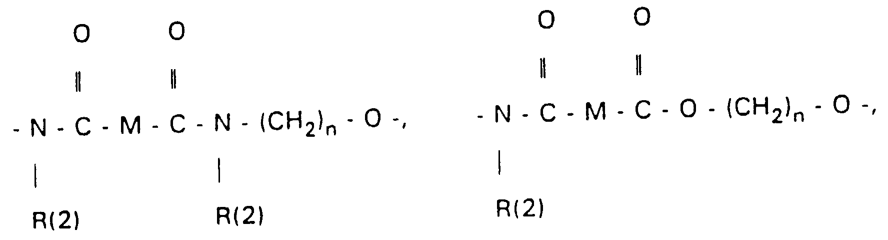 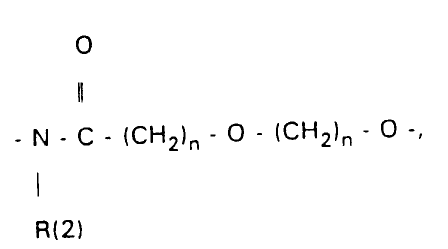 où
R(1) = H ou un groupe alkyle en C₁-C₈, 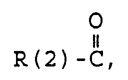 phényle, benzyle non substitué ou 1 à 3 fois substitué sur le noyau par un ou des atomes de F, Cl, Br ou groupes alkyle en en C₁-C₄ ou alcoxy ayant de 1 à 4 atomes de carbone;
R(2) = H ou un groupe alkyle en C₁-C₈, phényle, benzyle; chacun non substitué ou 1 à 3 fois substitué par un ou des atomes de F, Cl, Br ou groupes alkyle en C₁-C₄ ou alcoxy ayant de 1 à 4 atomes de carbone;
n = 1-16 et
M = -(CH₂)ₘ- avec m = 2.

7. Composé XXII selon la revendication 6, dans lequel
SG représente un atome d'hydrogène,
X -O(CH₂)₂₋₁₀-O-,
-HN-(CH₂)₂₋₁₀-O-,
-O(CH₂)₂₋₄-O-(CH₂)₂₋₄-O-,
-HN-(CH₂)₂₋₄-O-(CH₂)₂₋₄-O-

8. Composé XXII selon la revendication 6 ou 7, dans lequel
SG représente un atome d'hydrogène
X représente -O(CH₂)₂₋₄-O-,
-HN-(CH₂)₂₋₁₀-O-.
